# EUROPÄISCHE PATENTSCHRIFT

(11) **EP 1 017 659 B1**
(45) Veröffentlichungstag und Bekanntmachung des Hinweises auf die Patenterteilung: **27.04.2005**
(21) Anmeldenummer: 98951408.8
(22) Anmeldetag: 16.09.1998
(51) Int. Cl.: C07C 35/52, C07C 43/192, C07C 49/813, C07C 49/83, C07F 7/18

(54) **SUBSTITUIERTE TETRAHYDRO-NAPHTALINE UND ANALOGE VERBINDUNGEN**
SUBSTITUTED TETRAHYDRONAPHTHALINE AND ANALOGOUS COMPOUNDS
TETRAHYDRONAPHTALINES SUBSTITUEES ET COMPOSES ANALOGUES

(30) Priorität: 18.09.1997 DE 19741050; 17.07.1998 DE 19832159
(43) Veröffentlichungstag der Anmeldung: 12.07.2000
(73) Patentinhaber: Bayer HealthCare AG, 51368 Leverkusen (DE)
(72) Erfinder: BRANDES, Arndt, D-42115 Wuppertal (DE); LÖGERS, Michael, D-42327 Wuppertal (DE); STOLTEFUSS, Jürgen, D-42781 Haan (DE); SCHMIDT, Gunter, D-42115 Wuppertal (DE); BREMM, Klaus-Dieter, D-45661 Recklinghausen (DE); BISCHOFF, Hilmar, D-42113 Wuppertal (DE); SCHMIDT, Delf, D-42113 Wuppertal (DE); ANTONS, Stefan, D-51373 Leverkusen (DE); PAULSEN, Holger, D-42115 Wuppertal (DE); MÜLLER, Stephan, Nicholas, D-42349 Wuppertal (DE); NAAB, Paul, D-42287 Wuppertal (DE); SCHMECK, Carsten, D-42113 Wuppertal (DE)
(86) Internationale Anmeldenummer: PCT/EP1998/005874
(87) Internationale Veröffentlichungsnummer: WO 1999/014174

(56) Entgegenhaltungen:
- EP-A- 0 818 448
- US-A- 5 446 207

## Beschreibung

Die vorliegende Erfindung betrifft substituierte Tetrahydro-naphthaline und analoge Verbindungen, Verfahren zu ihrer Herstellung und ihre Verwendung in Arzneimitteln.

Aus der Publikation US-5 169 857-A2 sind 7-(polysubstituierte Pyridyl)-6-heptenoate zur Behandlung der Arteriosklerose, Lipoproteinaemia und Hyperproteinaemia bekannt. Außerdem wird die Herstellung von 7-(4-Aryl-3-pyridyl)-3,5-dihydroxy-6-heptenoate in der Publikation EP-325 130-A2 beschrieben.

Die vorliegende Erfindung betrifft.substituierte Tetrahydro-naphthaline und analoge Verbindungen der allgemeinen Formel (I), 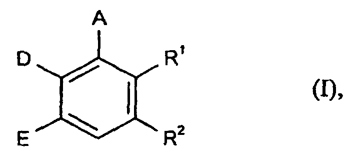 in welcher
- A: für Phenyl, Pyridyl oder Thienyl steht, die gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert sind,
- D: für einen Rest der Formel 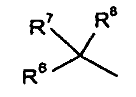 steht,
worin
R⁶ Phenyl, Pyridyl, Pyrimidyl und Pyrazinyl, bedeuten, wobei die Cyclen, gegebenenfalls bis zu 3-fach, im Fall der stickstoffhaltigen Ringe auch über die N-Funktion, gleich oder verschieden durch Fluor, Chlor oder Trifluormethyl substituiert sind
R⁷ Wasserstoff oder Fluor bedeutet, und
R⁸ Wasserstoff, Fluor, Chlor, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten, oder
R⁷ und R⁸ gemeinsam einen Rest der Formel =O bilden,
E für Cyclopropyl, Cyclobutyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
- R¹ und R²: gemeinsam eine geradkettige oder verzweigte Alkylenkette mit bis zu 7 Kohlenstoffatomen bilden, die durch eine Carbonylgruppe oder einen Rest der Formel

-OR¹⁹

substituiert sein muß,
worin
R¹⁹ Wasserstoff, Cyclopropyl, Cyclopentyl, Cyclohexyl, geradkettiges oder verzweigtes Silylalkyl mit bis zu 6 Kohlenstoffatomen oder geradkettiges oder verzweigtes Alkyl mit bis zu 4 Kohlenstoffatomen bedeutet, und die die von R¹ und R² gebildete Alkylenkette gegebenenfalls durch einen spiro-verknüpften Rest der Formel 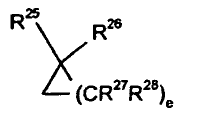 substituiert ist,
worin
e eine Zahl 1, 2, 3, 4 oder 5 bedeutet,
R²⁵, R²⁶, R²⁷ und R²⁸ gleich oder verschieden sind und Wasserstoff bedeuten,
und deren Stereoisomere, Stereoisomerengemische und Salze.

Ganz besonders bevorzugt sind die erfindungsgemäßen Verbindungen der allgemeinen Formel (I),
in welcher
- A: für gegebenenfalls durch Fluor substituiertes Phenyl, insbesondere 4-Fluorphenyl, steht,
und
- D: für einen Rest der Formel 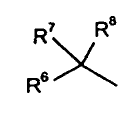 steht,
worin
R⁶ Phenyl oder Trifluormethyl-substituiertes Phenyl bedeutet, wobei Trifluormethyl-substituiertes Phenyl bevorzugt ist,
R⁷ Wasserstoff bedeutet,
R⁸ Wasserstoff, Fluor, Methoxy oder Hydroxy bedeutet, wobei Fluor bevorzugt ist oder
R⁷ und R⁸ gemeinsam eine Carbonylgruppe bilden,
- E: für Cyclopropyl, Cyclopentyl oder Cyclohexyl steht, oder für geradkettiges oder verzweigtes Alkyl mit bis zu 4 Kohlenstoffatomen steht, das gegebenenfalls durch Hydroxy substituiert ist,
und
- R¹ und R²: gemeinsam für einen Rest der Formel 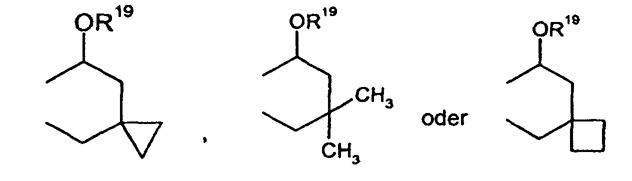 stehen, worin
R¹⁹ Wasserstoff bedeutet,
und deren Stereoisomere, Stereoisomerengemische und Salze.

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten, partiell ungesättigten oder ungesättigten 5- bis 7-gliedrigen, vorzugsweise 5- bis 6-gliedrigen Heterocyclus, der bis zu 4 Heteroatome aus der Reihe S, N und/oder O enthalten kann. Beispielsweise seien genannt: Indolyl, Isochinolyl, Chinolyl, Benzo[b]thiophen, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Die Verbindungen der allgemeinen Formel (I) können hergestellt werden, indem man
[A] Verbindungen der allgemeinen Formel (II) 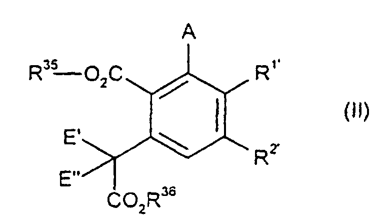 in welcher
   - A: die oben angegebene Bedeutung hat,
   - R³⁵ und R³⁶: gleich oder verschieden sind und geradkettiges oder verzweigtes Alkyl mit bis zu 4 Kohlenstoffatomen bedeuten,
   - R^{1'} und R^{2'}: gemeinsam für die unter R¹ und R² oben angegebene geradkettige oder verzweigte Alkylenkette mit bis zu 7 Kohlenstoffatomen stehen, die durch tert.Butyl-dimethyl-silanyloxy (OTBS) substituiert ist,
   und
   - E' und E": gemeinsam mit dem an sie gebundenen Kohlenstoffatom den oben angegebenen Bedeutungsumfang von E umfassen,
   zunächst durch selektive Reduktion des aliphatischen Esters und anschließende Umsetzung mit Verbindungen der allgemeinen Formel (III)

   R³⁷-Halogen (III)

   in welcher
   - R³⁷: für Mesyl, Tosyl oder Sulfonyl steht,
   und
   - Halogen: für Chlor, Brom oder Jod, vorzugsweise für Chlor steht,
   in inerten Lösemitteln, in Anwesenheit einer Base,
   in die Verbindungen der allgemeinen Formel (IV) 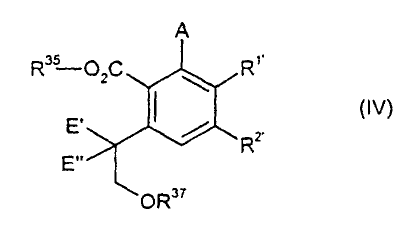 in welcher
   A, E', E", R^{1'}, R^{2'}, R³⁵ und R³⁷ die oben angegebene Bedeutung haben,
   überführt,
   diese in einem weiteren Schritt in Abhängigkeit von den oben angegebenen Definitionen von E'/E'' entweder durch eine zweifache Reduktion oder durch eine Verseifung, Bartonreaktion und eine Reduktion in die Verbindungen der allgemeinen Formel (V) 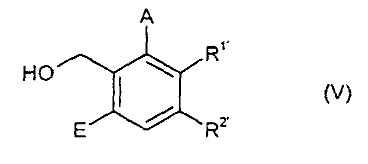 in welcher
   A, E, R^{1'} und R^{2'} die oben angegebene Bedeutung haben,
   überführt,
   anschließend durch Oxidation die Aldehyde der allgemeinen Formel (VI) 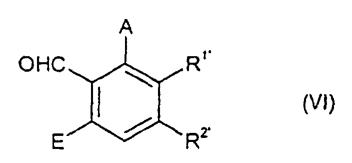 in welcher
   A, E, R^{1'} und R^{2'} die oben angegebene Bedeutung haben,
   herstellt,
   und abschließend beispielsweise durch eine Grignard-Reaktion die Formylgruppe in den Rest D überführt und die TBS-Gruppe nach üblichen Methoden abspaltet,
   oder
[B] Verbindungen der allgemeinen Formel (VI) 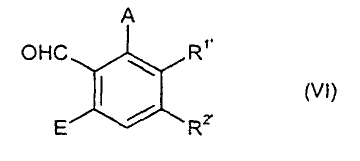 in welcher
   A, E, R^{1'} und R^{2'} die oben angegebene Bedeutung haben, zunächst wie unter [A] beschrieben in einer Grignard-Reaktion mit Verbindungen der allgemeinen Formel (VII)

   R³⁸-MgBr (VII)

   in welcher
   - R³⁸: die oben angebene Bedeutung von R⁶ umfaßt,
   in inerten Lösemitteln und unter Schutzgasatmosphäre in die Verbindungen der allgemeinen Formel (VIII) 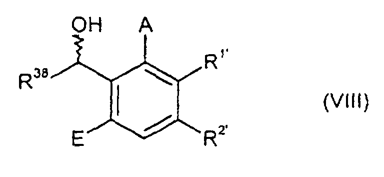 in welcher
   A, D, E, R³⁸, R^{1'} und R^{2'} die oben angegebene Bedeutung haben,
   überführt,
   gegebenenfalls auf dieser Stufe ausgehend von der Hydroxyfunktion die unter D angegebenenen Substituenten R⁷/R⁸ nach üblichen Methoden einführt,
   und abschließend die TBS-Gruppe mit Tetrabutylammoniumfluorid in inerten Lösemitteln abspaltet,
   oder
[C] Verbindungen der allgemeinen Formel (IX) 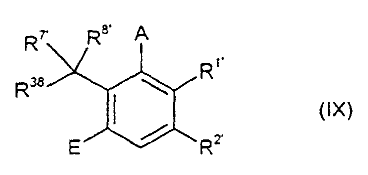 in welcher
   A, E, R^{1'}, R^{2'} und R³⁸ die oben angegebene Bedeutung haben
   und
   R^{7'} und R^{8'} gemeinsam für die Carbonylgruppe stehen,
   zunächst zu den Verbindungen der allgemeinen Formel (VIII) reduziert und anschließend wie unter [B] beschrieben umsetzt,
   oder
[D] Verbindungen der allgemeinen Formel (X) 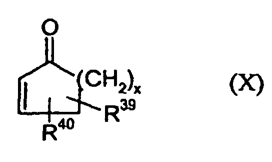 in welcher
   - x: eine Zahl 1, 2 oder 3 bedeutet,
   und
   - R³⁹ und R⁴⁰: gleich oder verschieden sind und Für Wasserstoff stehen oder für geradkettiges oder verzweigtes Alkyl mit bis zu 6 Kohlenstoffatomen stehen,
   wobei R³⁹ und R⁴⁰ auch geminal positioniert sein können,
   oder
   R³⁹ und R⁴⁰ gemeinsam einen spiroverknüpften Carbocyclus mit 3 bis 7 Kohlenstoffatomen bilden,
   mit den Verbindungen der allgemeinen Formel (XI) 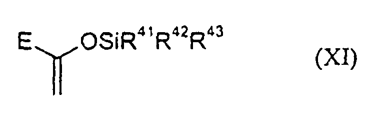 wobei
   - E: die oben angegebene Bedeutung hat,
   und
   - R⁴¹, R⁴² und R⁴³: gleich oder verschieden sind und für geradkettiges oder verzweigtes Alkyl mit bis zu 10 Kohlenstoffatomen stehen,
   in Anwesenheit eines Metall- oder Halbmetallreagenzes, zunächst über die intermediäre Stufe der allgemeinen Formel (XII) 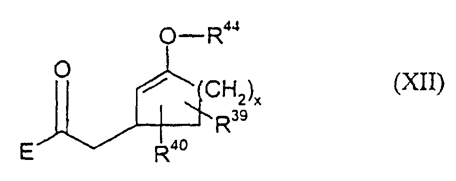 in weicher
   x, E, R³⁹ und R⁴⁰ die oben angegebene Bedeutung haben,
   und
   - R⁴⁴: für Metall- oder Halbmetallderivate, vorzugsweise für die des Titans steht,
   und durch Zugabe der Verbindungen der allgemeinen Formel (XIII) 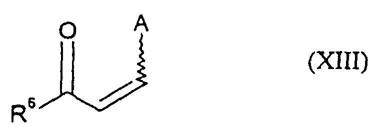 in welcher
   R⁶ und A die oben angegebene Bedeutung haben,
   über die Intermediate der allgemeinen Formel (XIV) 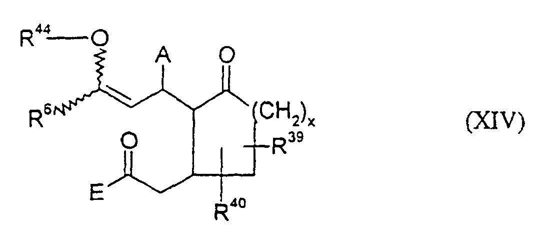 in welcher
   A, E, R⁶, R³⁹, R⁴⁰, R⁴⁴ und x die oben angegebene Bedeutung haben,
   in die Verbindungen der allgemeinen Formel (XV) 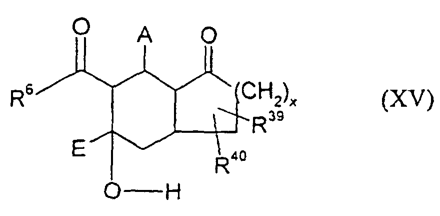 in welcher
   A, E, R⁶, R³⁹, R⁴⁰ und x, die oben angegebene Bedeutung haben,
   überführt,
   anschließend durch eine Elimierung die Verbindungen der allgemeinen Formel (XVI) 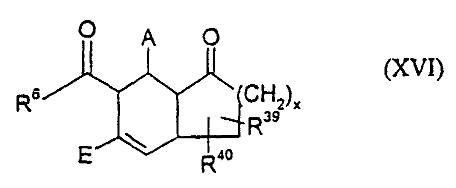 in welcher
   A, E, R⁶, R³⁹, R⁴⁰ und x die oben angegebene Bedeutungen haben,
   herstellt,
   in einem weiteren Schritt durch eine Halogenierung Halogenverbindungen, z.B. die Verbindungen der allgemeinen Formel (XVII) und/oder (XVIIa) 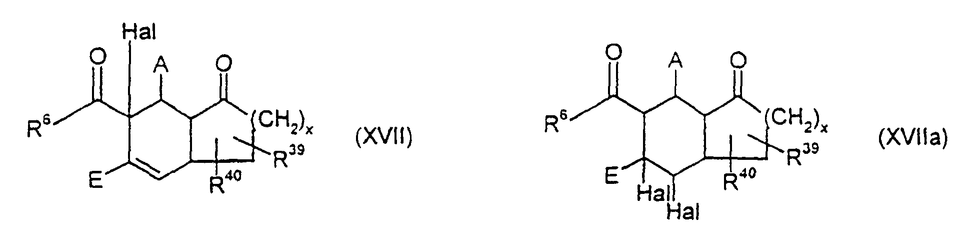 in welcher
   R⁶, R³⁹, R⁴⁰, x, A und E die oben angegebenen Bedeutungen haben,
   und
   - Hal: für Halogen, vorzugsweise für Chlor oder Brom steht.
   über die in situ entstehende Cyclohexadiene der allgemeinen Formel (XVIII) oder deren Isomere 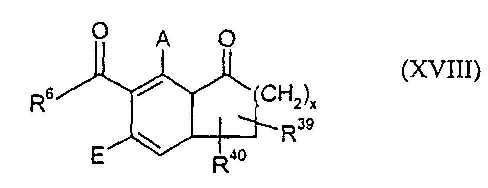 in welcher
   A, E, x, R⁶, R³⁹ und R⁴⁰ die oben angegebene Bedeutung haben,
   nach Oxidation die Verbindungen der allgemeinen Formel (XIX) 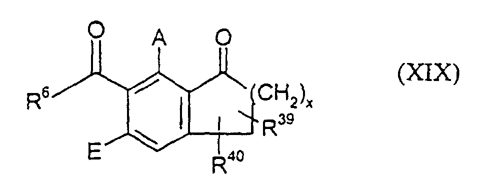 in welcher
   A, E, x, R⁶, R³⁹ und R⁴⁰ die oben angegebenen Bedeutungen haben
   herstellt,
   desweiteren durch die Reduktion der Ketofunktion die Verbindungen der allgemeinen Formel (XX) 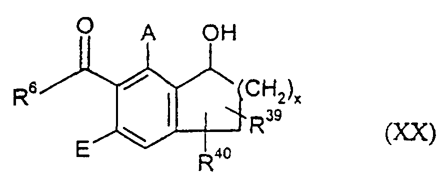 worin
   A, E, x, R⁶, R³⁹ und R⁴⁰ die oben angegebene Bedeutung haben,
   herstellt, wobei die Reduktion auch stereoselektiv durchgeführt werden kann,
   anschließend durch Silylierung, z.B. durch Umsetzung mit chlorierten oder Trifluormethansulfonyl-substituierten Silylverbindungen (-SiR⁴¹R⁴²R⁴³)
   die Verbindungen der allgemeinen Formel (XXI) 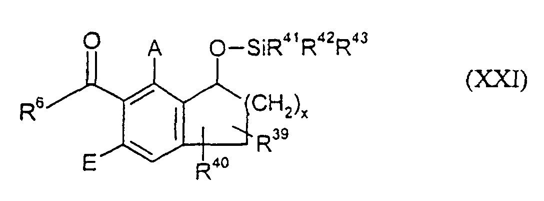 in welcher
   x, A, E, R⁶, R³⁹, R⁴⁰, R⁴¹, R⁴² und R⁴³ die oben angegebene Bedeutung haben,
   herstellt,
   desweiteren durch Reduktion der zweiten Ketofunktion zunächst die Verbindungen der allgemeinen Formel (XXII) 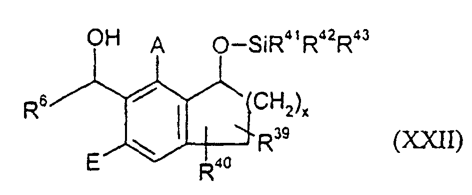 in welcher
   x, A, E, R⁶, R³⁹, R⁴⁰, R⁴¹, R⁴² und R⁴³ die oben angegebene Bedeutung haben,
   als Diastereomerengemisch erhält, aus diesem anschließend durch Trennung das Isomer der allgemeinen Formel (XXIII) 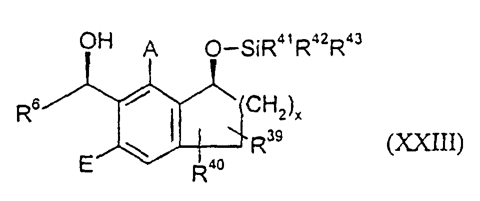 in welcher
   x, A, E, R⁶, R³⁹, R⁴⁰, R⁴¹, R⁴² und R⁴³ die oben angegebene Bedeutung haben,
   gewinnt,
   und gegebenenfalls durch nucleophile Substitution enantioselektiv die Hydroxyfunktion durch einen der oben unter R⁸ aufgeführten Substituenten ersetzt und somit Verbindungen der allgemeinen Formel (XXIV) 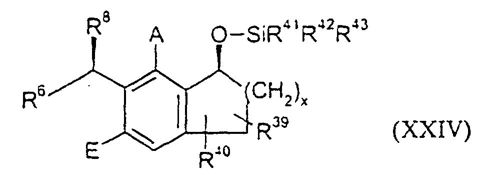 in welcher
   x, A, E, R⁶, R⁸, R³⁹, R⁴⁰, R⁴¹, R⁴² und R⁴³ die oben angegebene Bedeutung haben,
   erhält,
   und in einem letzten Schritt die Hydroxyfunktion unter Abspaltung der Silylschutzgruppe nach üblichen Methoden freisetzt,
   und im Fall der Enantiomere/Racemate nach üblichen Methoden trennt, wobei die Strukturen der allgemeinen Formeln (XV), (XVI), (XVII), (XVIIa) und (XVIII) in isomeren Formen (d.h. Enantiomere, Diastereomere, Regioisomere) auftreten können.

Die erfindungsgemäßen Verfahren können durch folgende Formelschemata beispielhaft erläutert werden:

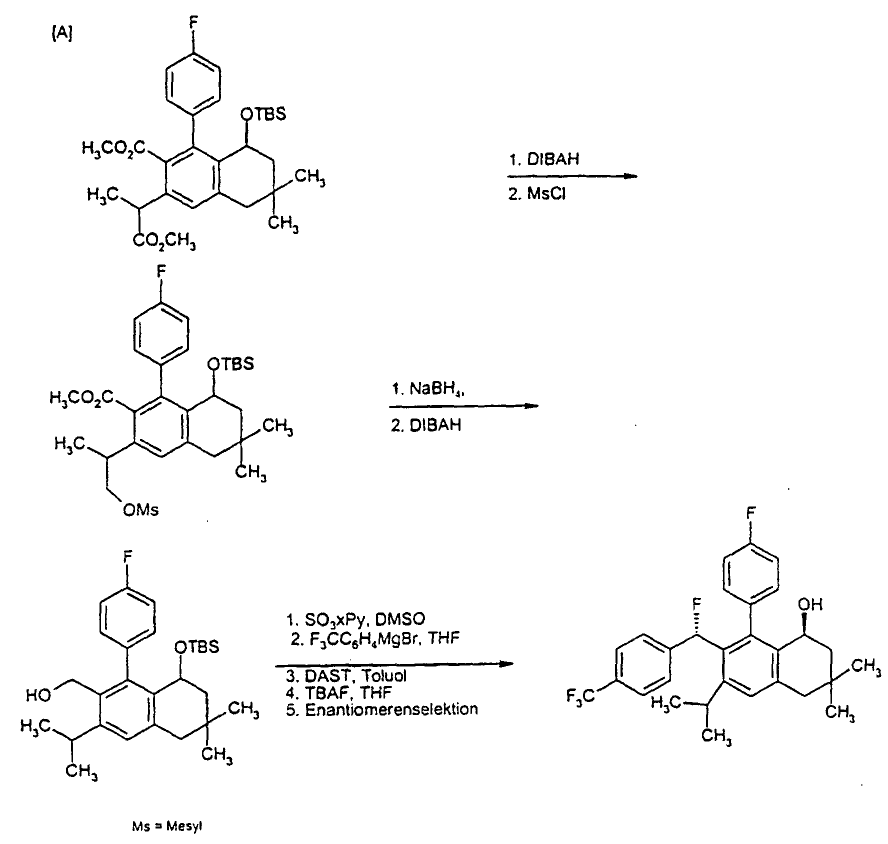

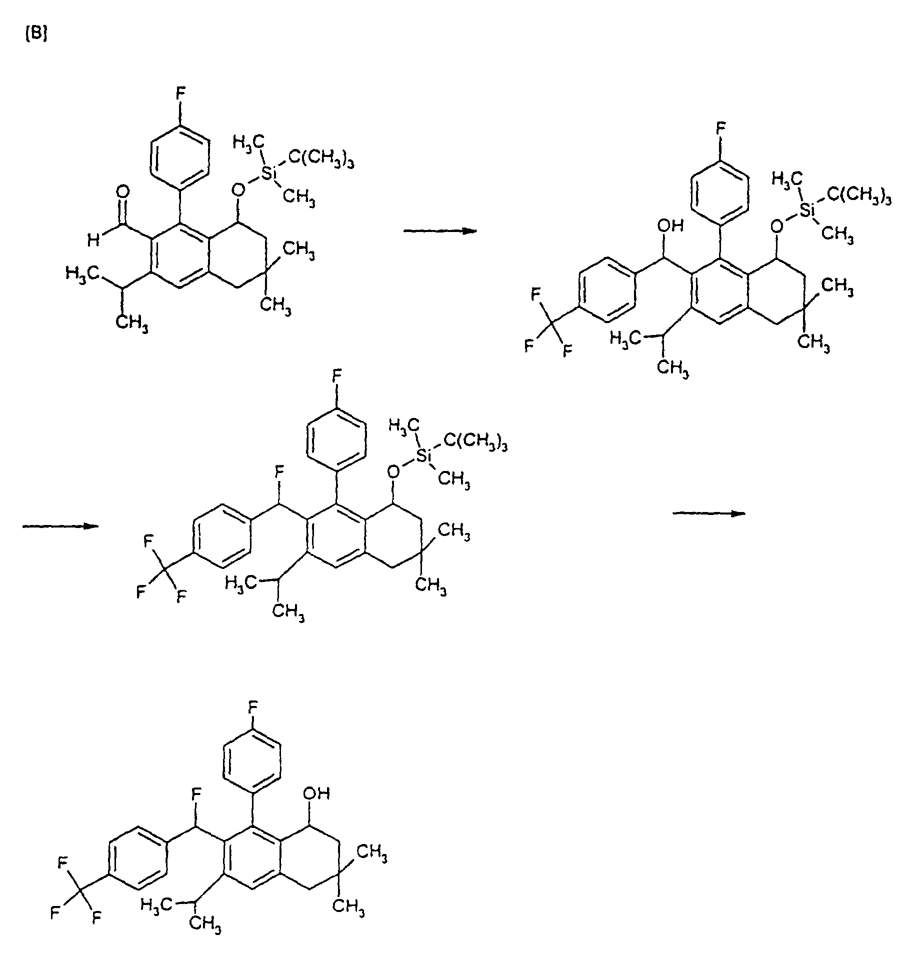

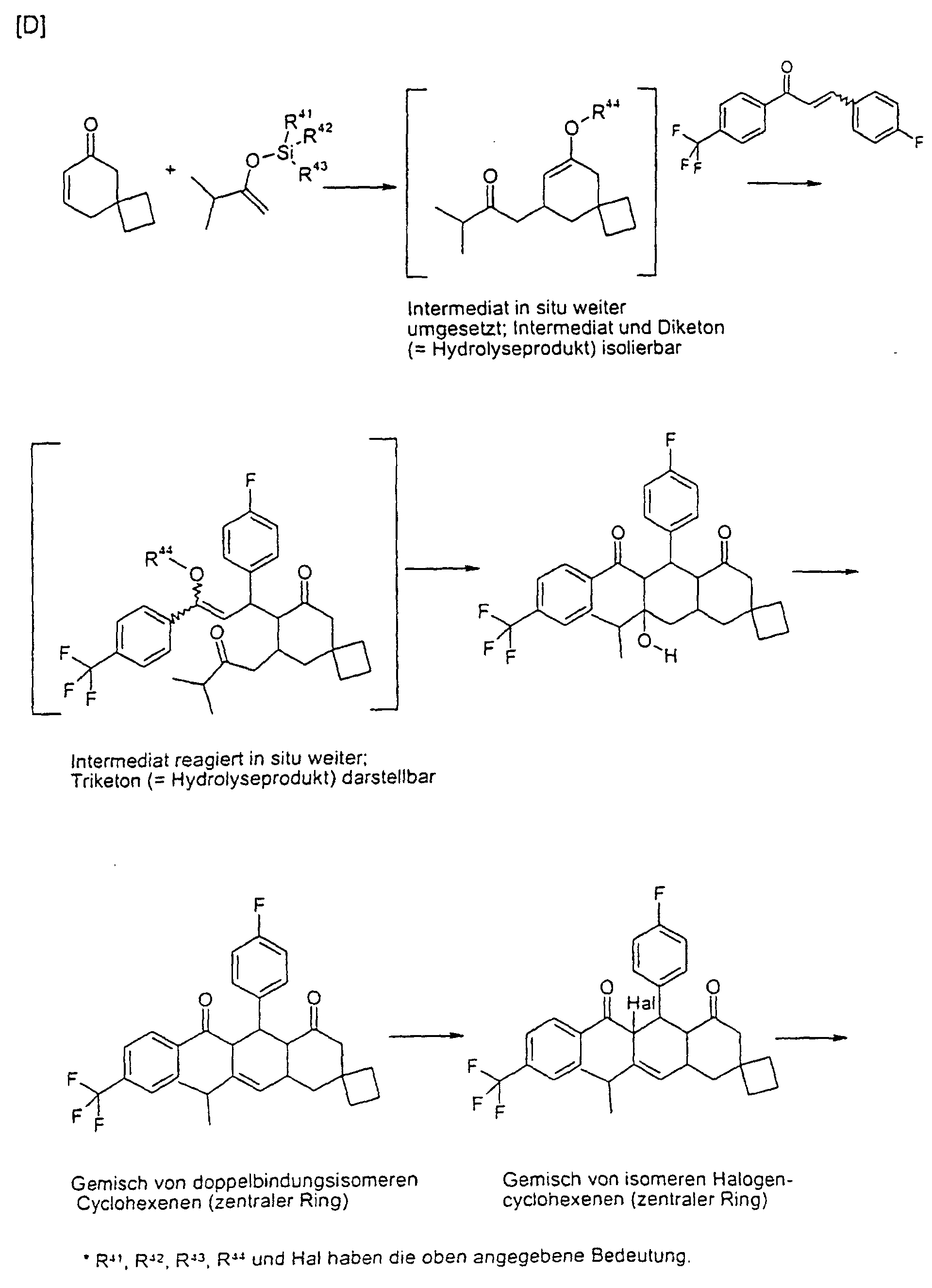

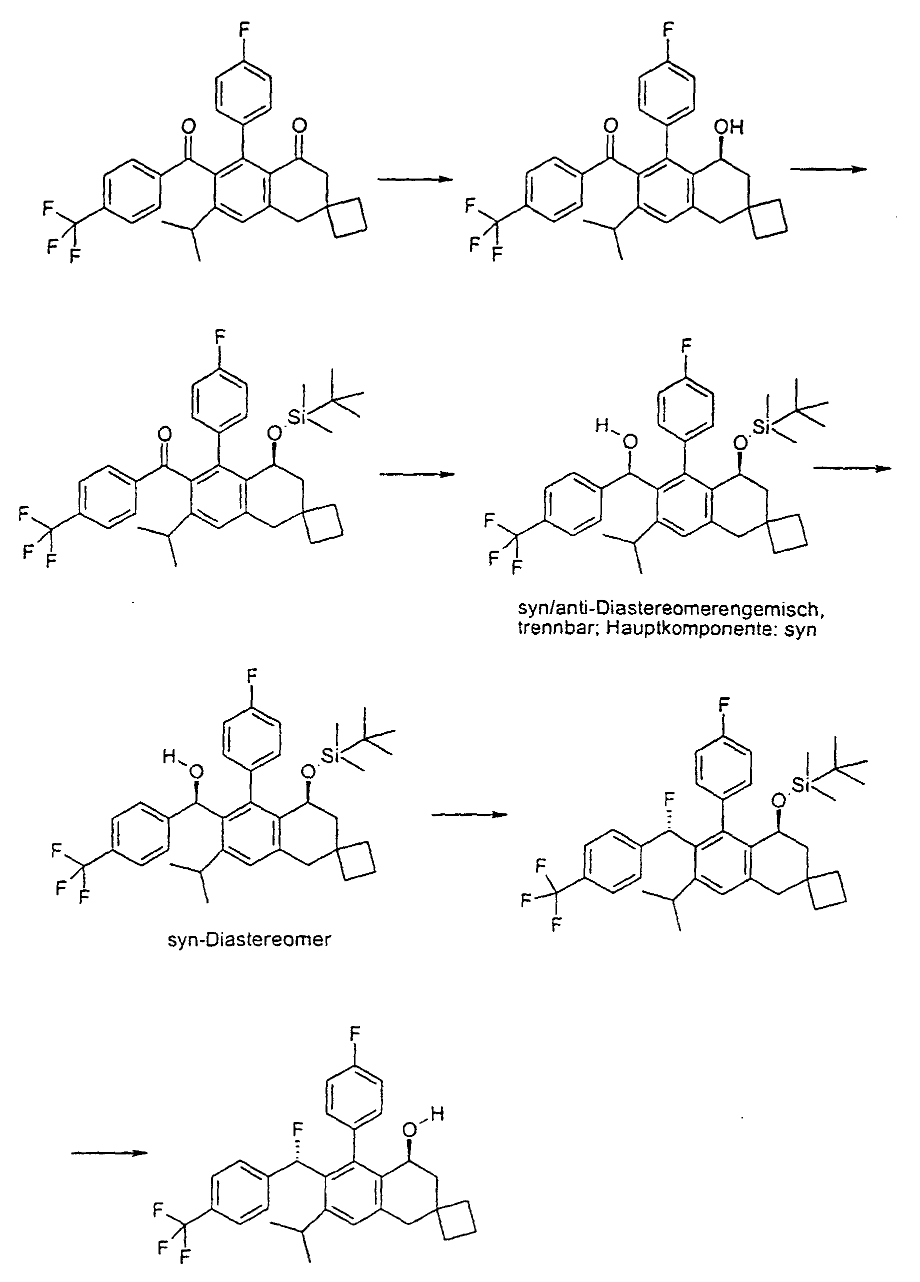

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Toluol und Dichlormethan.

Als Basen kommen für die einzelnen Schritte die üblichen stark basischen Verbindungen in Frage. Hierzu gehören bevorzugt lithiumorganische Verbindungen wie beispielsweise n-Butytlithium, sec.-Butyllithium, tert.Butyllithium oder Phenyllithium, oder Amide wie beispielsweise Lithiumdiisopropylamid, Natriumamid, DBU oder Kaliumamid, oder Lithiumhexamethylsilylamid, oder Alkalihydride wie Natriumhydrid oder Kaliumhydrid. Besonders bevorzugt wird n-Butyllithium, DBU, Natriumhydrid oder Lithiumdiisopropylamid eingesetzt.

Für die Verfahren eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natriumoder Kaliumcarbonat oder Natriumhydrogencarbonat. Besonders bevorzugt wird Natriumhydrid oder Kaliumhydroxid eingesetzt.

Als metallorganische Reagenzien eignen sich beispielsweise Systeme wie Mg/Brornbenzotrifluorid und p-Trifluormethylphenyllithium.

Die Reduktionen der Verbindungen der allgemeinen Formeln (II) und (IV) verlaufen im allgemeinen unter folgenden Reaktionsbedingungen:

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metaithydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH, 0°C bis Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Die Umsetzungen mit den Verbindungen der allgemeinen Formel (III) verlaufen in einem der oben aufgeführen Lösemitteln, vorzugsweise mit Diethylether.

Als Basen eignen sich hierfür im allgemeinen die üblichen stark basischen Verbindungen. Hierzu gehören bevorzugt Di- und Trialkylamine wie beispielsweise Triethylamin oder lithiumorganische Verbindungen wie beispielsweise n-Butyllithium, sec.-Butyllithium, tert.-Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird Triethylamin eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid. oder Alkalicarbonate wie Natrium- oder Kaliumcarbonat oder Natriumhydrogencarbonat. Besonders bevorzugt wird Kaliumhydroxid eingesetzt.

Die Basen werden im allgemeinen in einer Menge von 1 mol bis 4 mol, vorzugsweise 1 mol bis 2 mol bezogen auf 1 mol der Verbindungen der allgemeinen Formel (III) eingesetzt.

Die Umsetzung verläuft im allgemeinen bei einer Temperatur von -30°C bis Raumtemperatur, bevorzugt von -20°C bis 0°C.

Die Umsetzung verläuft im allgemeinen bei Normaldruck, es ist aber auch möglich, bei erhöhtem oder erniedrigtem Druck zu arbeiten.

Die Umsetzung der Verbindungen der allgemeinen Formel (V) erfolgt im allgemeinen unter folgenden Reaktionsbedingungen:

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen kommen für die einzelnen Schritte die üblichen stark basischen Verbindungen in Frage. Hierzu gehören bevorzugt Pyridin oder lithiumorganische Verbindungen wie beispielsweise n-Butyllithium, sec.-Butyllithium, tert.-Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird Pyridin eingesetzt.

Für die Verfahren [B] und [C] eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydrid oder Kaliumhydroxid eingesetzt.

Als metallorganische Reagenzien eignen sich beispielsweise Systeme wie Mg/Brombenzotrifluorid und p-Trifluormethylphenyllithium.

Die Base wird in einer Menge von 0,1 mol bis 5 mol, bevorzugt von 0,5 mol bis 2 mol, jeweils bezogen auf 1 mol der Ausgangsverbindung, eingesetzt.

Die Umsetzung mit Grignard-Reagenzien wird im allgemeinen in einem Temperaturbereich von 0°C bis 150°C, bevorzugt bei 25°C bis 40°C, durchgeführt.

Die Grignard-Reaktionen werden im allgemeinen bei Normaldruck durchgerührt. Es ist aber auch möglich, das Verfahren bei Unterdruck oder bei Überdruck durchzuführen (z.B. in einem Bereich von 0,5 bis 5 bar).

Die Halogenierungen erfolgen im allgemeinen in einem der oben aufgeführten chlorierten Kohlenwasserstoffen und Kohlenwasserstoffen, wobei Methylenchlorid und Toluol bevorzugt sind.

Als Halogenierungsmittel eignen sich beispielsweise Diethylamino-Schwefeltrifluorid (DAST), Morpholino-Schwefeltrifluorid oder SOCl₂.

Die Halogenierung verläuft im allgemeinen in einem Temperaturbereich von -78°C bis +50°C, bevorzugt von -78°C bis 0°C, jeweils in Abhängigkeit von der Wahl des Halogenierungsmittels sowie Lösemittel.

Die Halogenierung verläuft im allgemeinen bei Normaldurck, es ist aber auch möglich, bei erhöhtem oder erniedrigtem Druck zu arbeiten.

Die Abspaltung der Schutzgruppe erfolgt im allgemeinen in einem der oben aufgeführten Alkohole und THF, vorzugsweise Methanol / THF in Anwesenheit von Salzsäure in einem Temperaturbereich von 0°C bis 50°C, vorzugsweise bei Raumtemperatur, und Normaldruck. In besonderen Fällen wird die Abspaltung der Schutzgruppe mit Tetrabutylammoniumfluorid (TBAF) in THF bei Raumtemperatur bevorzugt.

Die Umsetzung der Verbindungen der allgemeinen Formel (VI) erfolgt mit den Verbindungen der allgemeinen Formel (VII) erfolgt in einem der oben aufgeführten Ether, vorzugsweise in Tetrahydrofuran unter Schutzgasatmosphäre in einem Temperaturbereich von -78°C bis -10°C, vorzugsweise bei -25°C.

Die Derivatisierung der Hydroxyfunktion in die Verbindungen der allgemeinen Formel (VIII) erfolgt beispielsweise durch Oxidationen, Sulfonierungen, Alkylierungen, Hydrierungen, Halogenierung, Wittig/Grignard-Reaktionen und Sulfoamidierungen.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird n-Butyllithium, Natriumhydrid oder Lithiumdiisopropylamid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Gegebenenfalls ist es nötig, einige Reaktionsschritte unter Schutzgasatmosphäre durchzuführen.

Die Reduktion der Carbonylgruppe in den Verbindungen der allgemeinen Formel (IX) erfolgt im allgemeinen unter den oben angegebenen Reduktionsbedingungen, vorzugsweise mit Natrium-bis-(2-methoxyethoxy)-dihydroaluminat in Toluol, in einem Temperaturbereich von -20°C bis 140°C, vorzugsweise von 0°C bis 110°C und Normaldruck.

Die Umsetzung der Verbindungen der allgemeinen Formeln (X) und (XI) erfolgt im allgemeinen unter folgenden Bedingungen:

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird N-Butyllithium, Natriumhydrid oder Lithiumdiisopropylamid eingesetzt.

Die Base wird in einer Menge von 0,1 mol bis 10 mol, bevorzugt von 1 mol bis 5 mol, jeweils bezogen auf 1 mol der Ausgangsverbindung, eingesetzt.

Die Umsetzung erfolgt im allgemeinen in einem Temperaturbereich von -10°C bis +10°C, vorzugsweise von 5°C bis 10°C und Normaldruck.

Für die Durchführung des Verfahrens [D] eignen sich folgende Bedingungen:

Die Umsetzung der Verbindungen der Formeln (X), (XI) und (XIII) zu Verbindungen der Formel (XV) kann ohne Isolierung der Zwischenprodukte (XII) und (XIV) erfolgen. Geeignete Reaktionstemperaturen liegen zwischen Raumtemperatur und -20°C. Als Metall- oder Halbmetallreagenzien werden bevorzugt Titanreagenzien, beispielsweise Gemische von Titantetrachlorid und Titantetra-iso-propoxid insbesondere im Molverhältnis 3:1 eingesetzt. Als Lösemittel eignen sich die üblichen inerten Lösemittel, insbesondere Dichlormethan.

Die Eliminierung zu den Verbindungen der Formel (XVI) führt in der Regel zu einem Gemisch der verschiedenen möglichen Doppelbindungsisomere. Die Eliminierung gelingt unter üblichen Bedingungen, bevorzugt wird sie mit Thionylchlorid in Pyridin bei Temperaturen im Bereich -25 bis +25°C, bevorzugt -10 bis +10°C durchgeführt.

Die Halogenierung zu Verbindungen der Formel (XVII) führt ausgehend von dem in der vorhergehenden Stufe erhaltenen Gemisch von Doppelbindungsisomeren wiederum zu einem Gemisch von Isomeren in Bezug auf die Position der Halogensubstituenten und der Doppel- bzw. Einfachbindung. Es werden übliche Reagenzien für die Halogenierung, bevorzugt für allylische Halogenierung sowie gegebenenfalls ein Radikalstarter verwendet. Bevorzugt sind hierfür z.B. N-Chlorsuccinimid, Brom oder N-Bromsuccinimid und N-Bromacetamid. Als Lösemittel eignen sich die üblichen inerten Lösemittel, insbesondere Dichlormethan.

Die anschließende Eliminierung und Oxidation zum Aromaten kann ohne Isolierung der Zwischenprodukte in einem der oben aufgeführten Ether, bevorzugt Dioxan, bei Temperaturen von 0°C bis 150°C, bevorzugt Raumtemperatur bis 120°C, durchgeführt werden.

Für die Aromatisierung werden gegebenenfalls milde Oxidationsmittel wie elementarer Schwefel oder bevorzugt Chalkone eingesetzt. Chalkone sind α,β-ungesättigte Ketone vom Typ C₆H₅-CO-CH=CH-C₆H₅. Die endständigen Phenylreste können jeweils einen oder mehrere Substituenten, ausgewählt aus der Gruppe Halogen, Trifluormethyl, geradkettiges oder verzweigtes Alkyl mit bis zu 4 Kohlenstoffatomen tragen. Bevorzugt wird z.B. das Chalkon 4-CF₃-C₆H₄-CO-CH=CH-C₆H₄-4-F eingesetzt.

Die anschließende Reduktion der Ketogruppe erfolgt positions- und enantioselektiv, beispielsweise mit Boran-Diethylanilin-Komplex unter Zusatz eines chiralen Aminoalkohols wie 1-Aminoindan-2-ol in wasserfreien Ethem, insbesondere THF, unter Schutzgas bei Temperaturen von -70°C bis 50°C.

Die entstandene Hydroxylgruppe wird durch eine Trialkylsilylgruppe, beispielsweise die tert.-Butyldimethylsilyl-Gruppe geschützt. Die Einführung erfolgt über die üblichen aktiven Silylderivate, beispielsweise die Chloride oder die Trifluormethansulfonate (Triflate) in Gegenwart von sterisch gehinderten Basen wie z.B. Lutidin bei Temperaturen von -20°C bis 0°C in inerten Lösemitteln wie Toluol unter Schutzgasatmosphäre.

Die zweite Ketofunktion kann nach üblichen Methoden beispielsweise mit Lithiumaluminiumhydrid in Ethem, insbesondere in THF, bei Temperaturen von 0°C bis 50°C reduziert werden. Aus dem entstehenden Diastereomerengemisch kann gegebenenfalls das gewünschte Diastereomer beispielsweise durch Chromatographie isoliert werden.

Details zu den weiteren möglichen Umsetzungen der Verbindungen der Formel (XXIII) wie z.B. Abspaltung der Silylschutzgruppen oder Einführung eines Fluorsubstituenten in den Substituenten D sind an anderer Stelle angegeben.

Die Verbindungen der allgemeinen Formel (II) sind neu und können beispielsweise hergestellt werden, indem man
Verbindungen der allgemeinen Formel (XXV) 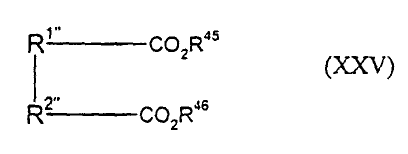 in welcher
- R^{1''} und R^{2''}: für eine geradkettige oder verzweigte Alkylenkette mit bis zu 7 Kohlenstoffatomen stehen,
und
- R⁴⁵ und R⁴⁶: gleich oder verschieden sind und geradkettiges oder verzweigtes Alkyl mit bis zu 4 Kohlenstoffatomen bedeuten,
durch 2-fache Umsetzung gemäß Literatur [M. Yamagushi et al., J. Org. Chem. 55, S. 1611-1623, 1990; vgl. auch z.B. M. Yamagushi, Tetrahedron Lett. 27, 21, 1986, 2401-2404] mit Verbindungen der allgemeinen Formel (XXVI) 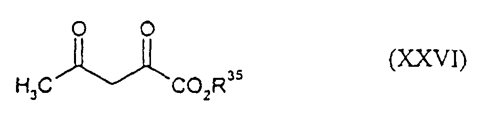 in welcher
- R³⁵: die oben angegebene Bedeutung hat,
in die Verbindungen der allgemeinen Formel (XXVII) 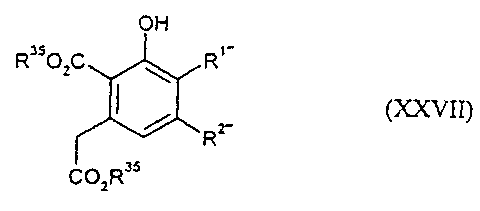 in welcher
R³⁵ die oben angegebene Bedeutung hat
und
R^{1'''} und R^{2'''} die oben angegebene Bedeutung von R^{1''} und R^{2''} haben, wobei die Alkylenkette durch eine Carbonylgruppe substituiert ist, umwandelt
und anschließend durch Einwirkung mit Tf₂O/Pyridin und Umsetzung in einem weiteren Schritt mit Verbindungen der allgemeinen Formel (XXVIII)

A-B(OH)₂ (XXVIII)

in welcher
- A: die oben angegebene Bedeutung hat,
in inerten Lösemitteln und in Anwesenheit eines Palladiumkomplexes in die Verbindungen der allgemeinen Formel (XXIX) 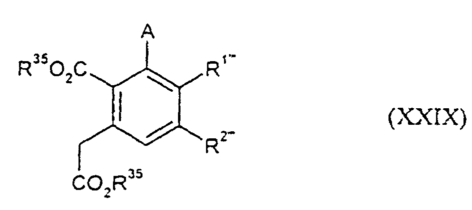 in welcher
R³⁵, R^{1'''} und R^{2'''} die oben angegebene Bedeutung haben,
die Carbonylfunktion zur Hydroxyfunktion reduziert, diese durch Umsetzung mit TBSOTf blockiert und abschließend eine Alkylierung mit Verbindungen der allgemeinen Formel (XXX)

E'''-I (XXX)

in welcher
- E''': den oben angegebenen Bedeutungsumfang von E' und E" umfaßt,
durchführt.

Die Einführung des Substitutenen A erfolgt im allgemeinen in 2 Schritten, wobei zunächst mit Tf₂O in Pyridin umgesetzt wird. In einem weiteren Schritt erfolgt die Umsetzung mit den Verbindungen der allgemeinen Formel (XXVIII)

Als Lösemittel für alle Verfahren eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dioxan.

Als Palladiumverbindungen im Rahmen der vorliegenden Erfindung eignen sich im allgemeinen PdCl₂((C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Die Umsetzung wird im allgemeinen in einem Temperaturbereich von Raumtemperaur bis +150°C, bevorzugt von +40°C bis +110°C, durchgeführt.

Die Umsetzung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Reduktion der Verbindungen der allgemeinen Formel (XIV) erfolgt im allgemeinen in einem der oben aufgeführten Lösemittel, vorzugsweise mit DIBAH.

Die Reduktionen werden im allgemeinen mit Reduktionsmitteln, bevorzugt mit solchen, die für die Reduktion von Ketonen zu Hydroxyverbindungen geeignet sind, durchgeführt. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH, 0°C bis Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Die anschließende Einführung der TBS-Gruppe erfolgt in einem der oben aufgeführten Lösemittel, vorzugsweise mit Toluol, in einem Temperaturbereich von -30°C bis Raumtemperatur, vorzugsweise -20°C bis 0°C und Normaldruck.

Die Alkylierung mit Alkylhalogeniden erfolgt im allgemeinen in inerten Lösemitteln in Anwesenheit einer Base.

Als Lösemittel eignen sich hierbei, je nach Art des Alkylierungsmittels alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Hexamethylphosphorsäuretriamid, oder Gemische der genannten Lösemittel.

Als Basen für die Alkylierung eignen sich die üblichen basischen Verbindungen. Hierzu gehören bevorzugt Alkalihydride wie Natriumhydrid, Alkaliamide wie Natriumamid oder Lithiumdiisopropylamid, Alkalialkoholate wie Natriummethanolat, Natriumethanolat, Kaliummethanolat, Kaliumethanolat oder Kalium-ter.butylat, oder organische Amine wie Trialkylamine, z.B. Triethylamin, oder lithiumorganische Verbindungen wie Butyllithium oder Phenyllithium. Bevorzugt ist Lithiumdiisopropylamid.

Die Alkylierung erfolgt im allgemeinen in einem Temperaturbereich von -70°C bis +80°C, bevorzugt von -70°C bis 0°C.

Die Alkylierung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Die Verbindungen der allgemeinen Formeln (III), (XXV), (XXVI), (XXVII), (XXVIII) und (XXX) sind an sich bekannt oder nach üblichen Methoden herstellbar.

Die Verbindungen der allgemeinen Formel (XXIX) sind neu und können wie oben beschrieben hergestellt werden.

Die Verbindungen der allgemeinen Formel (IV) sind neu und können wie oben beschrieben hergestellt werden.

Die Verbindungen der allgemeinen Formel (VIII) sind neu und können wie oben beschrieben hergestellt werden.

Die Verbindungen der allgemeinen Formel (VI) sind neu und können hergestellt werden, indem man

Verbindungen der allgemeinen Formel (V) 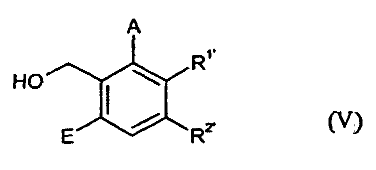 in welcher
A, E, R^{1'} und R^{2'} die oben angegebene Bedeutung haben,
unter Schutzgasatmosphäre in inerten Lösemitteln mit einem der oben aufgeführten Oxidationsmittel in Anwesenheit einer Base, vorzugsweise mit dem SO₃-Pyridin-Komplex umsetzt.

Als Lösemittel für die einzelnen Schritte eignen sich Ether wie Diethylether, Dioxan, Tetrahydrofuran, Glykoldimethylether, Diisopropylether oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol. Ebenso ist es möglich, Gemische der genannten Lösemittel zu verwenden.

Das Oxidationsmittel wird in einer Menge von 1 mol bis 10 mol, bevorzugt von 2 mol bis 5 mol, bezogen auf 1 mol der Verbindungen der allgemeinen Formel (Va), eingesetzt.

Als Basen kommen für die einzelnen Schritte die üblichen basischen Verbindungen in Frage. Hierzu gehören bevorzugt Pyridin oder lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird Pyridin eingesetzt.

Die Base wird in einer Menge von 1 mol bis 10 mol, bevorzugt von 2 mol bis 5 mol bezogen auf 1 mol der Verbindungen der allgemeinen Formel (Va) eingesetzt.

Die Oxidation verläuft im allgemeinen bei einer Temperatur von -50°C bis +100°C, bevorzugt von 0°C bis Raumtemperatur.

Die Verbindungen der allgemeinen Formeln (V), (VIII) und (IX) sind neu und können hergestellt werden, indem man

Verbindungen der allgemeinen Formel (XXXI) 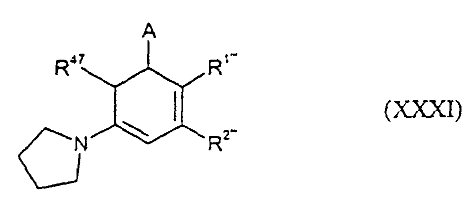 in welcher
A, R^{1'''} und R^{2'''} die oben angegebene Bedeutung haben,
und
- R⁴⁷: entweder für den Rest der Formel 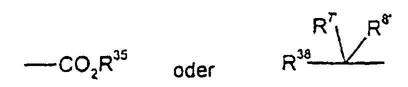 steht,
worin R³⁵, R³⁸, R^{7'} und R^{8'} die oben angegebene Bedeutung haben,
zunächst durch saure Hydrolyse mit wäßriger Zitronensäurelösung in die Verbindungen der allgemeinen Formel (XXXII) 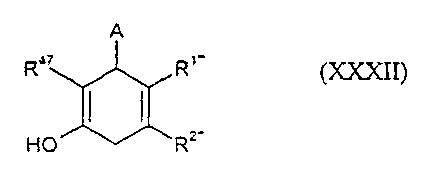 in welcher
A, R¹''',R^{2'''} und R⁴⁷ die oben angegebene Bedeutung haben,
überführt, anschließend in inerten Lösemitteln eine Oxidation zu den Verbindungen der allgemeinen Formel (XXXIII) 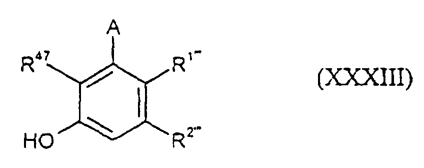 in welcher
A, R¹''',R^{2'''} und R⁴⁷ die oben angegebene Bedeutung haben,
durchführt, in einem weiteren Schritt die Verbindungen der allgemeinen Formel (XXXIV) 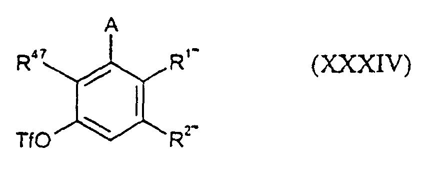 in welcher
A, R¹''',R^{2'''} und R⁴⁷ die oben angegebene Bedeutung haben,
durch Reaktion mit Trifluormethansulfonsäureanhydrid in Pyridin herstellt, anschließend mit Verbindungen der allgemeinen Formel (XXXV)

E^{IV} (XXXV)

in welcher
- E^{IV}: für Cycloalkenyl mit 3 bis 8 Kohlenstoffatomen steht, oder für geradkettiges oder verzweigtes Alkenyl mit bis zu 8 Kohlenstoffatomen steht, oder
für einen Rest der Formel -R⁴⁸-Sn(R⁴⁹R⁵⁰R⁵¹) steht,
in welcher
R⁴⁸ geradkettiges oder verzweigtes Alkenyl mit bis zu 8 Kohlenstoffatomen bedeutet,
R⁴⁹, R⁵⁰ und R⁵¹ gleich oder verschieden sind und geradkettiges oder verzweigtes Alkyl mit bis zu 8 Kohlenstoffatomen bedeuten,
im System Pd(dba)₂/1,2-Bis(diphenylphosphino)ethan, Pd(PPh₃)₄, PdCl₂(PPh₃)₂ und LiCl in Anwesenheit eines inerten Lösemittels und einer Base unter Druck und Schutzaasatmosphäre zu den Verbindungen der allgemeinen Formel (XXXVI) 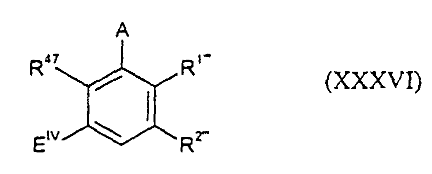 in welcher
A, E^{IV}, R¹''',R^{2'''} und R⁴⁷ die oben angegebene Bedeutung haben,
herstellt, eine Hydrierung in Anwesenheit eines Katalysators zu den Verbindungen der allgemeinen Formel (XXXVII) 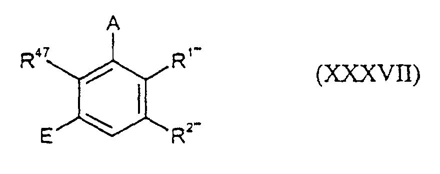 in welcher
A, E, R¹''',R^{2'''} und R⁴⁷ die oben angegebene Bedeutung haben,
durchführt, in einem weiteren Schritt die selektive Reduktion der Carbonylgruppe (R^{1''}/R^{2''}) in Ethem zu den Verbindungen der allgemeinen Formel (XXXVIII) 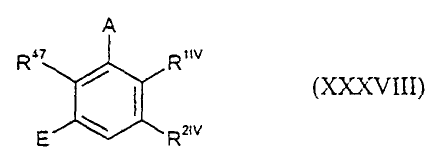 in welcher
A, E und R⁴⁷ die oben angegebene Bedeutung haben
und
- R^{11V} und R^{21V}: die oben angegebene Bedeutung von R^{1'} und R^{2'} haben, wobei die Alkylenkette durch Hydroxy substituiert ist,
durchführt und abschließend durch Schützen die freie Hydroxyfunktion mit tert.-Butyldimethylsilyltriflat (TBS) in inerten Lösemitteln zu den Verbindungen der allgemeinen Formel (XXXIX) 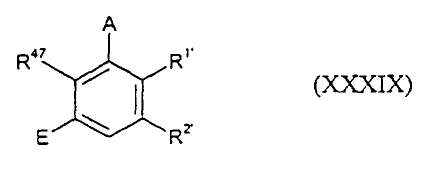 in welcher
A, E, R^{1'}, R^{2'} und R⁴⁷ die oben angegebene Bedeutung haben,
und im Fall R³⁸ = CO₂R³⁵ die Alkoxycarbonylfunktion nach üblichen Methoden zur Hydroxymethylfunktion selektiv reduziert.

Die Umsetzung der Verbindungen der allgemeinen Formel (XVI) erfolgt mit wäßrigen Lösungen gängiger Säuren bei einer Temperatur von 0°C bis 150'°C, bevorzugt mit Carbonsä.uren wie Zitronensäure und Oxalsäure bei 60°C und Normaldruck.

Die Oxidation zu den Verbindungen der allgemeinen Formel (XXXIII) erfolgt im Rahmen der vorliegenden Erfindung unter folgenden Bedingungen:
Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid. Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Toluol.

Als Oxidationsmittel eignen sich beispielsweise Cer(IV)-ammoniumnitrat, 2,3-Dichlor-5,6-dicyan-benzochinon, Pyridiniumchlorochromat (PCC), Pyridiniumchlorochromat auf basischem Aluminiumoxid, Osmiumtetroxid, Natriumacetat/Jod und Mangandioxid. Bevorzugt ist 2,3-Dichlor-5,6-dicyan-benzochinon.

Das Oxidationsmittel wird in einer Menge von 1 mol bis 10 mol, bevorzugt von 2 mol bis 5 mol, bezogen auf 1 mol der Verbindungen der allgemeinen Formel (XXXIII), eingesetzt.

Die Oxidation verläuft im allgemeinen in einem Temperaturbereich von 0°C bis +100°C, bevorzugt von Raumtemperatur bis 80°C.

Die Oxidation verläuft im allgemeinen bei Normaldruck. Es ist aber auch möglich, die Oxidation bei erhöhtem oder erniedrigtem Druck durchzuführen.

Die Einführung der Hydroxyschutzgruppe Trifluormethansulfonyl in die Verbindungen der allgemeinen Formel (XXXIII) erfolgt im allgemeinen in Pyridin mit Trifluormethansulfonsäureanhydrid in einem Temperaturbereich von -30°C bis +40°C, vorzugsweise bei -20°C bis 0°C und Normaldruck.

Die Umsetzung mit den Verbindungen der allgemeinen Formel (XXXV) erfolgt im Falle der Olefine in einer Heckreaktion in einem Autoklaven mit einem Phosphinound Palladiumkomplex, in Anwesenheit eines inerten Lösemittels und einer Base unter Stickstoffatmosphäre, in einem Temperaturbereich von +80°C bis +120°C, vorzugsweise bei 100°C oder im Fall der Alkenylstannylverbindungen bei Raumtemperatur bis 100°C.

Als Lösemittel eignen sich die oben aufgeführten cyclischen Kohlenwasserstoffe und Ether, wobei Toluol und Dioxan bevorzugt sind.

Als Basen eignen sich die oben aufgeführten Dialkylamine, vorzugsweise Ethyldiisopropylamin.

Die Base wird im allgemeinen in einer Menge von 0,5 mol bis 5 mol, vorzugsweise 1 mol bis 3 mol, bezogen auf 1 mol der Verbindungen der allgemeinen Formel (XXXV) eingesetzt.

Als Palladiumverbindungen im Rahmen der vorliegenden Erfindung eignen sich im allgemeinen PdCl₂((C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂), Pd(P(C₆H₅)₃)₄, Pd(PPh₃)₃, Pd (Oac)₂, gegebenenfalls unter Zusatz von Phosphinliganden (z.B. dppe, diphos, dba, Chiraphor, etc.)

Die Reduktion der Doppelbindung in Rest E''' erfolgt im allgemeinen mit einer Hydrierung.

Die Hydrierung erfolgt nach üblichen Methoden mit Wasserstoff in Anwesenheit von Edelmetalkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Die Reduktion zu den Verbindungen der allgemeinen Formel (XXXVIII) erfolgt im allgemeinen in einem der oben aufgeführten Ether, vorzugsweise Tetrahydrofuran in einem Temperaturbereich von 0°C bis +40°C, vorzugsweise 0°C bis Raumtemperatur und Normaldruck.

Als Reduktionsmittel eignen sich im allgemeinen Aluminiumhydride wie LiAlH₄, DIBAH, Red-Al; Borhydride wie NaBH₄, NaCNBH₃, LiBH₄; Boran-Komplexe wie BH₃ x THF, BH₃ x DMS, BH₃ x (Et₂)NC₆H₅ oder Boran-dimethylsulfid-Lösung in Tetrahydrofuran. Bevorzugt ist Boran-dimethylsulfid-Lösung in Tetrahydrofuran. Für stereoselektive Reduktionen können die Borankomplexe mit chiralen Auxiliaren wie chiralen Aminoalkoholen kombiniert werden; bevorzugt ist hier chirales Aminoindanol, z.B. 1R, 2S Aminoindanol.

Die Einführung der TBS-Gruppe zur Blockierung der Hydroxyfunktion erfolgt im allgemeinen in Toluol mit tert.Butyl-dimethylsilyltriflat und einer Base, bevorzugt 2,6-Lutidin, in einem Temperaturbereich von -30°C bis +10°C, vorzugsweise -20°C bis 0°C und Normaldruck.

Die Reduktion der Verbindungen der allgemeinen Formel (XXXIX) erfolgt im allgemeinen in Ether, THF oder in einem der oben aufgeführten cyclischen Kohlenwasserstoffe, vorzugsweise Toluol in einem Temperaturbereich von -78°C bis -20°C, vorzugsweise von -78°C bis -40°C und Normaldruck.

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH. 0°C bis Raumtemperatur im Falle des NaBH₄, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglch bei erhöhtem oder erniedrigtem Druck zu arbeiten.

Die Verbindungen der allgemeinen Formeln (XXXI), (XXXII), (XXXIII), (XXXIV), (XXXVI), (XXXVII), (XXXVIII) und (XXXIX) sind neu und können wie oben beschrieben hergestellt werden.

Die Verbindungen der allgemeinen Formel (XXXV) sind bekannt.

Die Verbindungen der allgemeinen Formel (XXXI) sind neu und können hergestellt werden, indem man Dimedon mit Verbindungen der allgemeinen Formel (XL)

A-CHO (XL)

in welcher
- A: die oben angegebene Bedeutung hat
und Verbindungen der allgemeinen Formel (XLI) 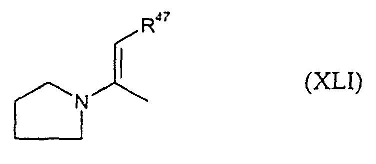 in welcher
R⁴⁷ die oben angegebene Bedeutung hat,
in gesättigten cyclischen Kohlenwasserstoffen, vorzugsweise Cyclohexan, unter Rückfluß und Normaldruck umsetzt.

Die Verbindungen der allgemeinen Formeln (XL) und (XLI) sind bekannt oder nach üblichen Methoden herstellbar.

Die Verbindungen der allgemeinen Formel (X) können in Analogie zu den im folgenden exemplarisch für die Verbindung Spiro[2,5]non-7-en-3-on beschriebenen Verfahren hergestellt werden.

Ausgehend von Cyclobutyl-dimedon(spiro[3,5]nonan-6,8-dion) der Formel (XLII) 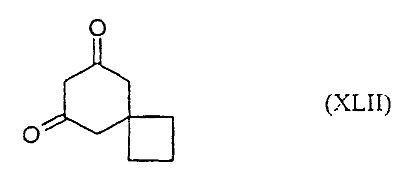 erhält man durch Umsetzung mit p-Toluolsulfonsäure (p-TSS) in Toluol und Isobutanol und anschließender Reduktion mit Red-A1 in Toluol die Verbindung Spiro[2,5]non-7-en-3-on der Formel (XLIII)

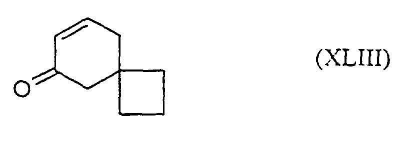

Die Umsetzung mit p-TSS / Isobutanol erfolgt unter Azeotropdestillation.

Die Reaktion erfolgt in einem Temperaturbereich von 0°C bis 60°C, vorzugsweise von 20°C bis 50°C. Beide Reaktionsschritte werden bei Normaldruck durchgeführt.

Die Reaktion kann folgende Zwischenstufen durchlaufen:

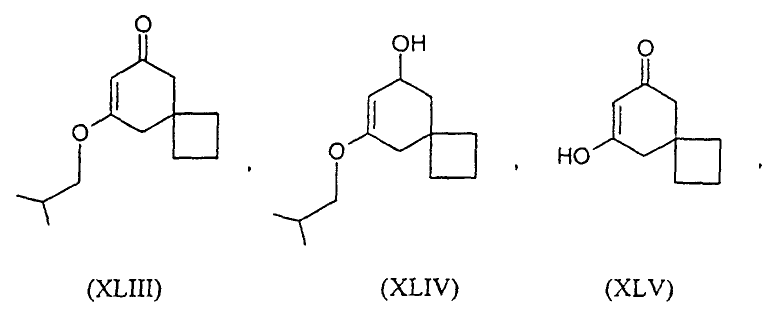

Die Herstellung der Verbindung der Formel (XLII) erfolgt, indem man zunächst durch Umsetzung von Cyclobutanon mit Triphenylphosphoranyliden-2-propanon und Benzoesäure die Verbindung Cyclobutyliden-2-propanon der Formel (XLVI) 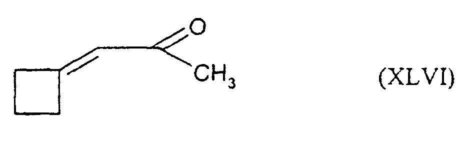 herstellt,
und abschließend mit Malonsäuredimethylester oder Malonsäurediethylester, gegebenenfalls über die Sequenz von nicht isolierten Zwischenstufen der Formeln 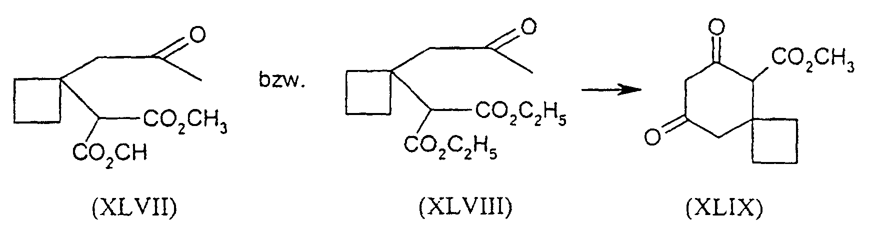 bzw. 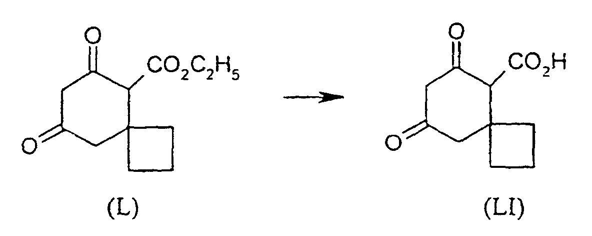 umsetzt,
und abschließend eine Decarboxylierung durchführt.

Die Herstellung der Verbindung der Formel (XLVI) erfolgt in einem Temperaturbereich von 80°C bis 120°C und Normaldruck.

Die Umsetzung mit den entsprechenden Malonsäurediestern erfolgt in Methanol bzw. Ethanol und unter Normaldruck und Rückflußtemperatur.

Die Herstellung der Verbindungen der allgemeinen Formel (XIII) erfolgt durch Umsetzung der Verbindungen der Formel (LII) 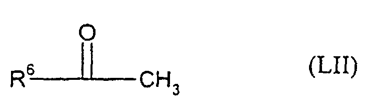 in welcher
- R⁶: die oben angegebene Bedeutung hat
und Verbindungen der allgemeinen Formel (LIII) 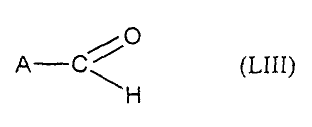 in welcher
- A: die oben angegebene Bedeutung hat,
in Methylcyclohexan und in Anwesenheit von konz. Schwefelsäure unter Rückflußtemperatur und Normaldruck umgesetzt.

Die Verbindungen der allgemeinen Formeln (LII) und (LIII) sind bekannt und nach üblichen Methoden herstellbar.

Die erfindungsgemäßen Verbindungen der allgemeinen Formel (I) haben ein nicht vorhersehbares pharmakologisches Wirkspektrum.

Die erfindungsgemäßen Verbindungen der allgemeinen Formel (I) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den Reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung und Prävention von Hypoalphalipoproteinämie, Dyslipidämien, Hypertriglyceridämien, Hyperlipidämien oder Arteriosklerose eingesetzt werden.

Die pharmakologische Wirkung der erfindungsgemäßen Stoffe wurde in folgendem Test bestimmt:

### CETP-Inhibitions-Testung

### Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 M NaCl/0,001 M TrisHCl pH 7,4 gewaschen und anschließend mit dest. Wasser eluiert. Die CETP-aktiven Fraktionen werden gepoolt, gegen 50mM NaAcetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### Gewinnung von radioaktiv markiertem HDL

50 ml frisches humanes EDTA-Plasma wird mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl ³H-Cholesterin (Dupont NET-725;
1 -µCi/ml gelöst in Ethanol !) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert.

Das Retentat enthält radioaktiv markiertes ³H-CE-HDL, das auf ca. 5x10⁶ cmp pro ml eingestellt zum Test verwendet wird.

### CETP-Test

Zur Testung der CETP-Aktivität wird die Übertragung von ³H-Cholesterolester von humanen HD-Lipoproteinen auf biotinylierte LD-Lipoproteine gemessen.

Die Reaktion wird durch Zugabe von Streptavidin-SPA®beads (Fa. Amersham) beendet und die übertragene Radioaktivität direkt im Liquid Scintillation Counter bestimmt.

Im Testansatz werden 10 µl HDL-³H-Cholesterolester (∼ 50.000 cpm) mit 10 µl Biotin-LDL (Fa. Amersham) in 50 mM Hepes / 0,15 m NaCl / 0,1% Rinderserumalbumin /0,05% NaN₃ pH 7,4 mit 10 µl CETP (1 mg/ml) und 3 µl Lösung der zu prüfenden Substanz (in 10% DMSO / 1 % RSA gelöst), für 18 h bei 37°C inkubiert. Anschließend werden 200 µl der SPA-Streptavidin-Bead-Lösung (TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.

Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle A sind die IC₅₀-Werte (mol/l) für CETP-Inhibitoren angegeben:

**Tabelle A:**

| **Beispiel-Nr.** | **IC**_{**50**}**-Wert (nmol/l)** |
|---|---|
| 25 | 3 |
| 31 | 2,7 |

### Ex vivo Aktivität der erfindungsgemäßen Verbindungen

Syrische Goldhamster aus werkseigener Zucht werden nach 24-stündigem Fasten narkotisiert (0,8 mg/kg Atropin, 0,8 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen.

Die Verabreichung der Testsubstanzen kann auch p.o. erfolgen, indem die Substanzen in DMSO gelöst und 0,5% Tylose suspendiert mittels Schlundsonde peroral verabreicht werden. Die Kontrolltiere erhalten identische Volumen Lösemittel ohne Testsubstanz.

Nach verschiedenen Zeitpunkten - bis zu 24 Stunden nach Applikation - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 x g zentrifugiert. Im so erhaltenen Serum wird die CETP-Aktivität durch den modifizierten CETP-Test bestimmt. Es wird wie für den CETP-Test oben beschrieben die Übertragung von ³H-Cholesterolester von HD-Lipoproteinen auf biotinylierte LD-Lipoproteine gemessen.

Die Reaktion wird durch Zugabe von Streptavidin-SPA^{R}beads (Fa. Amersham) beendet und die übertragene Radioaktivität direkt im Liquid Scintlation Counter bestimmt.

Der Testansatz wird wie unter "CETP-Test" beschrieben durchgeführt. Lediglich 10 µl CETP werden für die Testung der Seren durch 10 µl der entsprechenden Serumproben ersetzt. Als Kontrollen dienen entsprechende Inkubationen mit Seren von unbehandelten Tieren.

Die in den Kontrollansätzen mit Kontrollseren übertragene Aktivität wird als 100% Übertragung gewertet. Die Substanzkonzentration, bei der diese Übertragung auf die Hälfte reduziert ist wird als ED₅₀-Wert angegeben.

**Tabelle B:**

| ED₅₀-Werte für ex vivo Aktivität | | |
|---|---|---|
| Bsp.-Nr. | ED₅₀ | % Hemmung bei 3 mg/kg |
| 25 | < 0,3 mg/kg | 83,1% |
| 31 | < 0,3 mg/kg | 72,3% |

### In vivo Aktivität der erfindungsgemäßen Verbindungen

Bei Versuchen zur Bestimmung der oralen Wirkung auf Lipoproteine und Triglyceride wird syrischen Goldhamstern aus werkseigener Zucht Testsubstanz in DMSO gelöst und 0,5% Tylose suspendiert mittels Schlundsonde peroral verabreicht. Zur Bestimmung der CETP-Aktivität wird vor Versuchsbeginn durch retro-orbitale Punktion Blut entnommen (ca. 250 µl). Anschließend werden die Testsubstanzen peroral mittels einer Schlundsonde verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen.

Durch Inkubation von 4°C über Nacht wird die Gerinnung abgeschlossen, anschließend wird 10 Minuten bei 6000 x zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.

100 µl Serum-Verdünnung werden mit 100 µl Testsubstanz in 96-Lochplatten versetzt und 10 Minuten bei Raumtemperatur inkubiert. Anschließend wird die optische Dichte bei einer Wellenlänge von 492 nm mit einem automatischen Platten-Lesegerät bestimmt. Die in den Proben enthaltene Triglycerid- bzw. Cholesterinkonzentration wird mit Hilfe einer parallel gemessenen Standardkurve bestimmt.

Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt.

**Tabelle C:**

| HDL-Anstieg bei in vivo-Versuchen | | |
|---|---|---|
| Bsp.-Nr. | Dosis [mg/kg] | % HDL-Anstieg |
| 25 | 2 x 1 | + 15 |
| 31 | 2x0,3 | +19 |

### In vivo Wirksamkeit an transgenen hCETP-Mäusen

Transgenen Mäusen aus eigener Zucht (Dinchuck, Hart, Gonzalez, Karmann, Schmidt, Wirak; BBA (1995), 1295, 301) wurden die zu prüfenden Substanzen im Futter verabreicht. Vor Versuchsbeginn wurde den Mäusen retroorbital Blut entnommen, um Cholesterin und Triglyceride im Serum zu bestimmen. Das Serum wurde wie oben für Hamster beschrieben durch Inkubation bei 4°C über Nacht und anschließender Zentrifugation bei 6000 x g gewonnen. Nach einer Woche wurde den Mäusen wieder Blut entnommen, um Lipoproteine und Triglyceride zu bestimmen. Die Veränderung der gemessenen Parameter werden als prozentuale Veränderung gegenüber dem Ausgangswert ausgedrückt.

**Tabelle D:**

| Bsp.-Nr. | HDL | LDL | Triglyceride |
|---|---|---|---|
| 15 (30 ppm) | +60% | -18% | -15% |

Die Erfindung betrifft außerdem die Kombination von substituierten Tetrahydronaphthalinen der allgemeinen Formel (I) mit einem Glucosidase- und/oder Amylasehemmer zur Behandlung von familiärer Hyperlipidaemien, der Fettsucht (Adipositas) und des Diabetes meilitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Weiterhin können die erfindungsgemäßen Verbindungen mit Cholesterin senkenden Vastatinen oder ApoB-senkenden Prinzipien kombiniert werden, um Dyslipidemien, kombinierte Hyperlipidemien, Hypercholesterolemien oder Hypertriglyceridemien zu behandeln.

Die genannten Kombinationen sind auch zur primären oder sekundären Prävention koronarer Herzerkrankungen (z.B. Myokardinfarkt) einsetzbar.

Vastatine im Rahmen der Erfindung sind beispielsweise Lovastatin, Simvastatin, Pravastatin, Fluvastatin, Atorvastatin und Cerivastatin. ApoB senkende Mittel sind zum Beispiel MTP-Inhibitoren.

Bevorzugt ist die Kombination von Cerivastatin oder ApoB-Inhibitoren mit einer der oben aufgeführten erfindungsgemäßen Verbindungen der allgemeinen Formeln (I) und (Ia).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise intravenös, oral, parenteral oder perlingual, insbesondere oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### Methyl-8-hydroxy-7-(methoxycarbonyl)-3,3-dimethyl-1-oxo-1,2,3,4-tetrahydro-6-naphtylacetat

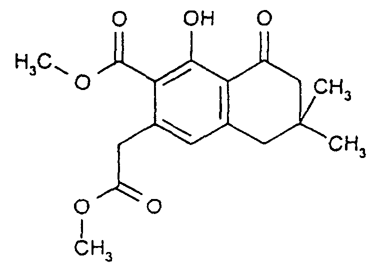

Unter Argon werden 48.48 g Natriumhydrid in 1200 ml THF vorgelegt und unter Eiskühlung bei 8-10° C innerhalb von 35-40 min. 139.92 g Acetessigsäuremethylester, gelöst in 375 ml THF, zugetropft und 1h im Eisbad nachgerührt. Anschließend wieder bei 8-10°C innerhalb von 35-40 min. 761.2 ml n-BuLi in Hexan zugetropft und wieder 1h im Eisbad nachgerührt. Danach 27 g 2-Dimethylglutarsäure-dimethylester, gelöst in 210 ml THF, innerhalb von 15 min. bei 5-7°C zugetropft und 2h nachgerührt, wobei man die Innentemperatur auf Raumtemperatur (RT) ansteigen läßt. Zur Aufarbeitung überführt man die Reaktionslösung in eine gerührte Mischung aus 7,2 Ltr. 10%ige Ammoniumchloridlsg. und 3,6 Ltr. Toluol und rührt noch 10 min. Die organische Phase wird abgetrennt und die wässrige Phase noch einmal mit 1,8 Ltr. Toluol nachextrahiert. Die vereinigten org. Phasen werden 2 mal mit je 1,8 Ltr. Wasser neutral gewaschen, über Natriumsulfat getrocknet und unter Vakuum eingeengt. Der Rückstand (ca.60 g) wird mit 30 ml Toluol versetzt, und unter Rühren läßt man langsam 60 ml Petrolether zulaufen. Nach Animpfen mit Produkt kristallisiert das Produkt aus und wird nach Kühlung auf 10-15°C über 1h abgesaugt und bei 50°C unter Vakuum getrocknet (Frakt.1). Die Mutterlauge wurde eingeengt und auf eine Säule aus 600 g Kieselgel (9,5 X 15 cm) gegeben,und zunächst mit reinem Toluol, später mit steigendem Essigester-Anteil (95:5, 90:10, 80:20) eluiert. Die Hauptfraktion wurde eingegengt und der Rückstand(15 g) mit 7,5 ml Toluol versetzt und unter Rühren 30 ml Petrolether zugegeben, wobei das Produkt auskristallisiert. Die neue Mutterlauge wurde mit einer verunreinigten Fraktion aus der Chromatographie vereinigt, eingeengt (10 g) und mit 5 ml Toluol und 45 ml Petrolether wie oben kristallisiert (Frakt.3).
Ausbeute: 24.9 g (54 % d. Th.)
R_{f} = 0.15 (Cy : EE 8 : 2)

### Beispiel II:

### Methyl-7-(methoxycarbonyl)-3,3-dimethyl-1-oxo-1,2,3,4-tetrahydro-8-trifluormethylsulfonyl-6-naphtylacetat

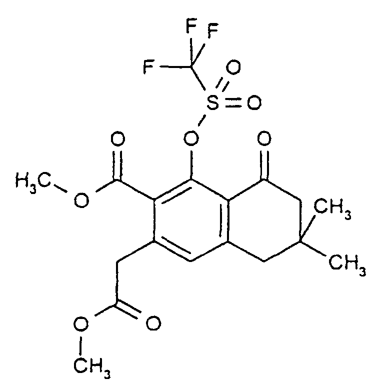

21 g der Verbindung aus Bsp. I werden in 328 ml Pyridin gelöst und 21.52 ml Trifluormethansulfonsäureanhydrid unter Eiskühlung bei max.5°C innerhalb von 30 min. zugetropft. 18h nachgerührt, wobei man die Temperatur auf Raumtemperatur ansteigen läßt. Zur Aufarbeitung wurde die Reaktionslsg. in eine gerührte Mischung aus 1,6 Ltr. Wasser und 0,8 Ltr. Toluol überführt (PH=6) und durch Zutropfen von 325 ml konz.Salzsäure auf PH=2 eingestellt. Die wässr. Phase wurde noch einmal mit 0,4 Ltr. Toluol nachextrahiert und die vereinigten org.Phasen mit je 0,4 Ltr. Wasser, 2,5% iger Natriumhydrogencarbonatlsg. und 2mal Wasser neutral gewaschen, über Natriumsulfat getrocknet und über eine Kieselgelschicht filtriert. Das Filtrat wurde unter Vakuum auf ca.100 ml eingeengt und unter Rühren 100 ml Petrolether zugetropft, wobei das Produkt auskristallisiert. Nach 2-stündiger Kühlung auf 10-15°C wurde das Produkt abgesaugt und bei 50°C unter Vakuum getrocknet.
Ausbeute: 16.6 g (56 % d. Th.)
R_{f} = 0.32 (Cy : EE 6 : 4)

### Beispiel III:

### Methyl-8-(4-fluorphenyl)-7-(methoxycarbonyl)-3,3-dimethyl-1-oxo-1,2,3,4-tetrahydro-6-naphtylacetat

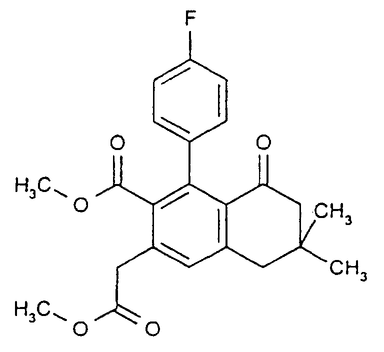

Reaktion unter extremem Ausschluß von Feuchtigkeit und Luftsauerstoff ! 16.5 g der Verbindung aus Bsp. II, 6.63 g 4-Fluorbenzolboronsäure, 13.16 g Trikaliumphosphat und 4.76 g Tetrakis(triphenylphosphin)palladium (0) werden im ausgeheizten Reaktionsgefäß fest vorgelegt, und die Apparatur durch mehrmaliges evakuieren und belüften mit Argon gespült. Danach wurde das luftentgaste Dioxan zugesetzt und 12 h unter Rückfluß gekocht. Nach Abkühlung wurde die Reaktionslsg. über eine Kieselgelschicht (40 g ) filtriert, und das Filtrat unter Vakuum eingeengt. Der Rückstand wurde in 600 ml Toluol gelöst (pH 3 ) und mit je 200 ml Wasser, 5% iger Natriumhydrogencarbonatlsg. und 2 mal Wasser neutral gewaschen, über Natriumsulfat getrocknet und nochmals über eine Kieselgelschicht filtriert. Nach Einengen auf ca. 50 ml und Zutropfen von ca.100 ml Petrolether unter Rühren, kristallisiert das Produkt aus und wurde nach Kühlung auf 10 - 15°C ( 1h ) abgesaugt und bei 50°C unter Vakuum getrocknet ( Frakt. 1 ). Die letzte Kieselgelschicht wurde nochmals mit je 200 ml Toluol / Essigester 90:10 und 80:20 eluiert und der nach Einengen der Lsg. erhaltene Rückstand und der Rückstand der Mutterlauge von Frakt. 1 (2.3g) durch Säulenchromatographie auf 250 g Kieselgel 60 / 0,04-0,063 mm (Säule 5 X 25 cm) mit Cyclohexan / Essigester 80:20 gereinigt.
Ausbeute: 12.8 g (87 % d. Th.)
R_{f} = 0.35 (Cy : EE 6 : 4)

### Beispiel IV:

### Methyl-8-(4-fluorphenyl)-7-(methoxycarbonyl)-3,3-dimethyl-1-hydroxy-1,2,3,4-tetrahydro-6-naphtylacetat

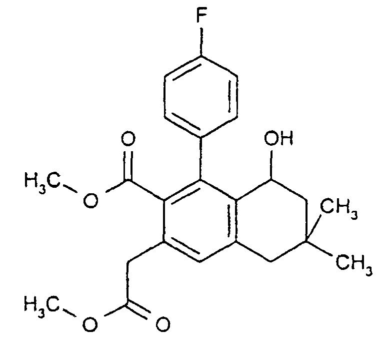

14.1 g der Verbindung aus Bsp. III in Methanol wird unter leichter Erwärmung gelöst, und bei 25°C Natronlauge zugesetzt. Danach 5.36 g Natriumborhydrid eingetragen und 1,5 h bei max.30°C unter leichter Wasserkühlung gerührt bis zur vollständigen Reaktion. Anschließend durch Zutropfen von 150 ml 1N Salzsäure auf pH 3 eingestellt, 15 min. nachgerührt , die Reaktionslsg. in eine Mischung aus 2,4 Ltr. Wasser und 1,2 Ltr. Toluol eingerührt, und die wässrige Phase noch einmal mit 0,6 Ltr. Toluol nachextrahiert. Die vereinigten org. Phasen wurden mit je 0,6 Ltr. 5% iger Natriumhydrogencarbonatlsg. und 2 mal Wasser neutral gewaschen, über Natriumsulfat getrocknet und unter Vakuum auf ca. 50 ml eingeengt. Nach Zutropfen von 100 ml Petrolether unter Rühren kristallisiert das Produkt aus und wird nach Kühlung auf 10-15°C abgesaugt und getrocknet Kristallisat und Mutterlauge wurden wieder vereinigt und auf 870 g Kieselgel 60 / 0,04-0,063mm ( Säule 6,5 X 52 cm ) mit Toluol / Essigester 90:10 chromatographiert. Die Hauptfraktionen wurden unter Vakuum eingeengt und kristallisieren zum Schluß aus Toluol. Die Feststoffe wurden unter Vakuum bei 50°C getrocknet.
Ausbeute: 11.5 g (73 % d. Th.)
R_{f} = 0.20 (Toluol : EE 9 : 1)

### Beispiel V:

### Methyl-8-(4-fluorphenyl)-7-(methoxycarbonyl)-3,3-dimethyl-1-(tert.-butyldimethylsilyloxy)-1,2,3,4-tetrahydro-6-naphtylacetat

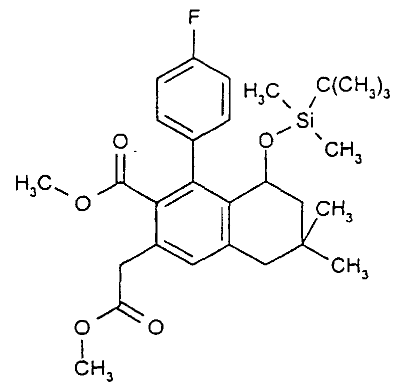

9.9 g der Verbindung aus Bsp. IV werden unter Argon in trockenem Dichlormethan gelöst und auf -20°C gekühlt. Dann werden 7.20 ml 2,6-Lutidin zugetropft und anschließend 7.38 ml t.-Butyldimethyl-silyltrifluormethansulfonat. Danach wird 1h bei -20 bis -15°C bis zur vollständigen Reaktion gerührt.

Zur Aufarbeitung wurden 100 ml ges. Ammoniumchloridlsg. zugetropft, 10 min bei Raumtemperatur verrührt, und die Phasen getrennt. Die wässrige Phase wurde noch 1 mal mit 100 ml Dichlormethan nachextrahiert, und die vereinigten organischen Phasen 2 mal mit je 100 ml Wasser neutral gewaschen, über Natriumsulfat getrocknet und eingeengt. Das Rohprodukt wurde auf 480 g Kieselgel 60 / 0.04 - 0,063 mm ( Säule 5 X 50 cm ) mit Cyclohexan / Essigester 90:10 chromatographiert.
Ausbeute: 10.5 g (83 % d. Th.)
R_{f} = 0.48 (Toluol : EE 9 : 1)

### Beispiel VI:

### 8-(tert.-Butyl-dimethylsilyloxy)-1-(4-fluorphenyl)-3-(1-methoxycarbonyl-ethyl)-6,6-dimethyl-5,6,7,8-tetrahydronaphtalin-2-carbonsäuremethylester

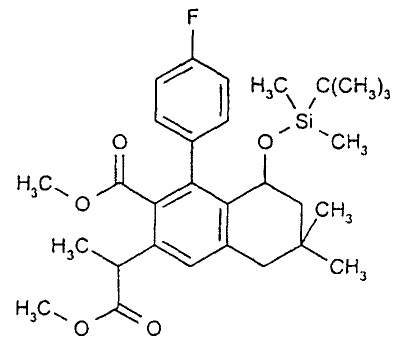

10.3 g der Verbindung aus Bsp. V werden unter Argon in 200 ml trockenem THF gelöst und auf -70°C gekühlt. Anschließend werden 13.98 ml 2 molare LDA-Lsg. innerhalb von 30 min. bei max. -60°C zugetropft, 1h nachgerührt und dabei die Innentemperatur auf -20°C ansteigen lassen. Danach wieder auf -70°C gekühlt, und zunächst 2.12 ml Methyliodid, gelöst in 20 ml THF, bei max. -60°C innerhalb von 30 min. zugetropft. Nach weiteren 30 min. DC-Kontrolle. Zur Aufarbeitung wurde die Reaktionslsg. in eine Mischung aus 1 Ltr. 10 % iger Ammoniumchloridlsg. und 0,5 Ltr. Toluol verrührt, und die Phasen getrennt. Die wässrige Phase wurde noch 1 mal mit 0,25 Ltr. Toluol nachextrahiert und die verreinigten organischen Phasen 2 mal mit je 0,25 Ltr. Wasser neutral gewaschen, über Natriumsulfat getrocknet und unter Vakuum bei 50°C eingeengt. Das Rohprodukt wurde auf 870 g Kieselgel 60 / 0,04 - 0,063 mm (Säule 8 X 52 cm ) mit Cyclohexan / Essigester 90:10 chromatographiert. Die Hauptfraktion wurde unter Vakuum bei 50°C eingeengt und kristallisiert zum Schluß nach Animpfen aus dem Cyclohexan. Der Feststoff wurde bei 50°C unter Vakuum getrocknet.
Ausbeute: 9.4 g (85 % d. Th.)
R_{f} = 0.27 (Cy : EE9 : 1)

### Beispiel VII:

### 8-(tert.-Butyl-dimethylsilyloxy)-1-(4-fluorphenyl)-3-(2-hydroxy-1-methyl-ethyl)-6,6-dimethyl-5,6,7,8-tetrahydronaphtalin-2-carbonsäuremethylester

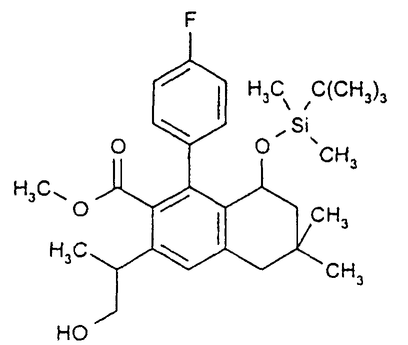

5.24 g der Verbinding aus Bsp. VI wird unter Argon in trockenem THF gelöst vorgelegt, und auf -70°C abgekühlt. Dann wurden 79.26 ml 1 molare Diisobutylaluminiumhydridlsg. innerhalb von 45 min. bei max. -65°C zugetropft und 90 min. nachgerührt. Anschließend wurden 393 ml einer 20% igen Kaliumnatriumtartratlsg. zugetropft, wobei man die Innentemperatur auf Raumtemperatur ansteigen läßt: Nach 60 min. Rühren wurde die Mischung mit 1050 ml Wasser und 525 ml Toluol verdünnt und die Phasen getrennt. Die wässrige Phase wurde noch 1 mal mit 250 ml Toluol nachextrahiert, und die vereinigten organischen Phasen 2 mal mit je 250 ml Wasser neutral gewaschen, über Natriumsulfat getrocknet und unter Vakuum bei 50°C eingeengt.
Ausbeute: 4.25 g (85 % d. Th.)
R_{f} = 0.17 (Cy : EE 8 : 2)

### Beispiel VIII:

### 8-(tert.-Butyl-dimethylsilyloxy)-1-(4-fluorphenyl)-6,6-dimethyl-3-(2-methylsulfonyloxy-1-methyl-ethyl)-5,6,7,8-tetrahydronaphtalin-2-carbonsäuremethylester

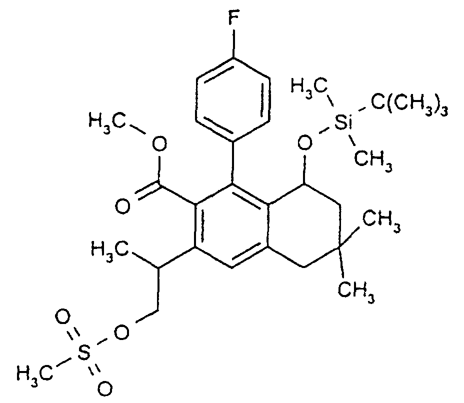

Unter Argon werden 4.25 g der Verbindung aus Bsp. VII in Diethylether gelöst, 3.29 ml Triethylamin zugesetzt und auf -20°C gekühlt. Dann innerhalb von 30 min. 1.71 ml Mesylchlorid bei -25 bis -20°C zugetropft, und 3h bei gleicher Temperatur nachgerührt. Anschließend 31 ml ges. Ammoniumchloridlsg. zugetropft und dabei den Ansatz auf Raumtemperatur kommen lassen (pH 4). Danach die Phasen getrennt, und die wässrige Phase noch 1 mal mit 31 ml Ether nachextrahiert. Die vereinigten org. Phasen wurden mit je 31 ml Wasser,1% iger Natriumhydrogencarbonatlsg. (pH 8) und 2 mal Wasser gewaschen, über Natriumsulfat getrocknet und unter Vakuum bei 50°C eingengt.
Ausbeute: 4.77 g (85 % d. Th.)
R_{f} = 0.28 (Cy : EE 8 : 2)

### Beispiel IX:

### 8-(tert.Butyldimethylsilyloxy)-1-(4-fluorphenyl)-3-isopropyl-6,6-dimethyl-5,6,7,8-tetrahydronaphtalin-2-carbonsäure-methylester

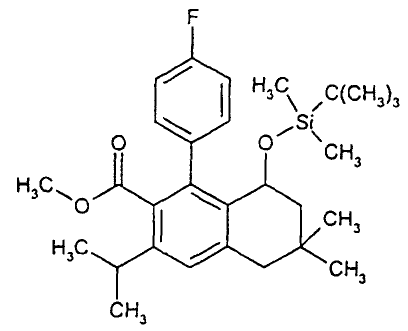

Unter Argon werden 4.7 g der Verbindung aus Bsp. VIII in 69 ml DMSO gelöst und folgend 0.78 g Natriumborhydrid zugesetzt. Anschließend 5h bei 85°C gerührt, bis das Edukt weitgehend umgesetzt ist.

Zur Aufarbeitung wurde die Reaktionslsg. in eine Mischung aus 350 ml 10%iger Natriumchloridisg. und 175 ml Toluol eingerührt, durch langsames Zutropfen von ca. 14 ml 1 M Salzsäure auf pH 5 gestellt und die Phasen getrennt. Die wässrige Phase wurde noch einmal mit 90 ml Toluol nachextrahiert, und die vereinigten org. Phasen mit je 90 ml 5%iger Natriumhydrogencarbonatlsg. und 2 mal Wasser neutral gewaschen, über Natriumsulfat getrocknet und eingeengt. Das Rohprodukt wurde in wenig Toluol gelöst auf eine kurze Kieselgelschicht (60 g) gegeben und mit Toluol eluiert. Hauptfraktion eingeengt und durch animpfen mit Produkt aus wenig Toluol kristallisiert und unter Vakuum bis zur Gewichtskonstanz getrocknet.
Ausbeute: 2.61 g (78 % d. Th.)
R_{f} = 0.30 (Cy : EE 95 : 5)

### Beispiel X:

### 8-(tert. Butyldimethylsilyloxy)-1-(4-fluorphenyl)-2-hydrorymethyl-3-isopropyl-6,6-dimethyl-5,6,7,8-tetrahydronaphthalin

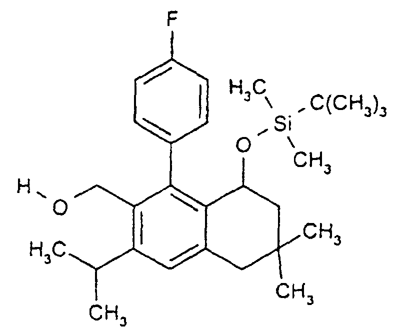

Unter Argon werden 2.57 g der Verbindung aus Bsp. IX in trockenem Toluol gelöst und auf -70°C gekühlt. Anschließend werden innerhalb von 20min. 15.9 ml 1 molare Diisobutylaluminiumhydridlsg. bei max. -65°C zugetropft und 1h bei gleicher Temperatur nachgerührt. Zur Aufarbeitung wurden zuerst 2,0 ml Methanol vorsichtig zugetropft, und nach beendeter Wasserstoffentwicklung 158 ml einer 20%igen Kaliumnatriumtartratlösung, wobei man den Ansatz auf Raumtemperatur kommen läßt. Nachdem noch 30 min. gerührt wurde, trennt man die Phasen und extrahiert die wässr. Phase noch Imal mit 30 ml Toluol. Die vereinigten org. Phasen wurden 2mal mit 30 ml Wasser neutral gewaschen, über Natriumsulfat getrocknet und bei 50°C unter Vakuum eingeengt, wobei das Produkt kristallisiert. Der Feststoff wurde bei 50°C unter Vakuum bis zur Gewichtskonstanz getrocknet.
Ausbeute: 2.30 g (82 % d. Th.)
R_{f} = 0.25 (Cy : EE 9 : 1)

### Beispiel XI:

### 8-(tert.Butyldimethylsilyloxy)-1-(4-fluorphenyl)-3-isopropyl-6,6-dimethyl-5,6,7,8-tetrahydro-naphthalin-2-carbaldehyd

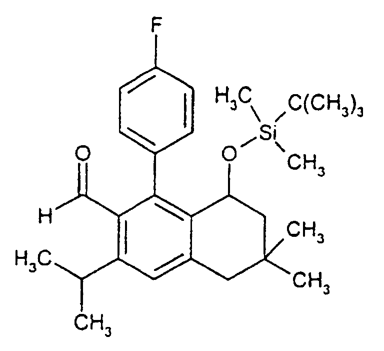

2.32 g der Verbindung aus Bsp. X werden unter Argon in Dichlormethan gelöst, 6.13 ml DMSO und 3.52 ml Triethylamin zugesetzt und auf 5°C abgekühlt. Dann werden 3.23 g Schwefeltrioxid-Pyridin-komplex innerhalb von 10min. bei 5-10°C in kleinen Portionen eingetragen und 1h nachgerührt. Zur Aufarbeitung wird 12 ml Wasser eingerührt und die Phasen getrennt. Anschließend wird die org. Phase noch 2mal mit je 12 ml Wasser gewaschen, über Natriumsulfat getrocknet und unter Vakuum bei 50°C auf ca.3 ml eingeengt. Durch Anreiben und Kühlen kristallisiert das Produkt aus, und der Kristallbrei wird jetzt vollständig eingeengt und unter Vakuum (0.2mb) bei Raumtemperatur bis zur Gewichtskonstanz getrocknet.
Ausbeute: 2.28 g (79 % d. Th.)
R_{f}= 0.57 (Cy : EE 9 : 1)

### Beispiel XII

### 1-(4-Fluor-phenyl)-6,6-dimethyl-8-oxo-3-pyrrolidin-1-yl-1,2,5,6,7,8-hexahydronaphthalin-2-carbonsäure-methylester

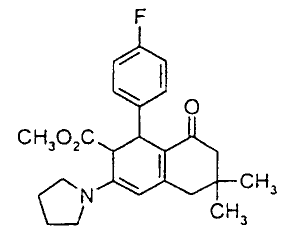

Eine Lösung von 1.69 g (10 mmol) 3-Pyrrolidin-1-yl-but-2-ensäure-methyl ester, 1.40 g (10 mmol) Dimedon und 1.24 g (10 mmol) 4-Fluorbenzaldehyd in 30 ml Cyclohexan wird über Nacht am Wasserabscheider refluxiert. Man kühlt ab, entfernt das Lösungsmittel im Vakuum und reinigt an Kieselgel 60 (Elution mit
EtOAc/Toluol 1:4).
Ausbeute: 0.90 g (23%)
R_{f} = 0.38 (EtOAc/Toluol 1:4).

### Beispiel XIII

### 1-(4-Fluor-phenyl)-3-hydroxy-6,6-dimethyl-8-oxo-1,4,5,6,7,8-hexahydro-naphthalin-2-carbonsäure-methylester

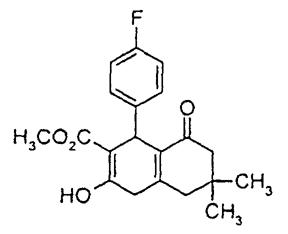

Eine Suspension von 13.0 g (32.7 mmol) der Verbindung aus Beispiel XII in 1100 ml ges. Zitronensäurelösung wird 16h bei 60°C gerührt. Man läßt abkühlen und extrahiert mit Essigester (3 x 600 ml). Nach Waschen der vereinigten organischen Phasen mit ges. NaCl-Lösung und Trocknen über Na₂SO₄ erhält man einen gelben Rückstand, der durch Flash-Filtration über 450 g Kieselgel 60 mit EtOAc/Cyclohexan 1:20 bis 20:1 gereinigt wird.
Ausbeute: 8.97 g (80%)
R_{f} = 0.32 (EtOAc/Petrolether 1:5).

### Beispiel XIV

### 1-(4-Fluor-phenyl)-3-hydroxy-6,6-dimethyl-8-oxo-5,6,7,8-tetrahydro-naphthalin-2-carbonsäure-methylester

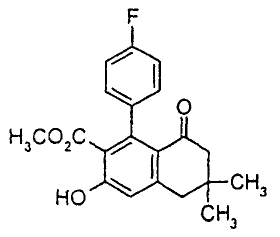

Eine Lösung von 4.0 g (11.6 mmol) der Verbindung aus Beispiel XIII und 13.2 g (58.1 mmol) DDQ in 400 ml Toluol wird 24 h bei 80°C gerührt. Man filtriert das Gemisch über Kieselgur und wäscht mit Toluol nach. Das Filtrat wird im Vakuum eingeengt und über 300 g Kieselgel 60 (CH₂Cl₂) chromatographiert.
Ausbeute: 2.62 g (64%)
R_{f} = 0.35 (CH₂Cl₂)

### Beispiel XV

### 1-(4-Fluor-phenyl)-6,6-dimethyl-8-oxo-3-trifluormethansulfonyloxy-5,6,7,8-tetrahydro-naphthalin-2-carbonsäure-methylester

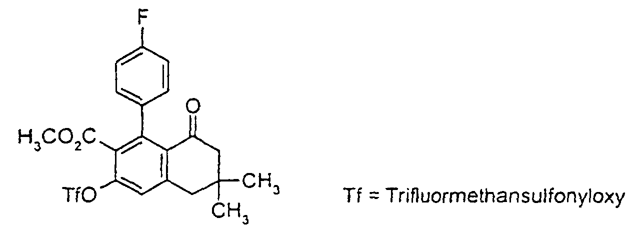

Zu einer Lösung von 3.14 g (9.17 mmol) der Verbindung aus Beispiel XIV in 20 ml Pyridin wird bei 0°C 4.66 g (16.5 mmol) Trifluormethansulfonsäureanhydrid über 5 Minuten zugetropft. Man läßt auf Raumtemperatur erwärmen und rührt 16 h bei dieser Temperatur. Das Reaktionsgemisch wird auf Eiswasser gegossen und mit EtOAc (3x) extrahiert. Man trocknet die vereinigten organischen Phasen über Na₂SO₄, engt im Vakuum ein und reinigt an 500 g Kieselgel 60 (CH₂Cl₂).
Ausbeute: 3.43 g (79%)
R_{f} = 0.50 (CH₂Cl₂).

### Beispiel XVI

### 3-Cyclopent-2-enyl-1-(4-fluor-phenyl)-6,6-dimethyl-8-oxo-5,6,7,8-tetrahydronaphthalin-2-carbonsäure-methylester

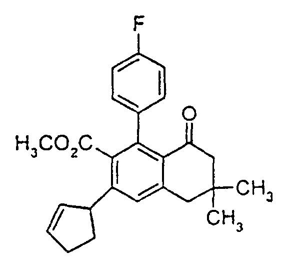

In einem abgedichteten Autoklaven erhitzt man ein Gemisch aus 3.43 g (7.22 mmol) der Verbindung aus Beispiel XV, 1.24 g (2.16 mmol) Pd(dba)₂, 1.29 g (3.24 mmol) 1,2-Bis-(diphenylphosphino)-ethan, 2.5 ml Ethyldiisopropylamin, 6.4 ml Cyclopenten und 65 ml trockenem Toluol unter einer Stickstoffatmosphäre über 16h auf 100°C. Man filtriert über eine Glasfritte mit einem Gemisch aus 5 g Kieselgel 60 und 5 g Kieselgur und engt ein. Das Rohprodukt wird an 500 g Kieselgel 60 chromatographiert (Elution mit CH₂Cl₂).
Ausbeute: 1.48 g (52%)
R_{f} = 0.36 (CH₂Cl₂).

### Beispiel XVII

### 3-Cyclopentyl-1-(4-fluor-phenyl)-6,6-dimethyl-8-oxo-5,6,7,8-tetrahydro-naphthalin-2-carbonsäure-methylester

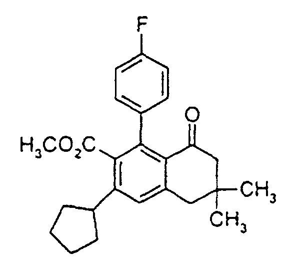

Ein Gemisch aus 1.17 g (2.98 mmol) der Verbindung aus Beispiel XVI und 150 mg Pd/C (10%) in 80 ml EtOAc wird 5h unter Wasserstoffatmosphäre (1 atm) hydriert. Man filtriert über Kieselgur, engt ein und reinigt an Kieselgel 60 (EtOAc/Petrolether 1:10).
Ausbeute: 1.01 g (86%)
R_{f} = 0.23 (EtOAc/Petrolether 1:10).

### Beispiel XVIII

### (8S)-3-Cyclopentyl-1-(4-fluor-phenyl)-8-hydroxy-6,6-dimethyl-5,6,7,8-tetrahydronaphthalin-2-carbonsäure-methylester

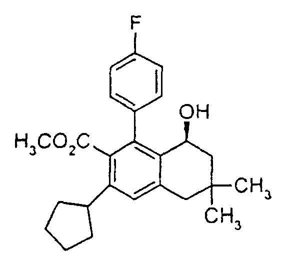

Zu einer Lösung von 59 mg (0.40 mmol) (1R, 2S)-Aminoindanol in 8 ml trockenem THF tropft man 1.75 ml (3.5 mmol) 2.0 M Boran-dimethylsulfid-Lösung in THF und rührt 1 h bei Raumtemperatur nach. Eine Lösung aus 783 mg (1.98 mmol) der Verbindung aus Beispiel XVII in 16 ml trockenem THF wird bei 0-5°C langsam zugetropft. Nach 5 Minuten läßt man auf Raumtemperatur erwärmen und rührt 1 h nach. Die Reaktion wird bei 0°C durch Zugabe von 1.5 ml Methanol abgebrochen, mit Wasser versetzt und mit Toluol extrahiert. Man wäscht die vereinigten organischen Phasen mit ges. NaCl-Lösung, trocknet mit Na₂SO₄ und engt ein. Das Rohprodukt wird an 75 g Kieselgel 60 chromatographiert (Elution: EtOAc/Petrolether/Triethylamin 1:10:0.1).
Ausbeute: 769 mg (97%), ee = 89.7 % (Chiralcel AD, Heptan/Ethanol 90:10)
R_{f} = 0.33 (Ether/Petrolether 1:5).

### Beispiel XIX

### (8S)-8-(tert-Butyl-dimethyl-silyloxy)-3-cyclopentyl-1-(4-fluor-phenyl)-6,6-dimethyl-5,6,7,8-tetrahydro-naphthalin-2-carbonsäure-methylester

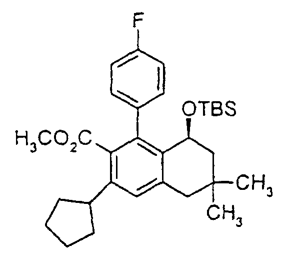

Zu einer Lösung aus 850 mg (2.14 mmol) der Verbindung aus Beispiel XVIII und 1.84 g (17.1 mmol) 2,6-Lutidin in 10 ml trockenem Toluol tropft man bei -16°C eine Lösung aus 1.70 g (6.43 mmol) *tert*-Butyl-dimethylsilyltriflat, rührt 30 Minuten bei dieser Temperatur und weitere 30 Minuten bei 0°C. Man quencht mit 5 ml Wasser und extrahiert mit EtOAc. Die vereinigten organischen Phasen werden mit ges. NaHCO₃-Lösung und ges. NaCl-Lösung gewaschen, über Na₂SO₄ getrocknet und im Vakuum eingeengt. Das Rohprodukt wird an Kieselgel 60 gereinigt (Elution: EtOAc/Cyclohexan/Triethylamin 1:10:0.1).
Ausbeute: 1.05 g (96%)
R_{f} = 0.63 (EtOAc/Petrolether 1:10).

### Beispiel XX

### (8S)-[8-(tert-Butyl-dimethyl-silyloxy)-3-cyclopentyl-1-(4-fluor-phenyl)-6,6-dimethyl-5,6,7,8-tetrahydro-naphthalin-2-yl]-methanol

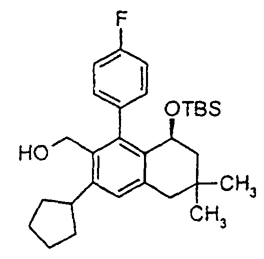

Zu einer Lösung von 1.13g (2.21 mmol) der Verbindung aus Beispiel XIX in 25 ml trockenem Toluol gibt man bei -78°C 6.2 ml (9.28 mmol) einer 1.5 M Lösung von Diisobutylaluminium-hydrid (DIBAH) in Toluol langsam zu und rührt 1 h bei dieser Temperatur. Nach weiteren 30 Minuten bei -40°C läßt man auf 0°C erwärmen und hydrolysiert mit 20 ml einer 20%igen K⁺/Na⁺-Tartratlösung. Man filtriert das Gemisch über Kieselgur und wäscht mit EtOAc und Wasser nach. Nach Abtrennen der wäßrigen Phase wird diese mit EtOAc reextrahiert und die vereinigten organischen Phasen werden mit ges. NaCl-Lösung gewaschen. Man trocknet über Na₂SO₄ und engt ein. Das Rohprodukt wird direkt weiter umgesetzt.
Ausbeute: 1.01 g (94%)
R_{f} = 0.42 (EtOAc/Petrolether 1:10).

### Beispiel XXI

### (8S)-(tert-Butyl-dimethyl-silyloxy)-3-cyclopentyl-1-(4-fluor-phenyl)-6,6-dimethyl-5,6,7,8-tetrahydro-naphthalin-2-carbaldehyd

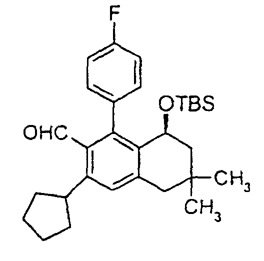

Zu einer Lösung aus 972 mg (2.01 mmol) der Verbindung aus Beispiel XX, 1.40 ml (10.1 mmol) Triethylamin und 2.5 ml Dimethylsulfoxid in 10 ml trockenem Dichlormethan gibt man bei 0°C im Argongegenstrom 1.28 g (8.05 mmol) Schwefeltrioxid-Pyridin-Komplex portionsweise über 5 Minuten zu. Man rührt 1 h bei 0-5°C, gibt 20 ml 5% NaHCO₃-Lösung hinzu und extrahiert mit EtOAc. Die vereinigten organischen Phasen werden mit 5% NaHCO₃-Lösung und ges. NaCl-Lösung gewaschen, über Na₂SO₄ getrocknet und eingeengt. Man reinigt durch Flash-Chromatographie an 100g Kieselgel 60 (Elution: EtOAc/Cyclohexan/Triethylamin 1:10:0.1).
Ausbeute: 840 mg (87%)
R_{f} = 0.60 (EtOAc/Petrolether 1:10).

### Beispiel XXII

### 8-(4-Fluorphenyl)-6-hydroxy-6-isopropyl-7-(4-trifluormethylbenzoyl)-3-spirocyclobutyl-decalon-1

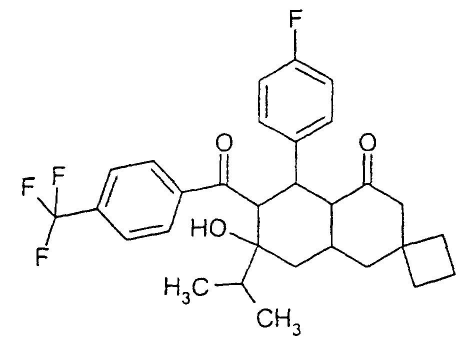

In eine Lösung von 11,36 g (71,78 mmol) Vinyl-isopropyl-silylenolether und 8,15 g (59,81 mmol) 5-Spiro[3,5]non-7-en-6-on (Beispiel XXVIII) in 40 ml Dichlormethan werden unter Argon und Eis/Aceton-Kühlung möglichst gleichzeitig 34,67 g (20,04 ml; 182,77 mmol) Titantetrachlorid und 17,32 g (17,98 ml; 60,92 mmol) Titantetra-i-Propoxid eingetropft, wobei die Temperatur zwischen -7°C und -1°C gehalten wird (ca. 30 Min.). Die Lösung färbt sich dunkelbraun. Es wird 5 Minuten nachgerührt und mit 17,60 g (59,81 mmol) 1-(4-Fluorphenyl)-3-(4-trifluormethylphenyl)-propen-3-on (Beispiel XXIX) (fest) versetzt. Dabei steigt die Temperatur von -7°C auf +3°C an. Es wird 5 Minuten unter Kühlung, dann über Nacht bei RT nachgerührt. Unter Eiskühlung wird tropfenweise mit 10 ml 1 N HCl versetzt. Die Phasen werden getrennt, die organische Phase wird noch einmal mit 1 N HCl und zweimal mit Wasser gewaschen, getrocknet und eingeengt. Die Kristallisation erfolgt mit
Petrolether.
Ausbeute: 13, 100 g (42,4% d. Th.)
Fp.: 236-238°C

### Beispiel XXIII

### Gemisch der verschiedenen 8-(4-Fluorphenyl)-6-isopropyl-7-(4-trifluormethylbenzoyl)-3-spirocyclobutyl-octahydronaphthalin-1-one

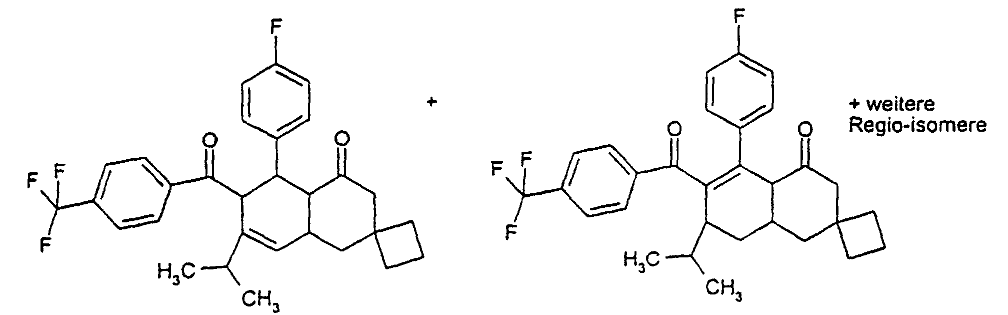

20 g (38,7 mmol) der Verbindung aus Beispiel XXII werden in 316 ml Pyridin vorgelegt und auf 0°C gekühlt. Innerhalb von 15 Minuten tropft man 33,7 ml Thionylchlorid bei 0°C dazu. Eine DC-Kontrolle nach 10 Minuten (Petrolether/-Essigester = 4:1) zeigt eine vollständige Umsetzung. Das Reaktionsgemisch wird bei 5-10°C mit 480 ml Essigester versetzt und 190 ml 4 N HCl werden tropfenweise zugegeben. Die organische Phase wird abgetrennt, die wäßrige Phase noch zweimal mit Essigester extrahiert. Die Essigester-Phasen werden vereinigt, noch einmal mit 2 N HCl (wäßrige Phase muß sauer sein) und zweimal mit Wasser gewaschen, getrocknet und eingeengt. Der Rückstand wird mit 80 ml Petrolether für 10 Minuten digeriert, abgesaugt und getrocknet.
Ausbeute: 16,0 g (82,9% d.Th.)

### Beispiel XXIV

### Gemisch chlorierter 8-(4-Fluorphenyl)-6-isopropyl-7-(4-trifluormethyl-benzoyl)-3-spirocyclo-butyl-octahydronaphthalin-1-one

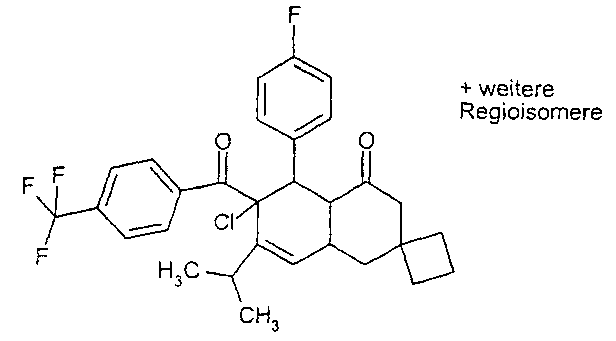

Zu 8,0 ml Dichlormethan gibt man 0,81 g (1,621 mmol) der Verbindung aus Beispiel XXIII, 0,97 g (7,296 mmol) N-Chlor-succinimid, 0,03 g Bisbenzoylperoxyd und rührt 3 Tage bei RT. Man versetzt mit 38,15 ml Dichlormethan, wäscht mit 38,15 ml Wasser, trocknet die organische Phase mit Na₂SO₄, filtriert, wäscht mit Dichlormethan nach und engt bei 50°C unter vermindertem Druck ein. Das Produkt wird durch Flash-Chromatographie an 25 g Kieselgel (Petrolether/Essigester = 10:1) gereinigt.
Ausbeute: 0,822 g (84,6% d. Th.)
MS (DCI/NH₃): = 550 (M+18)

### Beispiel XXV

### 5-Spirocyclobutyl-cyclohexandion-mono-isobutylether

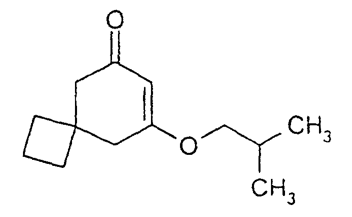

Zu 85 g (0,558 mol) 5-Spirocyclobutyl-cyclohexandion in 1,8 1 Toluol gibt man 62,10 g (0,838 mol) Isobutanol und 3,4 g (0,02 mol) p-Toluolsulfonsäure und erhitzt unter Rühren für 24 h am Wasserabscheider. Es wird abgekühlt, mit gesättigter Natriumhydrogencarbonat-Lösung und Wasser gewaschen, getrocknet und eingeengt. Man erhält 121,1 g Öl mit einer Reinheit von 98,55% nach HPLC.

### Beispiel XXVI

### Cyclobutyliden-2-propanon

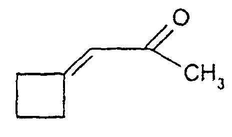

1,15 kg Triphenylphosphoranyliden-2-propanon werden in 1,8 l 100°C heißes Siliconöl gegeben und 42 g Benzoesäure und 242 g Cyclobutanon zugesetzt. Bei ca. 110°C wird 12 Stunden gerührt und anschließend das Produkt im Wasserstrahlvakuum abdestilliert.
Kp₁₀ = 62 bis 65°C.
Ausbeute: 330 g entsprechend 86,5 % d. Th. (NMR, CDCl3: 2,1 ppm t '(2H); 2,2 ppm s (3H); 2,9 und 3,2 tr (je 2 H); 5,95 ppm m (1H)

### Beispiel XXVII

### Cyclobutyl-dimedon (Spiro[3,5]nonan-6,8-dion)

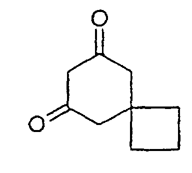

500 ml 30 %iges NaOMe in Methanol werden vorgelegt und mit 640 ml Methanol verdünnt. Bei ca. 60°C werden hierzu 359 g Malonsäuredimethylester gegeben und 10 Minuten auf Rückfluß erhitzt. Anschließend werden 300 g Cyclobutyliden-2-propanon zugesetzt und 4 Stunden unter Rückfluß erhitzt. Zur Verseifung werden 336 g KOH gelöst in 1600 ml Wasser zugesetzt und 1 Stunde unter Rückfluß erhitzt. Anschließend wird mit 20 %iger Salzsäure angesäuert und bei pH 3 bis 5 bis zum Ende der CO₂-Entwicklung gerührt. Nach Destillation des Methanols wird auf Raumtemperatur kaltgerührt und der ausgefallene Feststoff isoliert und neutralgewaschen und bei 55°C im Vakuum getrocknet.
Ausbeute: 412 g entsprechend 99,4 % d. Th. (NMR, DMSO, 1,7-1,95 ppm m (6H); 2,4 ppm s (4H), 5,2 ppm s (1H); 11,1 ppm br.s (-OH)

### Beispiel XXVIII

### Spiro[3,5]non-7-en-6-on (ohne Zwischenisolierung des Enolethers)

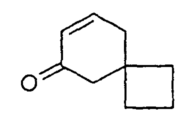

492,2 g Cyclobutyl-Dimedon werden in 1.7 l Toluol gegeben und mit 264 g Isobutanol und 3,7 g p-TSS versetzt. Nach ca. 5stündiger Azeotropdestillation ist die Umsetzung zum Enol vollständig. Die Reaktionsmischung wird auf 20°C gekühlt und mit 1 006 g 65 %igem Red-Al in Toluol versetzt. Hierbei erwärmt sich die Reaktionsmischung auf 40 bis 50°C. Bei dieser Temperatur wird ca. 2 Stunden bis zum vollständigen Umsatz gerührt. Anschließend werden 310 g KOH in 1 250 g Wasser zugegeben, die untere wäßrige Phase abgetrennt und die Toluolphase mit 364 g konzentrierter Salzsäure in 980 g Wasser versetzt. Nach 30 minütigem Nachrühren werden die Phasen getrennt und das Toluol im Vakuum abdestilliert. Der Rückstand wird im Wasserstrahlvakuum fraktioniert.
Ausbeute: 340 g Kp 10=92 bis 94°C (77 % d. Th.) /NMR, DMSO; 1,7 bis 1,9 ppm m (6H); 2,5 ppm s+d (4H); 5,9 + 6,9 (je 1H)

Die Herstellung des entsprechenden Enolethers ist unter Beispiel XXV beschrieben.

### Beispiel XXIX

### 1-(4-Fluorphenyl)-3-(4-trifluormethylphenyl)-propen-3-on

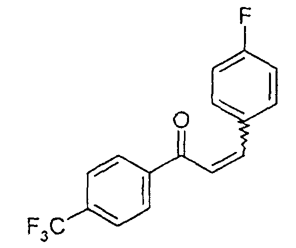

8 1 Methylcyclohexan werden vorgelegt und 3 000 g 4-Fluorbenzaldehyd, 5 000 g 4-Trifluormethylacetophenon und 150 ml konz. Schwefelsäure zugegeben. Es wird ca. 8 Stunden azeotropiert bis zum Ende der Wasserabscheidung. Nach Abkühlen auf 10 bis 15°C wird der ausgefallene Feststoff abgesaugt und mit kaltem Methylcyclohexan gewaschen.
Nach Trocknen im Vakuum werden 6 756 g Produkt isoliert (95 % d. Th.) (NMR, CDCl₃, 7,1 bis 8,2 ppm m (4 + 2 + 4H)

### Herstellungsbeispiele:

### Beispiel 1 und Beispiel 2

### 5-(tert. Butyldimethylsilyloxy)-7,7-dimethyl-4-(4-fluorphenyl)-3-[hydroxy-(4-trifluormethyl-phenyl)-methyl]-2-isopropyl-5,6,7,8-tetrahydronaphthalin

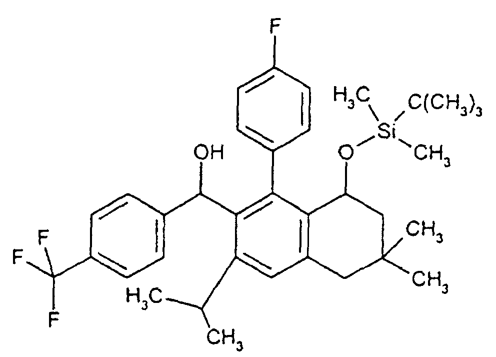

2.28 g der Verbindung aus Bsp. XI werden unter Argon in THF gelöst und auf 5°C abgekühlt. Dann werden 59.85 ml einer THF-Lsg. die 3 eq 4-Trifluormethylphenylmagnesiumbromid enthält innerhalb von 30 min. bei 5-10°C zugetropft und 1h nachgerührt. Zur Aufarbeitung werden zunächst 30 ml einer ges. Ammoniumchloridlsg. zugetropft, 10 min. nachgerührt, dann den Ansatz in eine Mischung aus 500 ml Wasser und 250 ml Toluol eingerührt, und die Phasen getrennt. Die wässrige Phase wurde noch einmal mit 125 ml Toluol nachextrahiert und die vereinigten org. Phasen 2 mal mit je 125 ml Wasser neutral gewaschen, über Natriumsulfat getrocknet und bei max. 50°C unter Vakuum eingeengt. Das Rohprodukt wurde auf 250 Kieselgel 60/0,04-0,063 mm (Säule 5X25 cm) mit Cyclohexan/Essigester 95:5 chromatographiert und die Hauptfraktionen fast vollständig eingeengt. Die beiden Diastereomeren (Diastereomeres A u. B) kristallisieren nach Anreiben und Kühlen aus den Cyclohexanresten aus und werden dann im Vakuum bis zur Gewichtskonstanz getrocknet.
Ausbeute: 1.29 g Diastereomeres A (52 % d. Th.) (Beispiel 1);
Ausbeute: 1.01 g Diastereomeres B (42 % d. Th.) (Beispiel 2)
R_{f} = 0.40 Diastereomeres A (Cy : EE 9 : 1) (Beispiel 1);
R_{f} = 0.33 Diastereomeres B (Cy : EE 9 : 1) (Beispiel 2)

### Beispiel 3:

### 5-(tert.-Butyldimethylsilyloxy)-7,7-dimethyl-4-(4-fluorphenyl)-3-[fluor-(4-trifluormethyl-phenyl)-methyl]-2-isopropyl-5,6,7,8-tetrahydronaphthalin

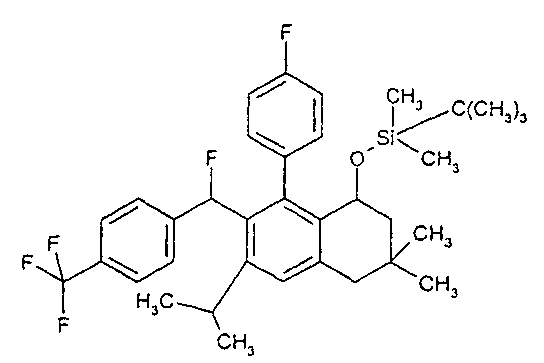

Unter Argon werden 0.8 g der Verbindung aus Bsp. 2 (Diastereomeres B) in Toluol gelöst und auf -70°C abgekühlt. Anschließend werden 0.26 ml Diethylaminoschwefeltrifluorid innerhalb von 5 min. zugetropft und 60 min. gerührt, wobei man die Innentemperatur bis auf -60°C ansteigen läßt. Zur Aufarbeitung werden 5 ml ges. Natriumhydrogencarbonatlsg. zugetropft, 10 min. gerührt und die Phasen getrennt. Die org. Phase wurde noch 2 mal mit je 5 ml Wasser neutral gewaschen, über Natriumsulfat getrocknet und bei 50°C unter Vakuum eingeengt. Das Rohprodukt kristallisiert, es wurde unter Vakuum bis zur Gewichtskonstanz getrocknet und ohne Reinigung in die nächste Stufe eingesetzt.
Ausbeute: 0.78 g (83 % d. Th.)
R_{f} = 0.65 (Cy : EE 9 : 1)

### Beispiel 4 und Beispiel 5

### [1S,7(1RS)]-1-(tert-Butyl-dimethyl-silylory)-6-cyclopentyl-8-(4-fluor-phenyl)-7-[hydroxy-(4-trifluormethyl-phenyl)-methyl]-3,3-dimethyl-1,2,3,4-tetrahydronaphthalin

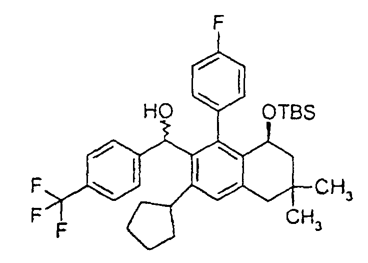

Zu einer Lösung von 830 mg (1.73 mmol) der Verbindung aus Beispiel XXI in 10 ml trockenem THF gibt man unter Argon bei -25°C 33.5 ml einer 0.224 M Lösung von 4-Trifluormethyl-phenyl-magnesiumbromid (hergestellt durch Refluxieren von 3.02 g Brom-4-trifluormethyl-benzol mit 324 mg Magnesium-Spänen in 60 ml trockenem THF) über 15 Minuten hinzu. Man rührt 40 Minuten bei -20°C und hydrolysiert bei 0°C durch Zugabe von 20 ml 5% NaHCO₃-Lösung. Das Gemisch wird mit EtOAc extrahiert und die vereinigten organischen Phasen mit 5% NaHCO₃-Lösung und NaCl-Lösung gewaschen, über Na₂SO₄ getrocknet und eingeengt. Man reinigt an 170 g Kieselgel (Elution EtOAc/Petrolether/Triethylamin 1:10:0.1).
Ausbeute: 972 mg (90%) eines 1:1-Gemisches zweier Diastereomere
R_{f} = 0.32 (Diastereomer 1) (EtOAc/Petrolether 1:10) (Beispiel 4) und
R_{f} = 0.24 (Diastereomer 2) (EtOAc/Petrolether 1:10) (Beispiel 5).

### Beispiel 6

### [1S, 7(1RS)]-1-(tert-Butyl-dimethyl-silyloxy)-6-cyclopentyl-8-(4-fluor-phenyl)-7-[fluor-(4-trifluormethyl-phenyl)-methyl]-3,3-dimethyl-1,2,3,4-tetrahydro-naphthalin

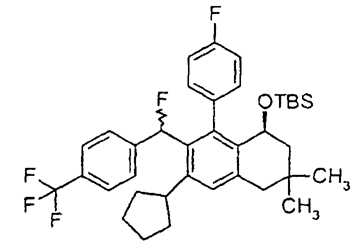

Zu einer Lösung von 960 mg (1.53 mmol) der Verbindung aus Beispiel 4 und 5 gibt man bei -78°C 407 mg (2.53 mmol) DAST hinzu. Nach 5 Minuten läßt man auf Raumtemperatur erwärmen und rührt 30 Minuten nach. Bei 0°C wird mit 15 ml einer 5% NaHCO₃-Lösung hydrolysiert. Man extrahiert das Gemisch mit EtOAc, wäscht die vereinigten organischen Phasen mit ges. NaCl-Lösung, trocknet über Na₂SO₄ und engt ein. Anschließend wird das Produkt an Kieselgel 60 gereinigt (Elution: EtOAc/Petrolether/Triethylamin 1:20:0.1).
Ausbeute: 874 mg (91%) eines 1:1-Gemisches zweier Diastereomere
R_{f} = 0.68 (EtOAc/Petrolether 1:20).

### Beispiel 7:

### 7,7-Dimethyl-4-(4-fluorphenyl)-3-[fluor-(4-trifluormethyl-phenyl)-methyl]-2-isopropyl-5,6,7,8-tetrahydronaphthalin-5-ol

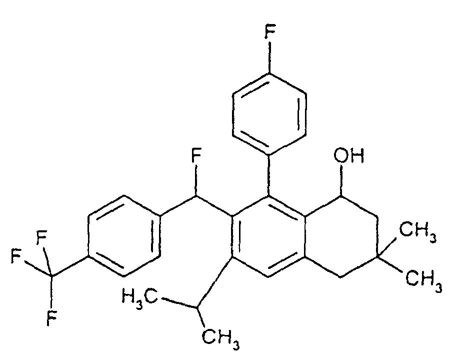

Bei Raumtemperatur werden 778 mg der Verbindung aus Bsp. 3 in 12.9 ml THF gelöst und 12.9 ml 1 molare Tetrabutylammoniumfluoridlsg. innerhalb von 5min. zugetropft und 3h gerührt. Zur Aufarbeitung wurde die Reaktionslsg. in eine Mischung aus 130 ml Wasser und 65 ml Toluol eingerührt, und die wässr. Phase noch einmal mit 30 ml Toluol nachextrahiert. Die vereinigten org. Phasen wurden 2 mal mit 30 ml Wasser gewaschen, über Natriumsulfat getrocknet und unter Vakuum bei max 50°C eingeengt. Das Rohprodukt wurde auf 90g Kieselgel 60/0.04-0.063mm (Säule 3X25cm) mit Cyclohexan/Diethylether 90:10 chromatographiert, die Hauptfraktion eingeengt und aus Cyclohexan kristallisiert.
Ausbeute: 0.45 g (66 % d. Th.)
R_{f} = 0.27 (Cy : EE 9 : 1)

### Beispiel 8 und Beispiel 9

### [1S,7(1S)]-6-Cyclopentyl-8-(4-fluor-phenyl)-7-[fluor-(4-trifluormethyl-phenyl)-methyl]-3,3-dimethyl-1,2,3,4-tetrahydro-naphthalin-1-ol

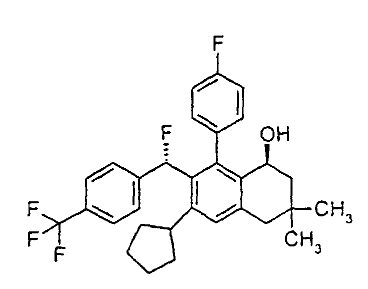

Eine Lösung aus 865 mg (1.38 mmol) der Verbindung aus Beispiel 6 in 30 ml THF wird mit 12.5 ml einer 1.1 M Lösung von Tetrabutylammoniumfluorid in THF versetzt und 16 h bei Raumtemperatur gerührt. Man gibt Wasser hinzu und extrahiert mit EtOAc. Die vereingten organischen Phasen werden mit ges. NaCl-Lösung gewaschen, über Na₂SO₄ getrocknet und eingeengt. Die Diastereomere werden an 140 g Kieselgel 60 getrennt (Elution: Toluol/ Cyclohexan 8:2)

### Beispiel 8

Ausbeute: 236 mg (33%) Diastereomer 2 (Titelverbindung, R_{f} = 0.38, Toluol/Cyclohexan 8:2)

### Beispiel 9

Ausbeute: 77 mg (11%) Diastereomer 1 (R_{f} = 0.29, Toluol/Cyclohexan 8:2).

### Beispiel 10

### 1-(4-Fluorphenyl)-3-isopropyl-8-oxo-6-spirocyclobutyl-2-(4-trifluormethylbenzoyl)-5,6,7,8-tetrahydronaphthalin

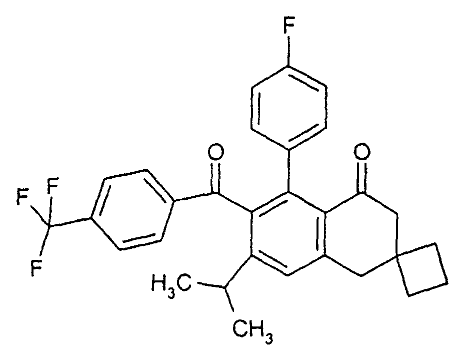

Die Reaktion wird unter Argon-Schutzgasatmosphäre durchgeführt. 0,5 g (0,801 mmol) der Verbindung aus Beispiel XXIV werden in 13 ml Dioxan vorgelegt, 0,5 g (3,283 mmol) 1,5-Diazabicyclo-[5.4.0]-undec-7-en (DBU) und 0,29 g (0,985 mmol) 1-(4-Trifluormethylphenyl)-3-(4-fluorphenyl)-propen-3-on werden zugegeben und 20 Stunden bei 96°C gerührt. Das Reaktionsgemisch wird abgekühlt, mit 150 ml Essigester versetzt, einmal mit 150 ml 1 N HCl und dreimal mit je 30 ml Wasser gewaschen, über Na₂SO₄ getrocknet, mit Essigester nachgewaschen und bei 60 °C unter vermindertem Druck eingeengt. Das Rohprodukt wird über 100 g Kieselgel chromatographiert (Petrolether/Essigester = 10:1).
Ausbeute: 0,311 g (64,1% d. TH.)
Fp.: 156 bis 157°C

### Beispiel 11

### (8S)-1-(4-Fluorphenyl)-8-hydroxy-3-isopropyl-6-spirocyclobutyl-2-(4-trifluormethyl-benzoyl)-5,6,7,8-tetrahydronaphthalin

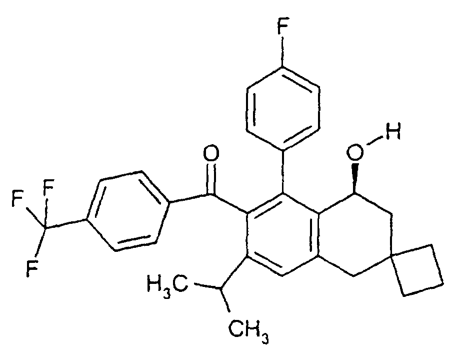

### Enantio- und positionsselektive (site-selective) Reduktion der Diketonstufe:

20,4 mg (0,14 mmol; 0,31 eq.) (1R, 2S)-1-Aminoindan-2-ol wurden unter Ausschluß von Feuchtigkeit unter Argon in 0,5 ml THF (wasserfrei) gelöst. Es folgte die Zugabe von 225 mg (1,38 mmol; 3,12 eq.) Boran-N,N-Diethylanilin-Komplex sowie 40 min Rühren bei RT (28°C) und 20 min bei Eiswasserkühlung. Bei dieser Temperatur wurden 219 mg der Verbindung aus Beispiel 10 gelöst in 1,5 ml THF (wasserfrei) binnen 2 min hinzugegeben und einmal mit 0,5 ml THF nachgespült. Das Reaktionsgemisch wurde über Nacht (17 h) unter langsamer Erwärmung auf RT gerührt. Durch Zugabe von 2 ml Methanol (Gasentwicklung!) wurde die Reaktion abgebrochen. Nach dem Verdünnen mit Essigsäureethylester wurde mit 10 ml 1 N Salzsäure ausgeschüttelt, die wäßriger Phase dreimal mit Essigsäureethylester extrahiert, die vereinigten organischen Phasen mit gesättigter wäßriger Natriumbicarbonatlösung ausgeschüttelt, die wäßrige Phase noch zweimal mit Essigsäureethylester extrahiert und die vereinten organischen Phasen über Natriumsulfat getrocknet. Nach dem Entfernen des Solvens i. Vak. wurden 250 mg festes Rohprodukt erhalten. Die chromatographische Reinigung an Kieselgel mit Essigsäureethylester/Petrolether = 1:9 ergab 182 mg (82%) Produkt als weißen Feststoff.
Schmp. (unkorrigiert): 199-201°C (Essigsäureethylester/Petrolether)

### Beispiel 12

### (8S)-1-(4-Fluorphenyl)-8-tert-butyldimethylsilyloxy-3-isopropyl-6-spirocyclobutyl-2-(4-trifluormethylbenzoyl)-5,6,7,8-tetrahydronaphthalin

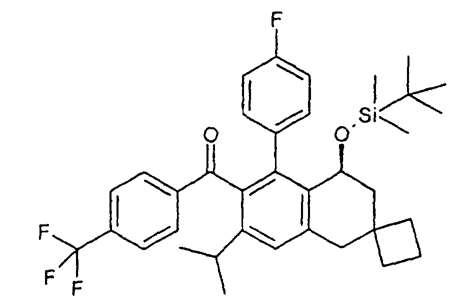

169 mg (0.34 mmol) der Verbindung aus Beispiel 11 wurden unter Ausschluß von Feuchtigkeit unter Argon-atmosphäre in 2 ml Toluol gelöst und im Eis/Acetonbad gekühlt (Badtemperatur: -11°C). Nach der Zugabe von 74 mg (0.69 mmol) Lutidin wurden 136 mg (0.52 mmol) tert.-Butyldimethylsilyltriflat in 1 ml Toluol tropfenweise addiert und mit 0.2 ml Toluol nachgespült. Nach 70min. Rühren unter Erwärmung auf -9°C (Badtemperatur) wurde auf Eiswasserkühlung gewechselt. Nachdem nach 130 min Reaktionsdauer keine weitere Umsetzung zu erkennen war, wurden nach 150 min weitere 109 mg (1.02 mmol) Lutidin sowie 135 mg (0.51 mmol) tert.-Butyldimethylsilyltriflat hinzugegeben. Nach 45minütigem Rühren unter Eiskühlung wurde 10 min auf RT erwärmt, das Reaktionsgemisch mit Essigsäureethylester verdünnt und die Reaktion durch Zugabe von 10 ml 1N Salzsäure abgebrochen. Nach dem Ausschütteln wurde die wäßr. Phase 3x mit Essigsäureethylester extrahiert, die vereinten organischen Phasen mit gesättigter wäßriger Bicarbonat-Lösung neutralisiert, die wäßrige Phase 2x mit Essigsäuremethylester extrahiert und die vereinten organischen Phasen über Natriumsulfat getrocknet. Das Solvens wurde i. Vak. entfernt. Nach der Chromatographie des Rückstands an Kieselgel mit Essigsäureethylester/Petrolether 1:20 wurden 160 mg (77%) weißer Feststoff isoliert, welcher als Verunreinigung das Eliminierungsprodukt enthielt. Das Nebenprodukt ließ sich durch Digerieren mit Methanol entfernen.
Schmp. (unkorrigiert): 137 bis 138°C (Methanol)

### Beispiel 13

### (S)-8-(t-Butyl-dimethyl)-silyloxy-1-(4-fluorphenyl)-2-[(R)-hydroxy-(4-trifluormethylphenyl)-methyl]-3-isopropyl-6-spiro-cyclobutyl-5,6,7,8-tetrahydronaphthalin

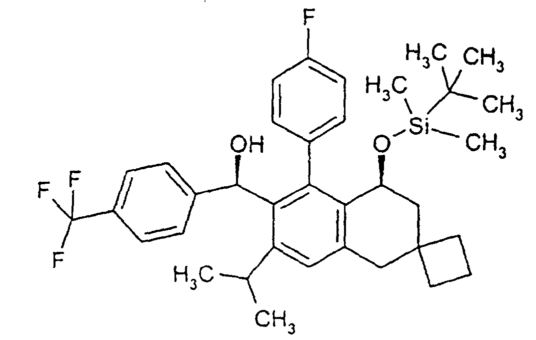

700 mg (1,22 mmol) des Keton-Vorproduktes aus Beispiel 12 werden in 40 ml THF gelöst, auf 0°C abgekühlt und tropfenweise mit einer 1 molaren Lösung von Lithiumaluminiumhydrid in THF versetzt. Es wird 15 min bei 0°C und 1,5 h bei RT gerührt. Es wird auf 0°C abgekühlt, tropfenweise mit 20 ml Wassser versetzt und dreimal mit Essigester extrahiert. Die vereinten Essigester-Phasen werden mit Wasser und gesättigter Kochsalzlösung gewaschen, getrocknet und eingeengt. Durch Umkristallisation aus Cyclohexan werden 200 mg (26,8%) farblose Kristalle erhalten. Aus der Mutterlauge konnten nach Chromatographie noch 210 mg (28,1% d. Th.) des gewünschten Diastereomeren erhalten werden.

### Beispiel 14

### (S)-8-(t-Butyl-dimethy])-silyloxy-1-(4-fluorphenyl)-2-[(S)-fluor-(4-trifluormethylphenyl)-methyl]-3-isopropyl-6-spiro-cyclobutyl-5,6,7,8-tetrahydronaphthalin

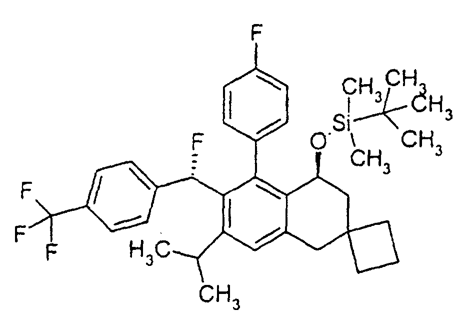

8,9 g (14,52 mmol) des Alkohols aus Beispiel 13 werden unter Inertgas in 125 ml Toluol gelöst und auf -70°C gekühlt. Bei dieser Temperatur tropft man 2,88 ml DAST hinzu und rührt eine halbe Stunde bei ca. -60°C nach. Aufarbeitung erfolgt durch Zugabe von 75 ml gesättigter Natriumhydrogencarbonatlösung, Extraktion der wäßrigen Phase mit Toluol, Waschen der vereinigten organischen Phasen mit Wasser und gesättigter Kochsalzlösung, Trocknen über Natriumsulfat und Einengen. Chromatographie an Kieselgel mit Petrolether/Methylenchlorid (95/5) ergaben 6,5 g Fluorverbindung als farblosen Feststoff (71% d.Th.).

### Beispiel 15

### (S)-8-Hydroxy-1-(4-fluorphenyl)-2-[(S)-fluor-(4-trifluormethyl-phenyl)-methyl]-3-isopropyl-6-spiro-cyclobutyl-5,6,7,8-tetrahydronaphtalin

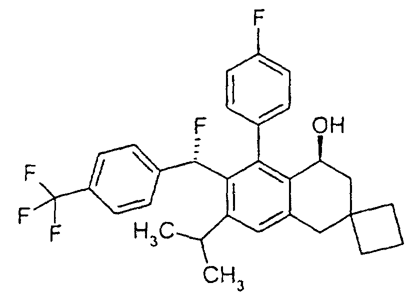

6,5 g (10,57 mmol) der Fluorverbindung aus Beispiel 14 werden unter Inertgas in 130 ml Tetrahydrofuran gelöst und bei Raumtemperatur mit 95 ml einer 1 Molaren Lösung von Tetrabutylammoniumfluorid 2 Stunden lang gerührt. Aufarbeitung erfolgt durch Zugabe von Wasser bei 10°C, Extraktion der wäßrigen Phase mit Toluol, Waschen der vereinigten organischen Phasen mit Wasser und gesättigter Kochsalzlösung, Trocknen über Natriumsulfat und Einengen. Chromatographie an Kieselgel mit Toluol/Cyclohexan (9/1) ergaben 4,6 g Alkohol als farblosen Feststoff (85% d.Th.).

In Analogie zu den gezeigten Herstellungsvorschriften werden die in der Tabelle 1 aufgeführten Verbindungen hergestellt:

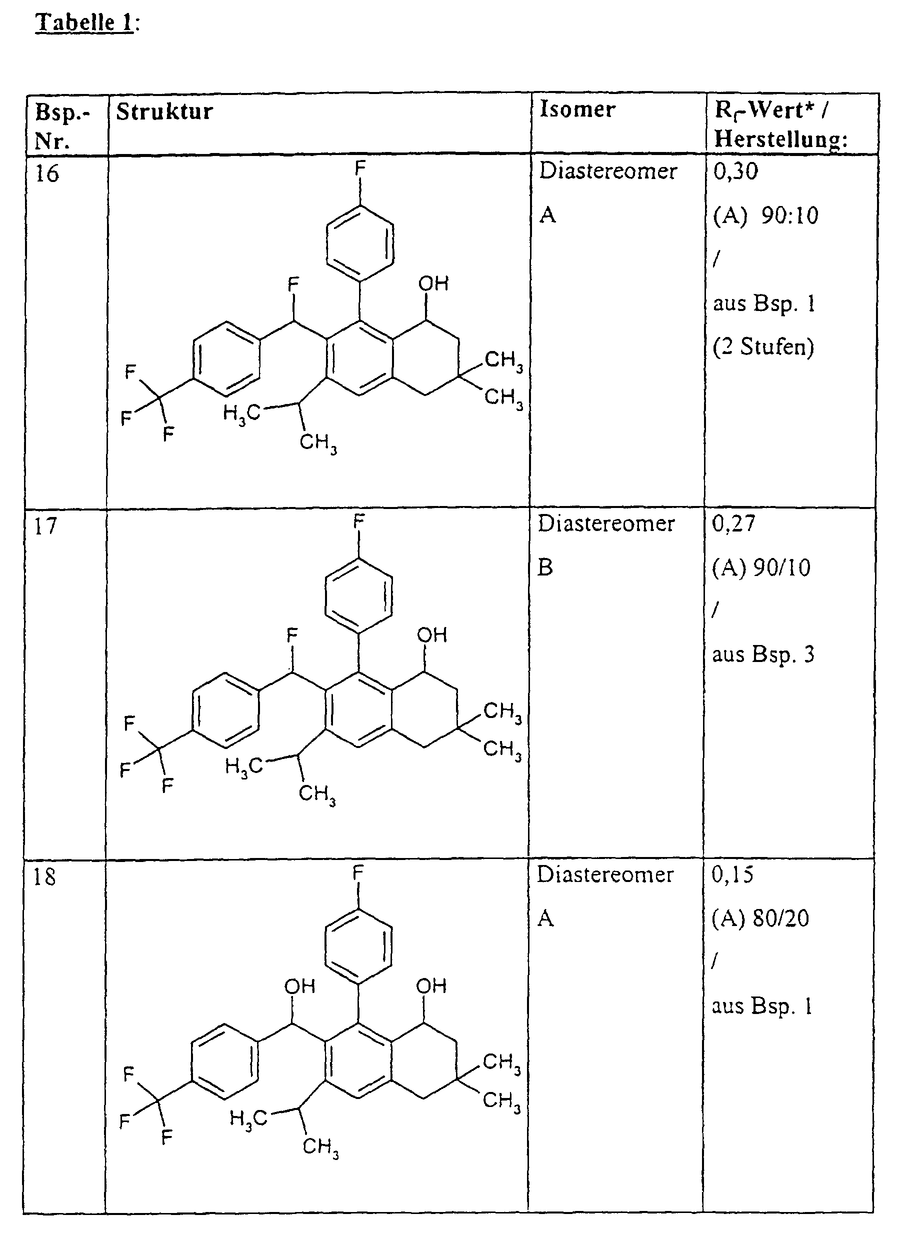

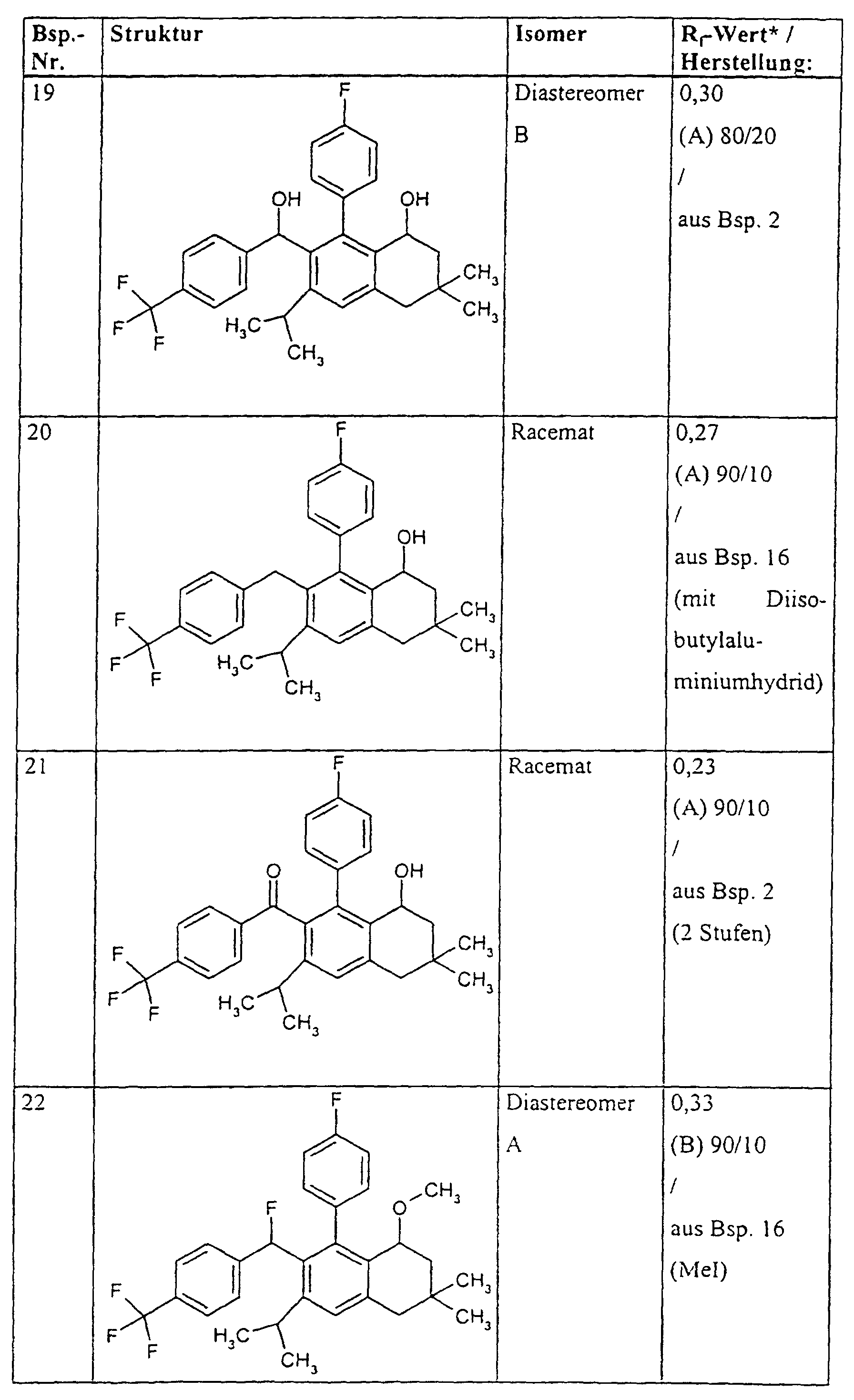

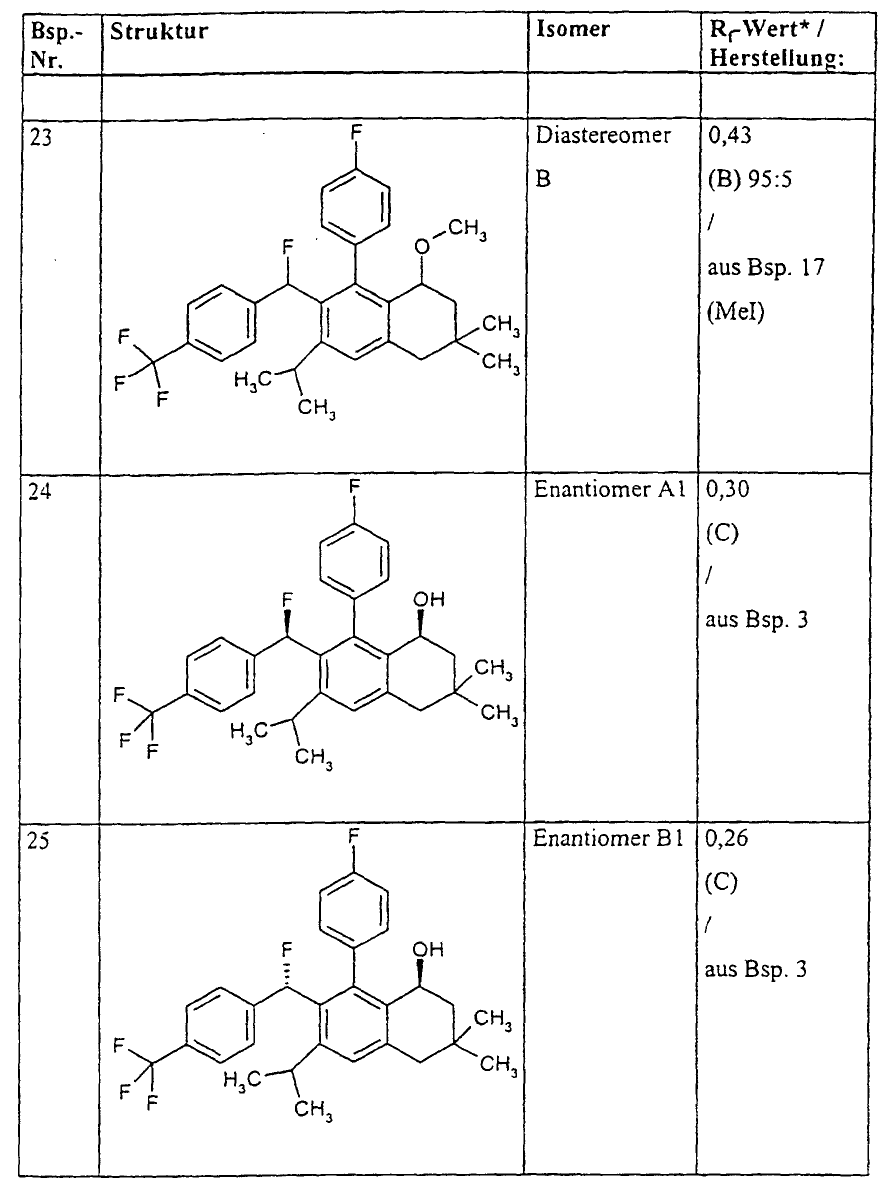

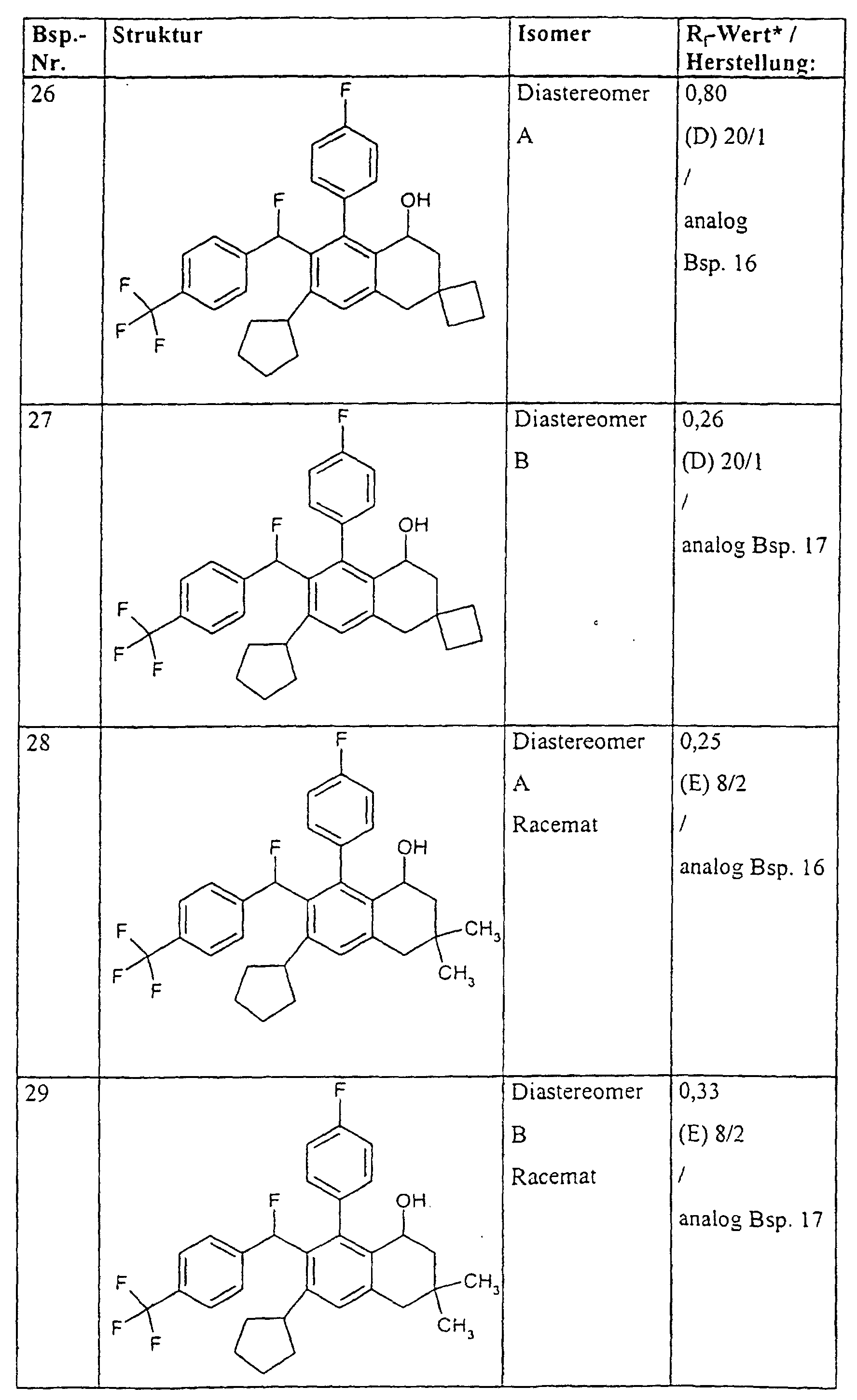

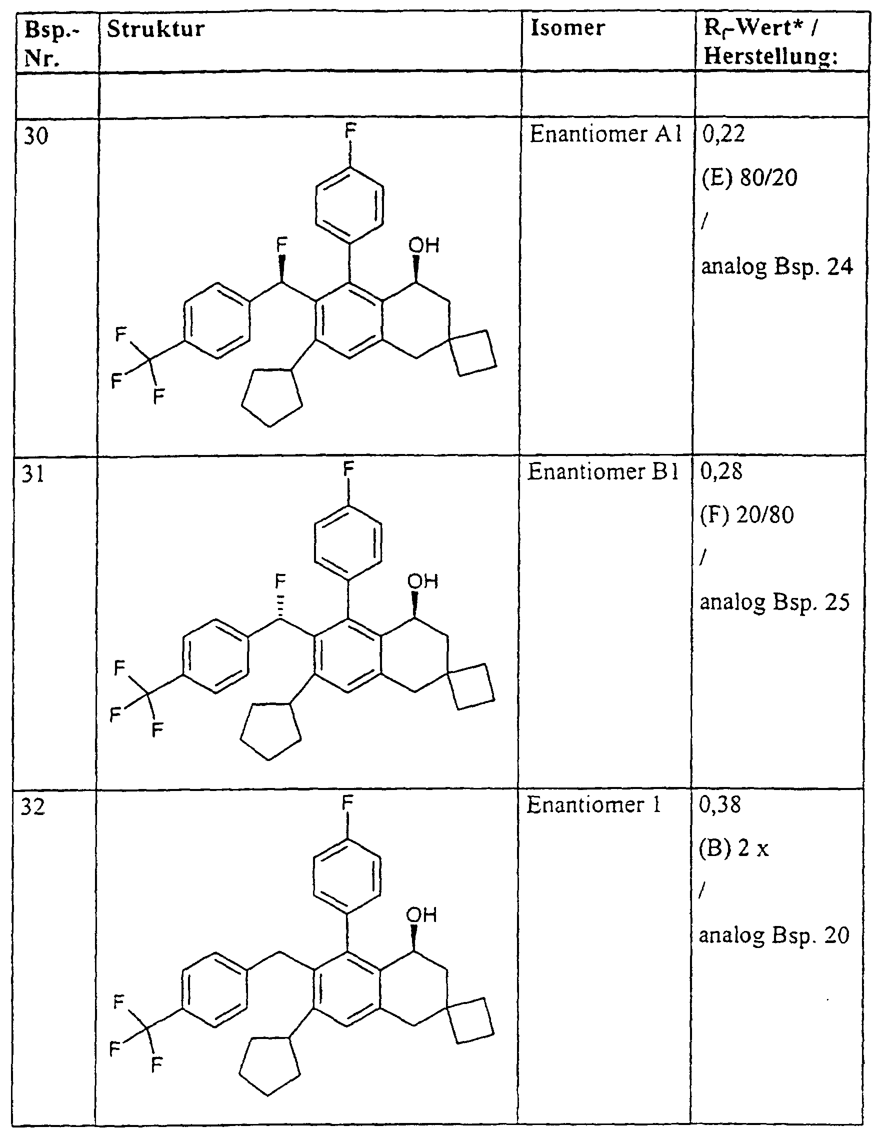

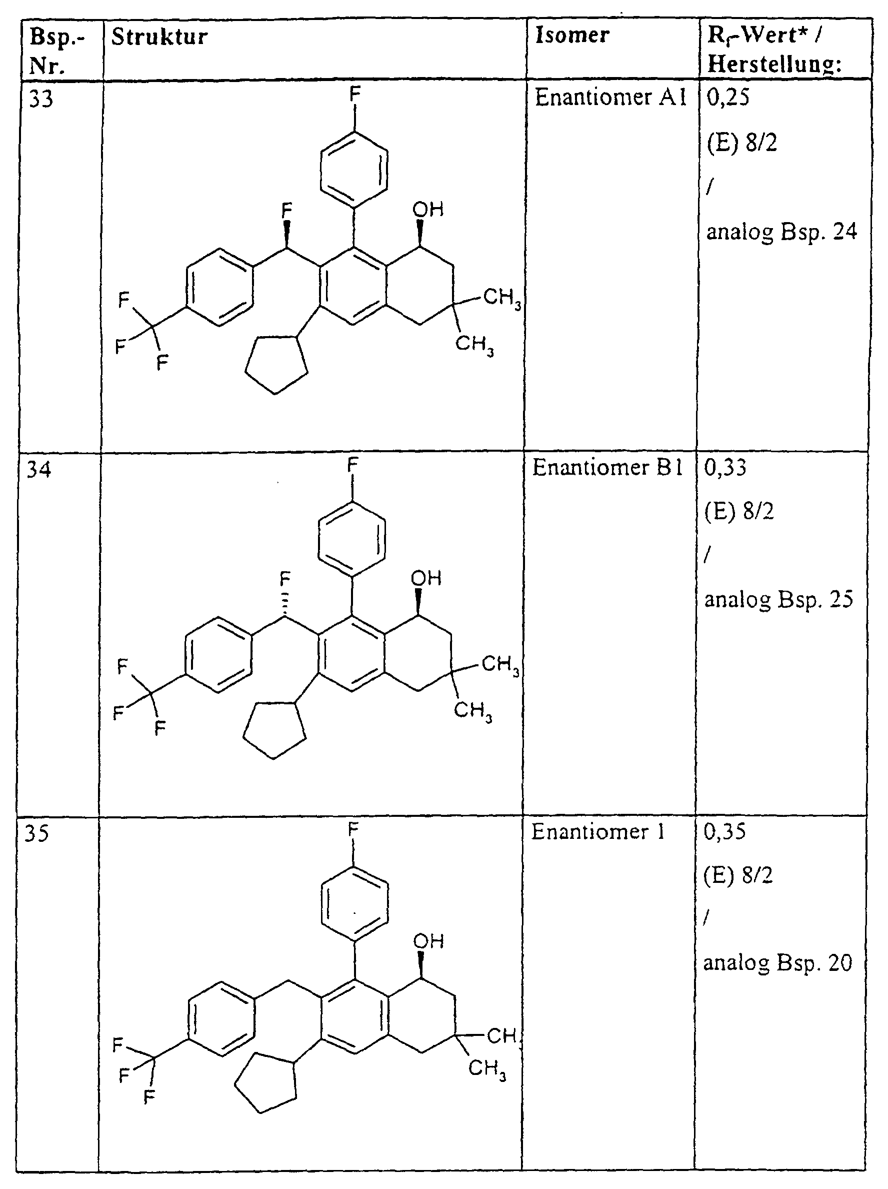

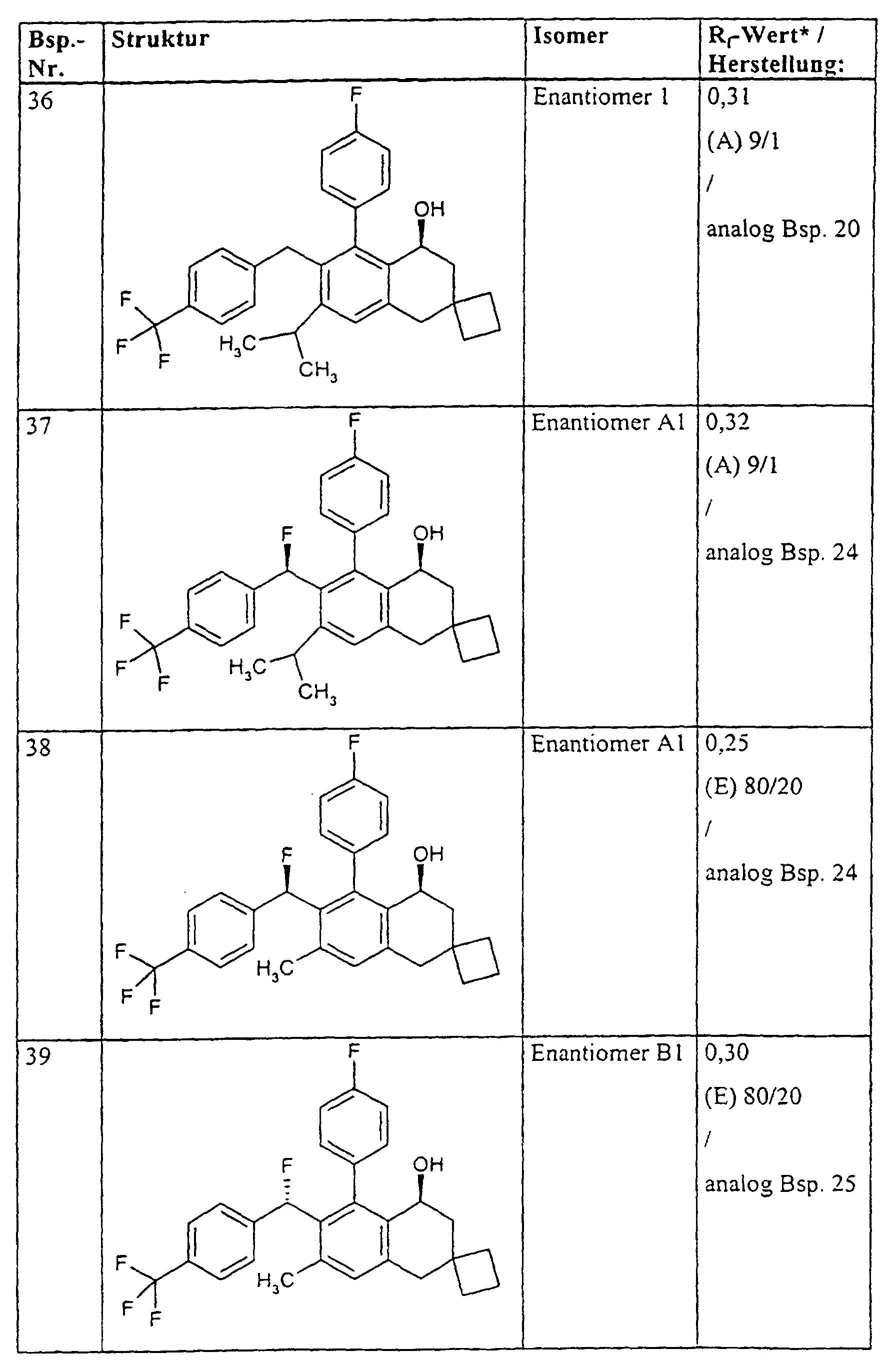

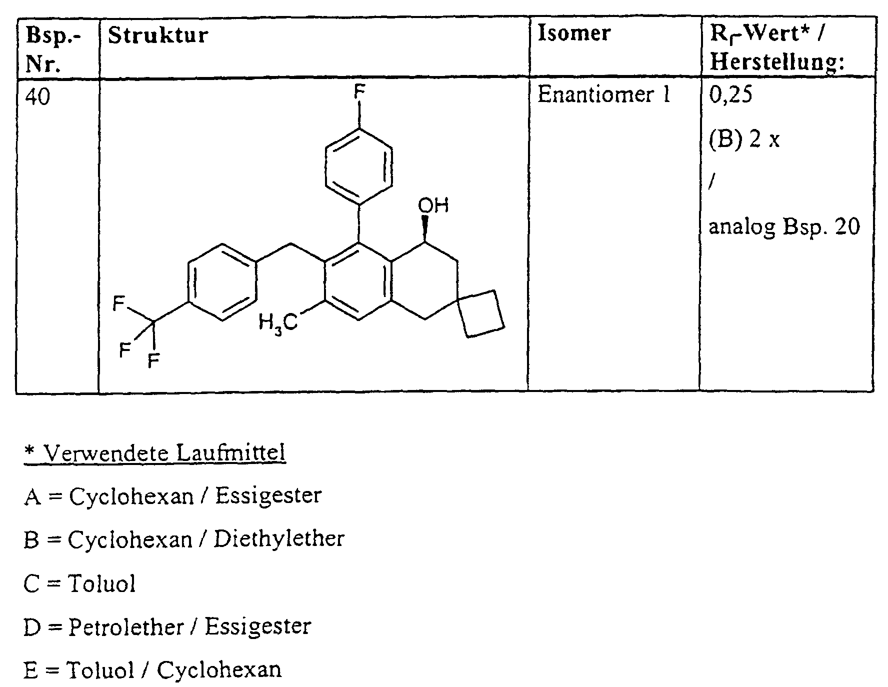

## Patentansprüche

1. Verbindungen der allgemeinen Formel (I), 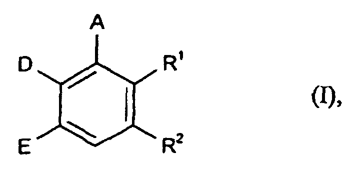 in welcher
A für Phenyl, Pyridyl oder Thienyl steht, die gegebenenfalls bis zu 2-fach gleich oder verschieden durch Fluor, Chlor, Brom, Hydroxy, Trifluormethyl, Trifluormethoxy oder durch geradkettiges oder verzweigtes Alkyl oder Alkoxy mit jeweils bis zu 5 Kohlenstoffatomen substituiert sind,
D für einen Rest der Formel 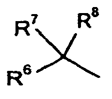 steht,
worin
R⁶ Phenyl, Pyridyl, Pyrimidyl und Pyrazinyl bedeuten, wobei die Cyclen, gegebenenfalls bis zu 3-fach, im Fall der stickstoffhaltigen Ringe auch über die N-Funktion, gleich oder verschieden durch Fluor, Chlor oder Trifluormethyl substituiert sind
R⁷ Wasserstoff oder Fluor bedeutet,
und
R⁸ Wasserstoff, Fluor, Chlor, Azido, Trifluormethyl, Hydroxy, Trifluormethoxy, oder geradkettiges oder verzweigtes Alkoxy oder Alkyl mit jeweils bis zu 4 Kohlenstoffatomen oder einen Rest der Formel -NR¹⁵R¹⁶ bedeutet,
worin
R¹⁵ und R¹⁶ gleich oder verschieden sind und Wasserstoff oder geradkettiges oder verzweigtes Alkyl mit bis zu 3 Kohlenstoffatomen bedeuten,
oder
R⁷ und R⁸ gemeinsam einen Rest der Formel =O bilden,
E für Cyclopropyl, Cyclobutyl, Cyclopentyl oder Cyclohexyl oder Phenyl steht, das gegebenenfalls durch Fluor oder Trifluormethyl substituiert ist, oder
für geradkettiges oder verzweigtes Alkyl mit bis zu 4 Kohlenstoffatomen steht, das gegebenenfalls durch Hydroxy substituiert ist,
R¹ und R² gemeinsam eine geradkettige oder verzweigte Alkylenkette mit bis zu 7 Kohlenstoffatomen bilden, die durch eine Carbonylgruppe oder einen Rest der Formel
-OR¹⁹
substituiert sein muß,
worin
R¹⁹ Wasserstoff, Cyclopropyl, Cyclopentyl, Cyclohexyl, geradkettiges oder verzweigtes Silylalkyl mit bis zu 6 Kohlenstoffatomen oder geradkettiges oder verzweigtes Alkyl mit bis zu 4 Kohlenstoffatomen bedeutet,
und die die von R¹ und R² gebildete Alkylenkette gegebenenfalls durch einen spiro-verknüpften Rest der Formel 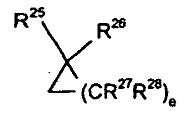 substituiert ist,
worin
e eine Zahl 1, 2, 3, 4 oder 5 bedeutet,
R²⁵, R²⁶, R²⁷ und R²⁸ gleich oder verschieden sind und Wasserstoff bedeutet,
und deren Stereoisomere, Stereoisomerengemische und Salze.

2. Verbindungen der Formel (I) nach Anspruch 1,
in welcher
A für gegebenenfalls durch Fluor substituiertes Phenyl, insbesondere 4-Fluorphenyl, steht,
und
D für einen Rest der Formel 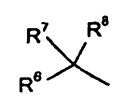 steht, worin
R⁶ Phenyl oder Trifluormethyl-substituiertes Phenyl bedeutet,
R⁷ Wasserstoff bedeutet,
R⁸ Wasserstoff, Fluor, Methoxy oder Hydroxy bedeutet,
oder
R⁷ und R⁸ gemeinsam eine Carbonylgruppe bilden,
E für Cyclopropyl, Cyclopentyl oder Cyclohexyl steht, oder für geradkettiges oder verzweigtes Alkyl mit bis zu 4 Kohlenstoffatomen steht, das gegebenenfalls durch Hydroxy substituiert ist,
und
R¹ und R² gemeinsam für einen Rest der Formel 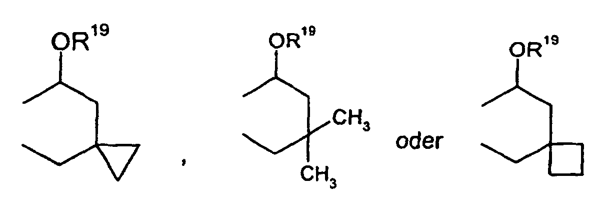 stehen,
worin
R¹⁹ Wasserstoff bedeutet,
und deren Stereoisomere, Stereoisomerengemische und Salze.

3. Verbindungen nach Anspruch 1 zur Verwendung bei der Bekämpfung von Krankheiten.

4. Verfahren zur Herstellung von Verbindungen nach Anspruch 1, **dadurch gekennzeichnet, daß** man
[A] Verbindungen der allgemeinen Formel (II) 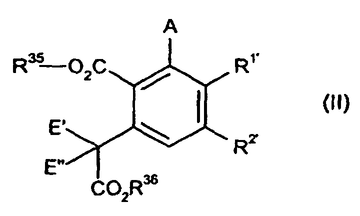 in welcher
A die oben angegebene Bedeutung hat,
R³⁵ und R³⁶ gleich oder verschieden sind und geradkettiges oder verzweigtes Alkyl mit bis zu 4 Kohlenstoffatomen bedeuten,
R^{1'} und R^{2'} gemeinsam für die unter R¹ und R² oben angegebene geradkettige oder verzweigte Alkylenkette mit bis zu 7 Kohlenstoffatomen stehen, die durch tert.Butyl-dimethyl-silanyloxy (OTBS) substituiert ist,
und
E' und E" gemeinsam mit dem an sie gebundenen Kohlenstoffatom den oben angegebenen Bedeutungsumfang von E umfassen,
zunächst durch selektive Reduktion des aliphatischen Esters und anschließende Umsetzung mit Verbindungen der allgemeinen Formel (III)
R³⁷-Halogen (III)
in welcher
R³⁷ für Mesyl, Tosyl oder Sulfonyl steht,
und
Halogen für Chlor, Brom oder Jod, vorzugsweise für Chlor steht,
in inerten Lösemitteln, in Anwesenheit einer Base,
in die Verbindungen der allgemeinen Formel (IV) 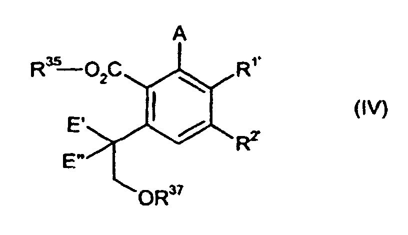 in welcher
A, E', E", R^{1'}, R^{2'}, R³⁵ und R³⁷ die oben angegebene Bedeutung haben,
überführt,
diese in einem weiteren Schritt in Abhängigkeit von den oben angegebenen Definitionen von E'/E" entweder durch eine zweifache Reduktion oder durch eine Verseifung, Bartonreaktion und eine Reduktion in die Verbindungen der allgemeinen Formel (V) 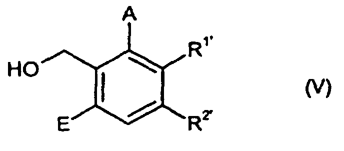 in welcher
A, E, R^{1'} und R^{2'} die oben angegebene Bedeutung haben,
überführt,
anschließend durch Oxidation die Aldehyde der allgemeinen Formel (VI) 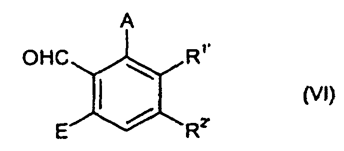 in welcher
A, E, R^{1'} und R^{2'} die oben angegebene Bedeutung haben,
herstellt,
und abschließend beispielsweise durch eine Grignard-Reaktion die Formylgruppe in den Rest D überführt und die TBS-Gruppe nach üblichen Methoden abspaltet,
oder
[B] Verbindungen der allgemeinen Formel (VI) 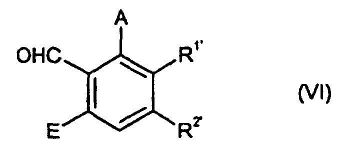 in welcher
A, E, R^{1'} und R^{2'} die oben angegebene Bedeutung haben,
zunächst wie unter [A] beschrieben in einer Grignard-Reaktion mit Verbindungen der allgemeinen Formel (VII)
R³⁸-MgBr (VII)
in welcher
R³⁸ die oben angebene Bedeutung von R⁶ umfaßt,
in inerten Lösemitteln und unter Schutzgasatmosphäre in die Verbindungen der allgemeinen Formel (VIII) 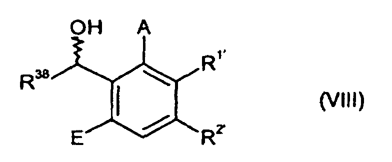 in welcher
A, D, E, R³⁸, R^{1'} und R^{2'} die oben angegebene Bedeutung haben,
überführt,
gegebenenfalls auf dieser Stufe ausgehend von der Hydroxyfunktion die unter D angegebenenen Substituenten R⁷/R⁸ nach üblichen Methoden einführt,
und abschließend die TBS-Gruppe mit Tetrabutylammoniumfluorid in inerten Lösemitteln abspaltet,
oder
[C] Verbindungen der allgemeinen Formel (IX) 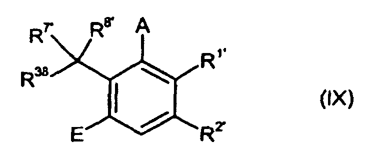 in welcher
A, E, R^{1'}, R^{2'} und R³⁸ die oben angegebene Bedeutung haben
und
R^{7'} und R^{8'} gemeinsam für die Carbonylgruppe stehen,
zunächst zu den Verbindungen der allgemeinen Formel (VIII) reduziert und anschließend wie unter [B] beschrieben umsetzt,
oder
[D] Verbindungen der allgemeinen Formel (X) 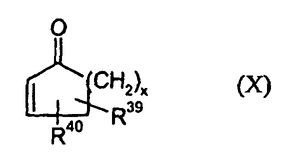 in welcher
x eine Zahl 1, 2 oder 3 bedeutet,
und
R³⁹ und R⁴⁰ gleich oder verschieden sind und für Wasserstoff stehen oder für geradkettiges oder verzweigtes Alkyl mit bis zu 6 Kohlenstoffatomen stehen,
wobei R³⁹ und R⁴⁰ auch geminal positioniert sein können,
oder
R³⁹ und R⁴⁰ gemeinsam einen spiroverknüpften Carbocyclus mit 3 bis 7 Kohlenstoffatomen bilden,
mit den Verbindungen der allgemeinen Formel (XI) 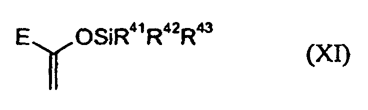 wobei
E die oben angegebene Bedeutung hat,
und
R⁴¹, R⁴² und R⁴³ gleich oder verschieden sind und für geradkettiges oder verzweigtes Alkyl mit bis zu 10 Kohlenstoffatomen stehen,
in Anwesenheit eines Metall- oder Halbmetallreagenzes, zunächst über die intermediäre Stufe der allgemeinen Formel (XII) 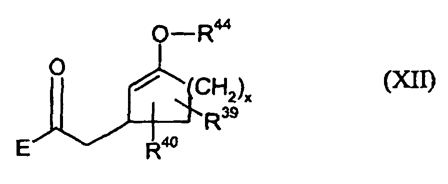 in welcher
x, E, R³⁹ und R⁴⁰ die oben angegebene Bedeutung haben,
und
R⁴⁴ für Metall- oder Halbmetallderivate, vorzugsweise für die des Titans steht,
und durch Zugabe der Verbindungen der allgemeinen Formel (XIII) 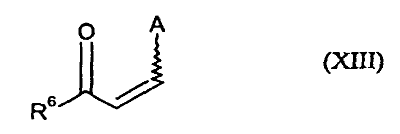 in welcher
R⁶ und A die oben angegebene Bedeutung haben,
über die Intermediate der allgemeinen Formel (XIV) 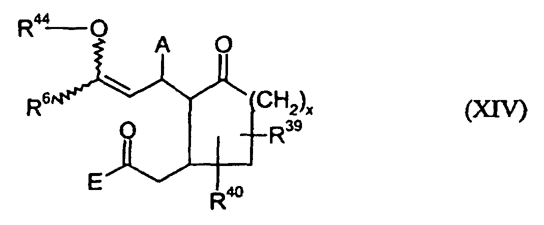 in welcher
A, E, R⁶, R³⁹, R⁴⁰, R⁴⁴ und x die oben angegebene Bedeutung haben,
in die Verbindungen der allgemeinen Formel (XV) 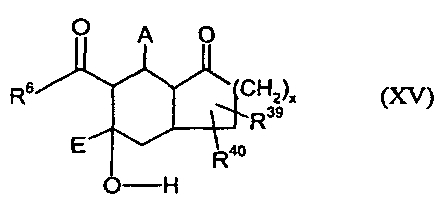 in welcher
A, E, R⁶, R³⁹, R⁴⁰ und x, die oben angegebene Bedeutung haben,
überführt,
anschließend durch eine Elimierung die Verbindungen der allgemeinen Formel (XVI) 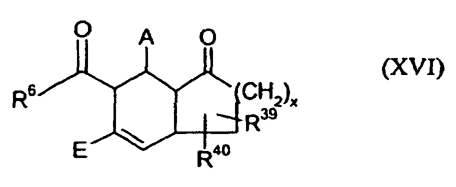 in welcher
A, E, R⁶, R³⁹, R⁴⁰ und x die oben angegebene Bedeutungen haben, herstellt,
in einem weiteren Schritt durch eine Halogenierung Halogenverbindungen, z.B. die Verbindungen der allgemeinen Formel (XVII) 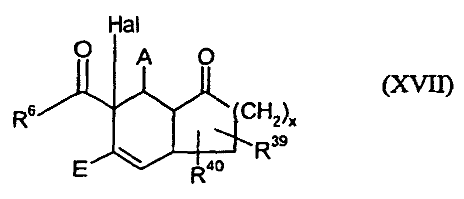 in welcher
R⁶, R³⁹, R⁴⁰, x, A und E die oben angegebenen Bedeutungen haben,
und
Hal für Halogen, vorzugsweise für Chlor oder Brom steht,
über die in situ entstehende Cyclohexadiene der allgemeinen Formel (XVIII) oder deren Isomere 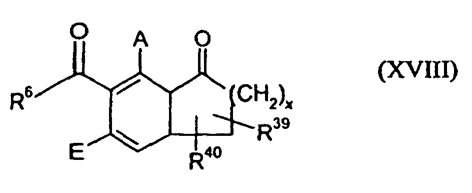 in welcher
A, E, x, R⁶, R³⁹ und R⁴⁰ die oben angegebene Bedeutung haben,
nach Oxidation die Verbindungen der allgemeinen Formel (XIX) 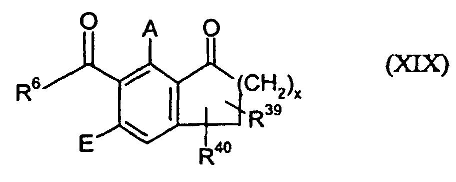 in welcher
A, E, x, R⁶, R³⁹ und R⁴⁰ die oben angegebenen Bedeutungen haben
herstellt,
desweiteren durch die Reduktion der Ketofunktion die Verbindungen der allgemeinen Formel (XX) 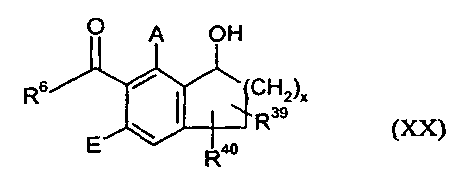 worin
A, E, x, R⁶, R³⁹ und R⁴⁰ die oben angegebene Bedeutung haben,
herstellt, wobei die Reduktion auch stereoselektiv durchgeführt werden kann,
anschließend durch Silylierung, z.B. durch Umsetzung mit chlorierten oder Trifluormethansulfonyl-substituierten Silylverbindungen (-SiR⁴¹R⁴²R⁴³)
die Verbindungen der allgemeinen Formel (XXI) 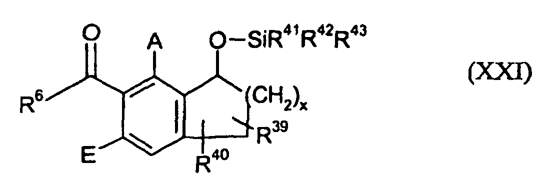 in welcher
x, A, E, R⁶, R³⁹, R⁴⁰, R⁴¹, R⁴² und R⁴³ die oben angegebene Bedeutung haben,
herstellt,
desweiteren durch Reduktion der zweiten Ketofunktion zunächst die Verbindungen der allgemeinen Formel (XXII) 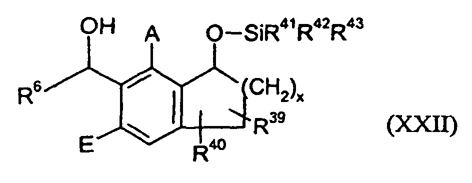 in welcher
x, A, E, R⁶, R³⁹, R⁴⁰, R⁴¹, R⁴² und R⁴³ die oben angegebene Bedeutung haben,
als Diastereomerengemisch erhält, aus diesem anschließend durch Trennung das Isomer der allgemeinen Formel (XXIII) 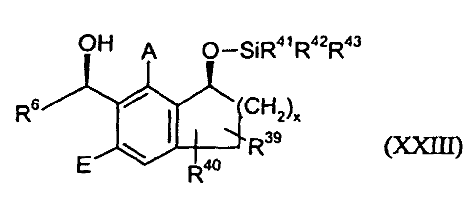 in welcher
x, A, E, R⁶, R³⁹, R⁴⁰, R⁴¹, R⁴² und R⁴³ die oben angegebene Bedeutung haben,
gewinnt,
und gegebenenfalls durch nucleophile Substitution enantioselektiv die Hydroxyfunktion durch einen der oben unter R⁸ aufgeführten Substituenten ersetzt und somit Verbindungen der allgemeinen Formel (XXIV) 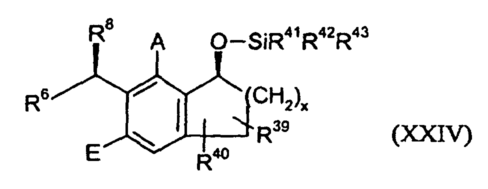 in welcher
x, A, E, R⁶, R⁸, R³⁹, R⁴⁰, R⁴¹, R⁴² und R⁴³ die oben angegebene Bedeutung haben,
erhält,
und in einem letzten Schritt die Hydroxyfunktion unter Abspaltung der Silylschutzgruppe nach üblichen Methoden freisetzt,
und im Fall der Enantiomere/Racemate nach üblichen Methoden trennt.

5. Verbindungen der allgemeinen Formel (XV) 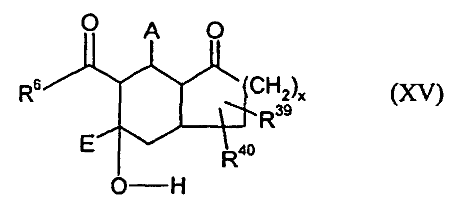 in welcher
A, E, und R⁶ die in Anspruch 1 angegebenen Bedeutungen haben und R³⁹, R⁴⁰ und x die in Anspruch 4 angegebenen Bedeutungen haben.

6. Arzneimittel enthaltend mindestens eine Verbindung nach Anspruch 1 sowie pharmakologisch verträgliche Formulierungshilfsmittel.

7. Arzneimittel nach Anspruch 6 zur Behandlung von Hyperlipoproteinämie.

8. Arzneimittel nach Anspruch 6 zur Behandlung von Arteriosklerose.

9. Verwendung von Verbindungen nach Anspruch 1 zur Herstellung von Arzneimitteln.

10. Verwendung nach Anspruch 9 zur Herstellung von Arzneimitteln zur Behandlung von Arteriosklerose, insbesondere Dyslipidämien.

## Claims

1. Compounds of the general formula (I), 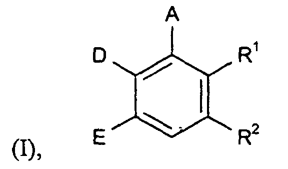 in which
A represents phenyl, pyridyl or thienyl, which are optionally substituted up to 2 times by identical or different substituents selected from the group consisting of fluorine, chlorine, bromine, hydroxyl, trifluoromethyl, trifluoromethoxy or by straight-chain or branched alkyl or alkoxy having in each case up to 5 carbon atoms,
D represents a radical of the formula 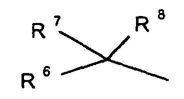 or
in which
R⁶ represents phenyl, pyridyl, pyrimidyl, and pyrazinyl, where the cycles are optionally substituted up to 3 times, in the case of the nitrogen-containing rings also via the N-function, by identical or different substituents selected from the group consisting of fluorine, chlorine and trifluoromethyl,
R⁷ represents hydrogen or fluorine,
and
R⁸ represents hydrogen, fluorine, chlorine, azido, trifluoromethyl, hydroxyl, trifluoromethoxy, or straight-chain or branched alkoxy or alkyl having in each case up to 4 carbon atoms or a radical of the formula -NR¹⁵R¹⁶,
in which
R¹⁵ and R¹⁶ are identical or different and each represents hydrogen or straight-chain or branched alkyl having up to 3 carbon atoms,
or
R⁷ and R⁸ together form a radical of the formula =O,
E represents cyclopropyl, cyclobutyl, cyclopentyl or cyclohexyl or phenyl which is optionally substituted by fluorine or trifluoromethyl, or
represents straight-chain or branched alkyl, having up to 4 carbon atoms, which is optionally substituted by hydroxyl,
R¹ and R² together form a straight-chain or branched alkylene chain, having up to 7 carbon atoms, which has to be substituted by a carbonyl group or a radical of the formula
-OR¹⁹
in which
and the alkylene chain formed by R¹ and R² is optionally substituted by a spire-linked radical of the formula 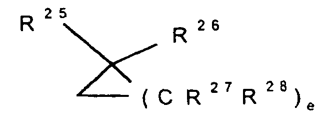 in which
e represents a number 1, 2, 3, 4 or 5,
R²⁵, R²⁶, R²⁷ and R²⁸ are identical or different and represents hydrogen,
and stereoisomers, stereoisomer mixtures and salts thereof.

2. Compounds of the formula (I) according to Claim 1,
in which
A represents optionally fluorine-substituted phenyl, in particular 4-fluorophenyl,
and
D represents a radical of the formula 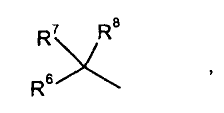 in which
R⁶ represents phenyl or trifluoromethyl-substituted phenyl,
R⁷ represents hydrogen,
R⁸ represents hydrogen, fluorine, methoxy or hydroxyl,
or
R⁷ and R⁸ together form a carbonyl group,
E represents cyclopropyl, cyclopentyl or cyclohexyl, or
represents straight-chain or branched alkyl, having up to 4 carbon atoms, which is optionally substituted by hydroxyl,
and
R¹ and R² together represent a radical of the formula 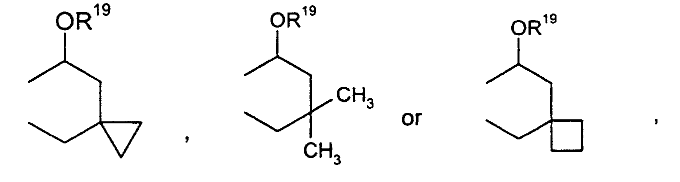 in which
R¹⁹ represents hydrogen,
and stereoisomers, stereoisomer mixtures and salts thereof.

3. Compounds according to Claim 1 for use in the control of diseases.

4. Process for preparing compounds according to Claim 1, **characterized in that**
[A] compounds of the general formula (II) 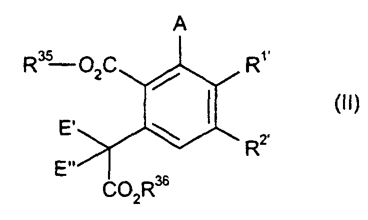 in which
A is as defined above,
R³⁵ and R³⁶ are identical or different and each represents straight-chain or branched alkyl having up to 4 carbon atoms,
R^{1'} and R^{2'} together represent the straight-chain or branched alkylene chain, having up to 7 carbon atoms, mentioned above under R¹ and R², which is substituted by tert-butyl-dimethyl-silanyloxy (OTBS),
and
E' and E" together with the carbon atom to which they are attached have the range of meanings of E given above,
are initially converted by selective reduction of the aliphatic ester and subsequent reaction with compounds of general formula (III)
R³⁷-halogen (III)
in which
R³⁷ represents mesyl, tosyl or sulphonyl,
and
Halogen represents chlorine, bromine or iodine, preferably chlorine,
in inert solvents, in the presence of a base,
into the compounds of the general formula (IV) 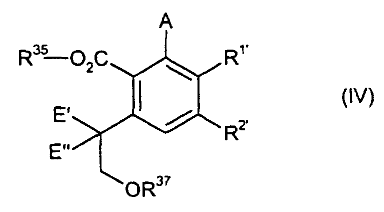 in which
A, E', E", R^{1'}, R^{2'}, R³⁵ and R³⁷ are each as defined above,
these are converted in a further step, depending on the definitions of E'/E" given above, either by a two-fold reduction or by hydrolysis, Barton reaction and a reduction, into the compounds of the general formula (V) 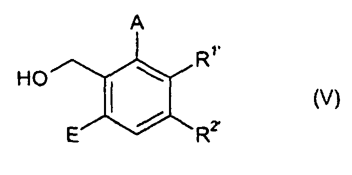 in which
A, E, R^{1'} and R^{2'} are each as defined above,
the aldehydes of the general formula (VI) 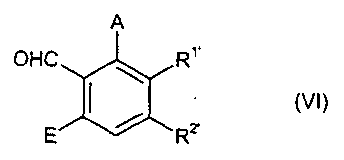 in which
A, E, R^{1'} and R^{2'} are each as defined above,
are subsequently prepared by oxidation
and finally, for example by a Grignard reaction, the formyl group is converted into the radical D and the TBS group is cleaved off by customary methods,
or
[B] compounds of the general formula (VI) 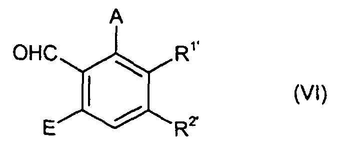 in which
A, E, R^{1'} and R^{2'} are each as defined above,
are initially, as described in [A], converted in a Grignard reaction with compounds of the general formula (VII)
R³⁸-MgBr (VII)
in which
R³⁸ has the meaning of R⁶ given above,
in inert solvents and under an atmosphere of protective gas, into the compounds of the general formula (VIII) 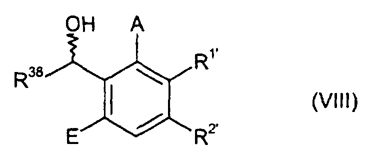 in which
A, D, E, R³⁸, R^{1'} and R^{2'} are each as defined above,
if appropriate on this stage starting from the hydroxyl function the substituents R⁷/R⁸ given under D are introduced by customary methods,
and the TBS group is subsequently cleaved off using tetrabutylammoniumfluoride in inert solvents,
or
[C] compounds of the general formula (IX) 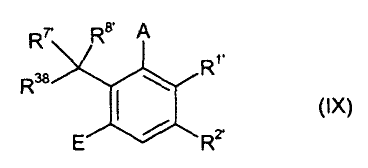 in which
A, E, R^{1'}, R^{2'} and R³⁸ are each as defined above
R^{7'} and R^{8'} together represent the carbonyl group,
are initially reduced to the compounds of the general formula (VIII) and subsequently reacted as described under [B],
or
[D] compounds of the general formula (X) 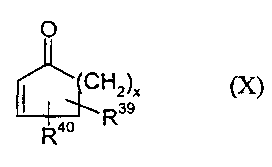 in which
x represents a number 1, 2 or 3,
and
R³⁹ and R⁴⁰ are identical or different and each represents hydrogen or represents straight-chain or branched alkyl having up to 6 carbon atoms,
it also being possible for R³⁹ and R⁴⁰ to be positioned geminally,
or
R³⁹ and R⁴⁰ together form a spiro-linked carbocycle having 3 to 7 carbon atoms,
are converted with compounds of the general formula (XI) 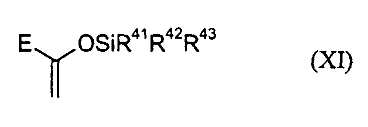 where
E is as defined above,
and
R⁴¹, R⁴² and R⁴³ are identical or different and each represents straight-chain or branched alkyl having up to 10 carbon atoms,
in the presence of a metal or semi-metal reagent, initially via the intermediate stage of the general formula (XII) 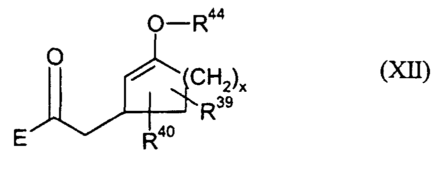 in which
x, E, R³⁹ and R⁴⁰ are each as defined above,
and
R⁴⁴ represents metal or semi-metal derivatives, preferably those of titanium,
and by addition of the compounds of the general formula (XIII) 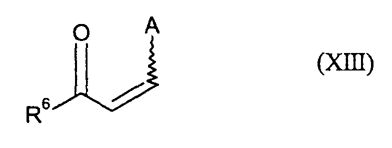 in which
R⁶ and A are each as defined above,
via intermediates of the general formula (XIV) 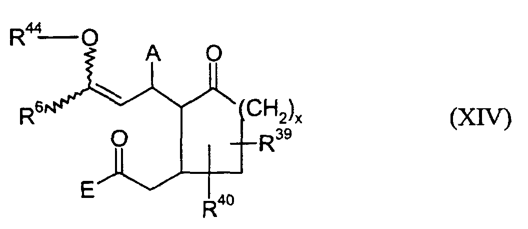 in which
A, E, R⁶, R³⁹, R⁴⁰, R⁴⁴ and x are each as defined above,
into the compounds of the general formula (XV) 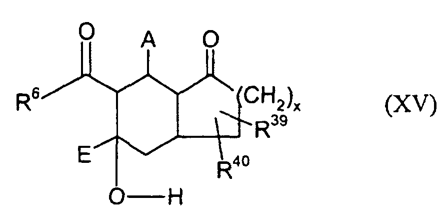 in which
A, E, R⁶, R³⁹, R⁴⁰ and x are each as defined above,
the compounds of the general formula (XVI) 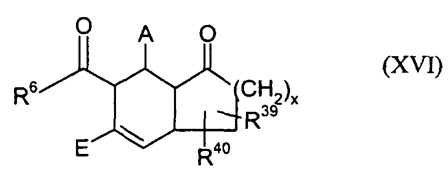 in which
A, E, R⁶, R³⁹, R⁴⁰ and x are each as defined above,
are subsequently prepared by an elimination,
halogen compounds, for example the compounds of the general formula (XVII) 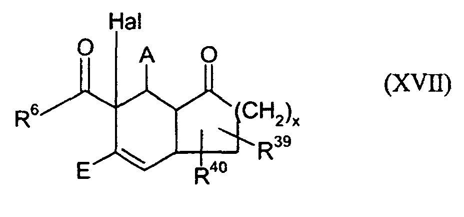 in which
R⁶, R³⁹, R⁴⁰, x, A and E are as defined above,
and
Hal represents halogen, preferably chlorine or bromine,
are prepared in a further step by a halogenation
via the cyclohexadienes of the general formula (XVIII), which are formed in situ, or isomers thereof 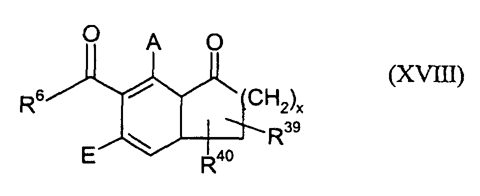 in which
A, E, x, R⁶, R³⁹ and R⁴⁰ are each as defined above,
compounds of the general formula (XIX) 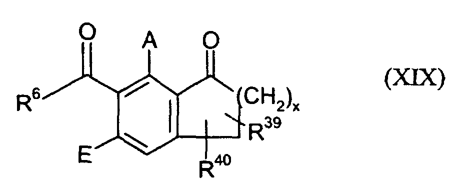 in which
A, E, x, R⁶, R³⁹ and R⁴⁰ are each as defined above
are prepared after oxidation,
furthermore, the compounds of the general formula (XX) 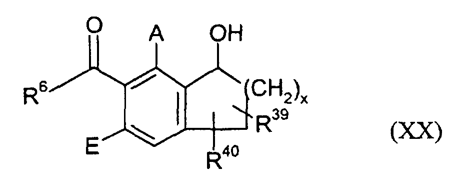 in which
A, E, x, R⁶, R³⁹ and R⁴⁰ are each as defined above,
are prepared by the reduction of the ketofunction, it also being possible to carry out the reduction stereoselectively,
the compounds of the general formula (XXI) 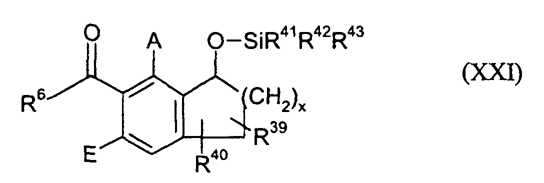 in which
x, A, E, R⁶, R³⁹, R⁴⁰, R⁴¹, R⁴² and R⁴³ are each as defined above, are subsequently prepared by silylation, for example by reaction with chlorinated or trifluoromethanesulfonyl-substituted silyl compounds (-SiR⁴¹R⁴²R⁴³)
furthermore, the compounds of the general formula (XXII) 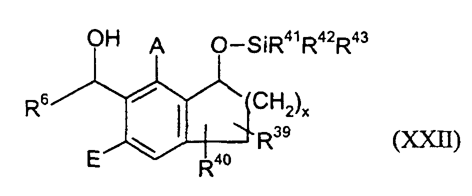 in which
x, A, E, R⁶, R³⁹, R⁴⁰, R⁴¹, R⁴² and R⁴³ are each as defined above,
are obtained initially by reduction of the second ketone function, as a mixture of diastereomers from which subsequently, by separation, the isomer of the general formula (XXIII) 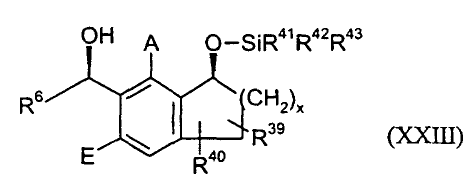 in which
x, A, E, R⁶, R³⁹, R⁴⁰, R⁴¹, R⁴² and R⁴³ are each as defined above
are obtained,
and, if appropriate, the hydroxyl function is replaced enantioselectively by nucleophilic substitution by one of the substituents listed above under R⁸, thus giving compounds of the general formula (XXIV) 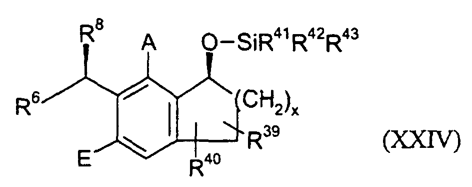 in which
x, A, E, R⁶, R⁸, R³⁹, R⁴⁰, R⁴¹, R⁴² and R⁴³ are each as defined above
and, in a last step, the hydroxyl function is liberated according to customary methods by cleaving off the silyl protective group,
and, in the case of enantiomers/racemates, is separated by customary methods.

5. Compounds of the general formula (XV) 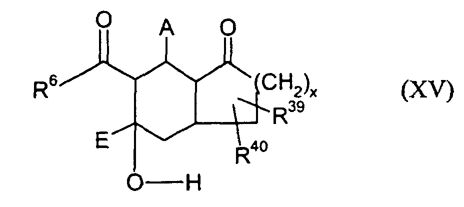 in which
A, E, and R⁶ are each as defined in Claim 1 and
R³⁹, R⁴⁰ and x are each as defined in Claim 4.

6. Pharmaceutical, comprising at least one compound according to Claim I and pharmacologically acceptable formulation auxiliaries.

7. Pharmaceutical according to Claim 6 for treating hyperlipoproteinaemia.

8. Pharmaceutical according to Claim 6 for treating arteriosclerosis.

9. Use of compounds according to Claim 1 for preparing pharmaceuticals.

10. Use according to Claim 9 for preparing pharmaceuticals for treating arteriosclerosis, in particular dyslipidaemias.

## Revendications

1. Composés de formule générale (I), 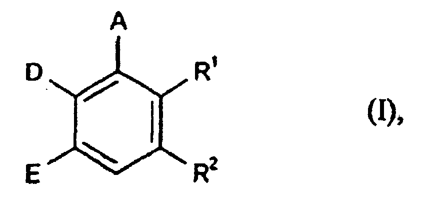 dans laquelle
A est un reste phényle, un reste pyridyle ou un reste thiényle, qui sont éventuellement substitués jusqu'à 2 fois identiques ou différentes par du fluor, du chlore, du brome, un radical hydroxy, trifluorométhyle, trifluorométhoxy ou par un radical alkyle ou alkoxy linéaire ou ramifié ayant chacun jusqu'à 5 atomes de carbone,
D représente un reste de formule 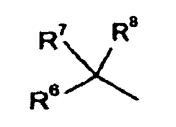 dans laquelle
R⁶ représente les restes phényle, pyridyle, pyrimidyle et pyrazinyle, les cycles étant éventuellement substitués jusqu'à 3 fois identiques ou différentes, également par l'intermédiaire de la fonction N dans le cas des noyaux contenant de l'azote, par du fluor, du chlore ou un radical trifluorométhyle,
R⁷ désigne l'hydrogène ou le fluor,
et
R⁸ représente l'hydrogène, le fluor, le chlore, un groupe azido, trifluorométhyle, hydroxy, trifluorométhoxy ou un reste alkoxy ou alkyle linéaire ou ramifié ayant chacun jusqu'à 4 atomes de carbone ou un reste de formule-NR¹⁵R¹⁶,
dans laquelle
R¹⁵ et R¹⁶ sont identiques ou différents et représentent l'hydrogène ou un reste alkyle linéaire ou ramifié ayant jusqu'à 3 atomes de carbone,
ou bien
R⁷ et R⁸ forment ensemble un reste de formule =O,
E est un reste cyclopropyle, cyclobutyle, cyclopentyle ou cyclohexyle ou un reste phényle qui est éventuellement substitué par du fluor ou par un radical trifluorométhyle, ou bien
un reste alkyle linéaire ou ramifié ayant jusqu'à 4 atomes de carbone, qui est éventuellement substitué par un radical hydroxy,
R¹ et R² forment ensemble une chaîne alkylénique linéaire ou ramifiée ayant jusqu'à 7 atomes de carbone, qui doit être substituée par un groupe carbonyle ou par un reste de formule
-OR¹⁹
dans laquelle
R¹⁹ représente l'hydrogène, un reste cyclopropyle, cyclopentyle, cyclohexyle, un reste silylalkyle linéaire ou ramifié ayant jusqu'à 6 atomes de carbone ou un reste alkyle linéaire ou ramifié ayant jusqu'à 4 atomes de carbone,
et la chaîne alkylénique formée de R¹ et R² est éventuellement substituée par un reste à liaison spiro de formule 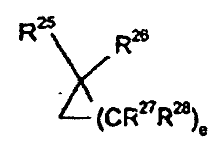 où
e représente le nombre 1, 2, 3, 4 ou 5,
R²⁵, R²⁶, R²⁷ et R²⁸ sont identiques ou différents et représentent l'hydrogène,
et leurs stéréoisomères, leurs mélanges de stéréoisomères et leurs sels.

2. Composés de formule (I) suivant la revendication 1,
formule dans laquelle
A représente un reste phényle éventuellement substitué par du fluor, en particulier le reste 4-fluorophényle,
et
D est un reste de formule 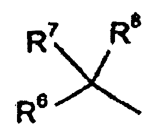 dans laquelle
R⁶ désigne un reste phényle ou un reste phényle à substituant trifluorométhyle,
R⁷ représente l'hydrogène,
R⁸ représente l'hydrogène, le fluor, un groupe méthoxy ou hydroxy,
ou bien
R⁷ et R⁸ forment ensemble un groupe carbonyle,
E est un groupe cyclopropyle, cyclopentyle ou cyclohexyle, ou bien un groupe alkyle linéaire ou ramifié ayant jusqu'à 4 atomes de carbone, qui est éventuellement substitué par un radical hydroxy,
et
R¹ et R² forment ensemble un reste de formule 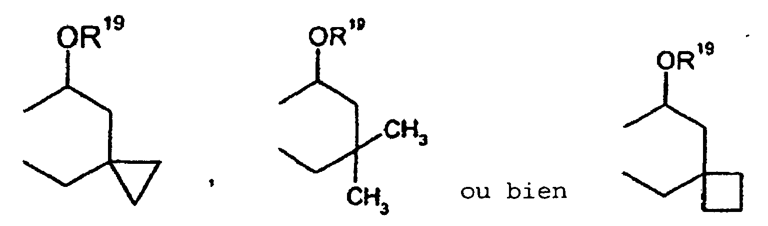 dans laquelle
R¹⁹ représente l'hydrogène,
et leurs stéréoisomères, leurs mélanges de stéréoisomères et leurs sels.

3. Composés suivant la revendication 1, destinés à être utilisés pour combattre des maladies.

4. Procédé de production de composés suivant la revendication 1, **caractérisé en ce que** :
[A] on transforme tout d'abord des composés de formule générale (II) 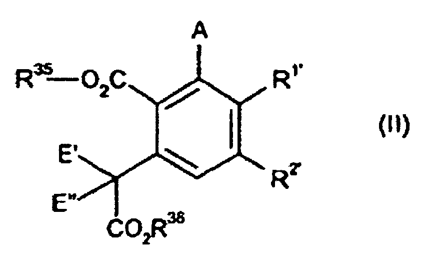 dans laquelle
A a la définition indiquée ci-dessus,
R³⁵ et R³⁶ sont identiques ou différents et désignent un reste alkyle linéaire ou ramifié ayant jusqu'à 4 atomes de carbone,
R^{1'} et R^{2'} forment ensemble la chaîne alkylénique linéaire ou ramifiée indiquée ci-dessus pour R¹ et R², ayant jusqu'à 7 atomes de carbone, qui est substituée par un reste tertiobutyldiméthylsilanyloxy (OTBS),
et
E' et E", conjointement avec l'atome de carbone auquel ils sont liés, couvrent l'étendue de la définition donnée ci-dessus pour E,
à réduction sélective de l'ester aliphatique suivie de la réaction avec des composés de formule générale (III)
R³⁷-halogène (III)
dans laquelle
R³⁷ est un reste mésyle, tosyle ou sulfonyle,
et
halogène représente le chlore, le brome ou l'iode, avantageusement le chlore,
dans des solvants inertes en présence d'une base,
en composés de formule générale (IV) 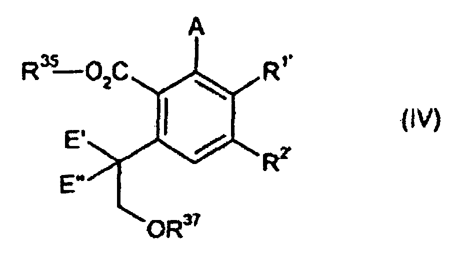 dans laquelle
A, E', E", R^{1'}, R^{2'}, R³⁵ et R³⁷ ont la définition indiquée ci-dessus,
on transforme ces composés dans une autre étape, en fonction des définitions indiquées ci-dessus pour E'/E", soit par une double réduction, soit par une saponification, une réaction de Barton et une réduction, en les composés de formule générale (V) 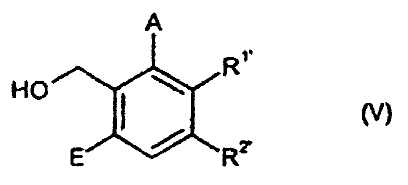 dans laquelle
A, E, R^{1'} et R^{2'} ont la définition indiquée ci-dessus,
puis on prépare par oxydation les aldéhydes de formule générale (VI) 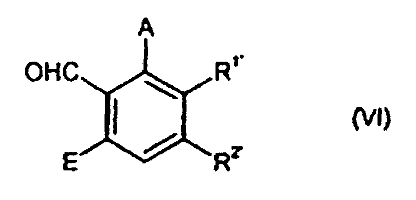 dans laquelle
A, E, R^{1'} et R^{2'} ont la définition indiquée ci-dessus,
et finalement, par exemple, par une réaction de Grignard, on transforme le groupe formyle en le reste D et on élimine le groupe TBS par des moyens classiques,
ou bien
[B] on transforme tout d'abord des composés de formule générale (VI) 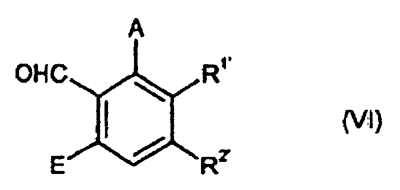 dans laquelle
A, E, R^{1'} et R^{2'} ont la définition indiquée ci-dessus,
de la manière décrite en [A], dans une réaction de Grignard, avec des composés de formule générale (VII)
R³⁸-MgBr (VII)
dans laquelle
R³⁸ englobe la définition indiquée ci-dessus pour R⁶,
dans des solvants inertes et en atmosphère de gaz protecteur, en composés de formule générale (VIII) 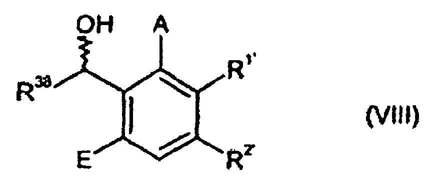 dans laquelle
A, D, E, R³⁸, R^{1'} et R^{2'} ont la définition indiquée ci-dessus,
on introduit éventuellement par des moyens classiques à la suite de cette étape, à partir de la fonction hydroxy, les substituants R⁷/R⁸ indiqués pour D,
et finalement, on élimine le groupe TBS par le fluorure de tétrabutylammonium dans des solvants inertes,
ou bien
[C] on réduit tout d'abord des composés de formule générale (IX) 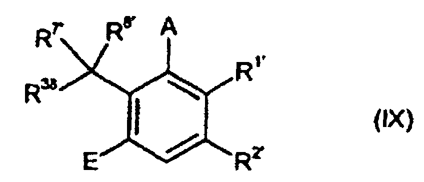 dans laquelle
A, E, R^{1'}, R^{2'} et R³⁸ ont la définition indiquée ci-dessus
et
R^{7'} et R^{8'} forment ensemble le groupe carbonyle,
en composés de formule générale (VIII) qu'on fait ensuite réagir comme décrit en [B],
ou bien
[D] on transforme des composés de formule (X) 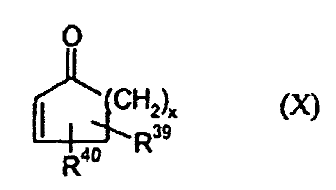 dans laquelle
x représente le nombre 1, 2 ou 3,
et
R³⁹ et R⁴⁰ sont identiques ou différents et représentent l'hydrogène ou un reste alkyle linéaire ou ramifié ayant jusqu'à 6 atomes de carbone,
R³⁹ et R⁴⁰ pouvant aussi occuper une position géminée,
ou bien
R³⁹ et R⁴⁰ forment ensemble un carbocycle à liaison spiro ayant 3 à 7 atomes de carbone,
avec les composés de formule générale (XI) 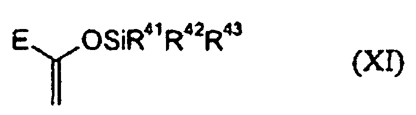 dans laquelle
E a la définition indiquée ci-dessus
et
R⁴¹, R⁴² et R⁴³ sont identiques ou différents et représentent un reste alkyle linéaire ou ramifié ayant jusqu'à 10 atomes de carbone,
en présence d'un réactif métallique ou semi-métallique, en passant tout d'abord par le stade intermédiaire de formule générale (XII) 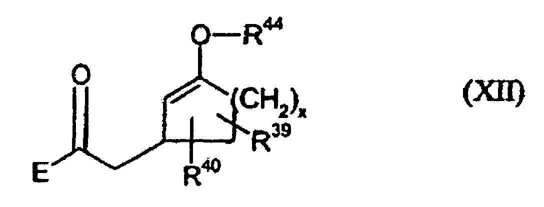 dans laquelle
x, E, R³⁹ et R⁴⁰ ont la définition indiquée ci-dessus
et
R⁴⁴ représente des dérivés métalliques ou semi-métalliques, avantageusement les dérivés du titane,
et par addition des composés de formule générale (XIII) 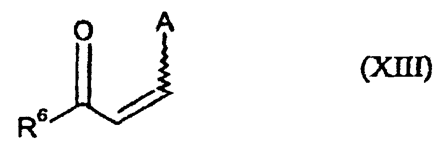 dans laquelle
R⁶ et A ont la définition indiquée ci-dessus,
en passant par les stades intermédiaires de formule générale (XIV) 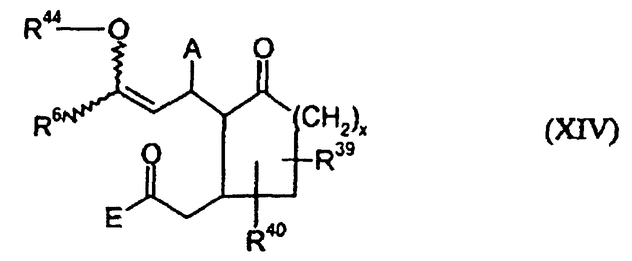 dans laquelle
A, E, R⁶, R³⁹, R⁴⁰, R⁴⁴ et x ont la définition indiquée ci-dessus,
en composés de formule générale (XV) 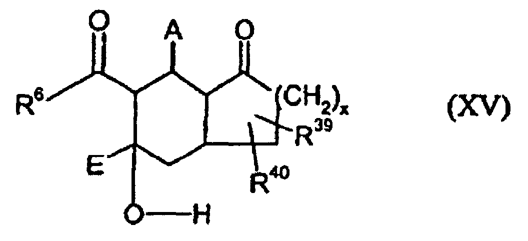 dans laquelle
A, E, R⁶, R³⁹, R⁴⁰ et x ont la définition indiquée ci-dessus,
puis on prépare par une élimination les composés de formule générale (XVI) 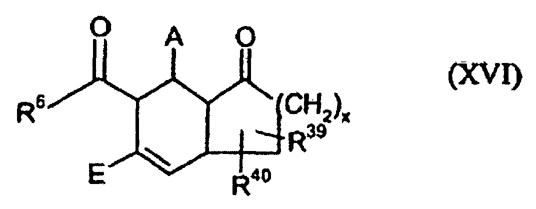 dans laquelle
A, E, R⁶, R³⁹, R⁴⁰ et x ont les définitions indiquées ci-dessus,
on prépare dans une autre étape par une halogénation, des composés halogénés, par exemple des composés de formule générale (XVII) 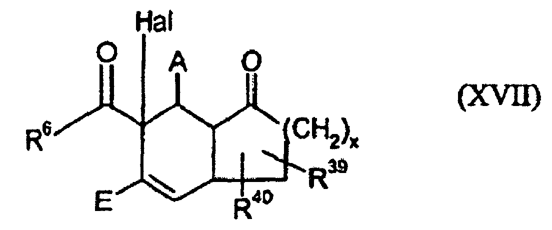 dans laquelle
R⁶, R³⁹, R⁴⁰, x, A et E ont les définitions indiquées ci-dessus,
et
Hal est un halogène, avantageusement le chlore ou le brome, en passant par les cyclohexadiènes produits in situ de formule générale (XVIII) ou leurs isomères 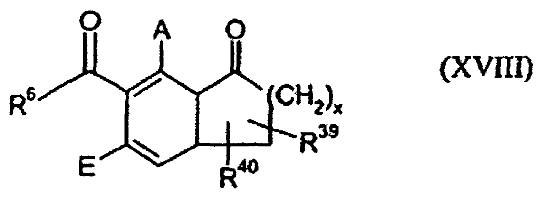 formule dans laquelle
A, E, x, R⁶, R³⁹ et R⁴⁰ ont la définition indiquée ci-dessus,
on prépare après oxydation les composés de formule générale (XIX) 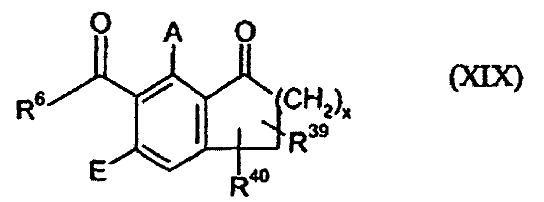 dans laquelle
A, E, x, R⁶, R³⁹ et R⁴⁰ ont les définitions indiquées ci-dessus,
on prépare en outre par réduction de la fonction céto les composés de formule générale (XX) 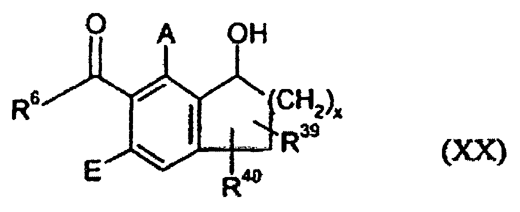 dans laquelle
A, E, x, R⁶, R³⁹ et R⁴⁰ ont la définition indiquée ci-dessus,
la réduction pouvant aussi être conduite de façon stéréosélective,
on prépare ensuite par silylation, par exemple par réaction avec des composés silylés (-SiR⁴¹R⁴²R⁴³) chlorés ou à substituant trifluorométhanesulfonyle
les composés de formule générale (XXI) 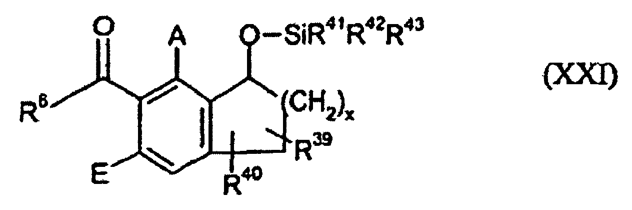 dans laquelle
x, A, E, R⁶, R³⁹, R⁴⁰, R⁴¹, R⁴² et R⁴³ ont la définition indiquée ci-dessus,
on obtient en outre par réduction de la seconde fonction céto, tout d'abord les composés de formule générale (XXII) 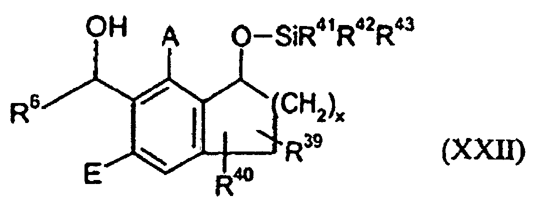 dans laquelle
x, A, E, R⁶, R³⁹, R⁴⁰, R⁴¹, R⁴² et R⁴³ ont la définition indiquée ci-dessus,
sous forme de mélange de diastéréoisomères, à partir duquel on obtient ensuite par séparation l'isomère de formule générale (XXIII) 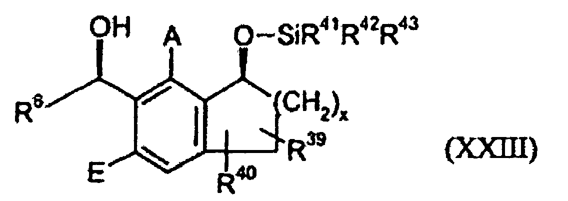 dans laquelle
x, A, E, R⁶, R³⁹, R⁴⁰, R⁴¹, R⁴² et R⁴³ ont la définition indiquée ci-dessus,
et on remplace éventuellement par substitution nucléophile, de façon énantiosélective, la fonction hydroxy par l'un des substituants indiqués ci-dessus pour R⁸ et on obtient ainsi des composés de formule générale (XXIV) 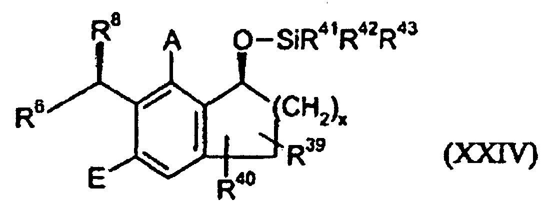 dans laquelle
x, A, E, R⁶, R⁸, R³⁹, R⁴⁰, R⁴¹, R⁴² et R⁴³ ont la définition indiquée ci-dessus,
et dans une dernière étape, on libère la fonction hydroxy en éliminant le groupe silyle protecteur par des moyens usuels,
et dans le cas des énantiomères/racémates, on effectue une séparation par des moyens usuels.

5. Composés de formule générale (XV) 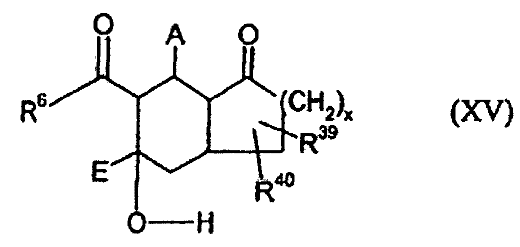 dans laquelle
A, E et R⁶ ont les définitions indiquées dans la revendication 1 et R³⁹, R⁴⁰ et x ont les définitions indiquées dans la revendication 4.

6. Médicament contenant au moins un composé suivant la revendication 1, ainsi que des auxiliaires de formulation acceptable du point de vue pharmacologique.

7. Médicament suivant la revendication 6, destiné au traitement de l'hyperlipoprotéinémie.

8. Médicament suivant la revendication 6, destiné au traitement de l'artériosclérose.

9. Utilisation de composés suivant la revendication 1, pour la préparation de médicaments.

10. Utilisation suivant la revendication 9, pour la préparation de médicaments destinés au traitement de l'artériosclérose, en particulier de dyslipidémies.
